(19) Europäisches Patentamt
European Patent Office
Office européen des brevets

(11) EP 4 462 101 A1

(12) EUROPEAN PATENT APPLICATION

(43) Date of publication:
**13.11.2024 Bulletin 2024/46**

(21) Application number: **24174781.5**

(22) Date of filing: **08.05.2024**

(51) International Patent Classification (IPC):
***G01N 15/1433*** *(2024.01)* ***G01N 15/1429*** *(2024.01)*

(52) Cooperative Patent Classification (CPC):
**G01N 15/1433;** G01N 2015/1006; G01N 2015/1402

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.05.2023 US 202363465057 P**

(71) Applicant: **Becton, Dickinson and Company**
**Franklin Lakes, NJ 07417-1880 (US)**

(72) Inventors:
- **Ramachandran, Supriya Balaji**
**Fremont (US)**
- **Middlebrook, Aaron Jacob**
**San Jose (US)**

(74) Representative: **Bals & Vogel Patentanwälte PartGmbB**
**Konrad-Zuse-Str. 4**
**44801 Bochum (DE)**

# (54) METHODS, SYSTEM AND NON-TRANSITORY COMPUTER-READABLE STORAGE MEDIUM FOR CLASSIFYING ANALYTE DATA INTO CLUSTERS

(57) Computer-implemented methods of classifying analyte data are provided. Methods of interest include applying a regression model to determine a relationship between an initial set of analyte features and a cluster criterion, generating a sparse set from at most a portion of the initial set of the analyte features based on the relationship, generating a classification model based on the sparse set, and applying the classification model to classify the analyte data into the clusters. Systems and non-transitory computer-readable storage media configured to carry out the subject methods are also provided.

FIG. 1

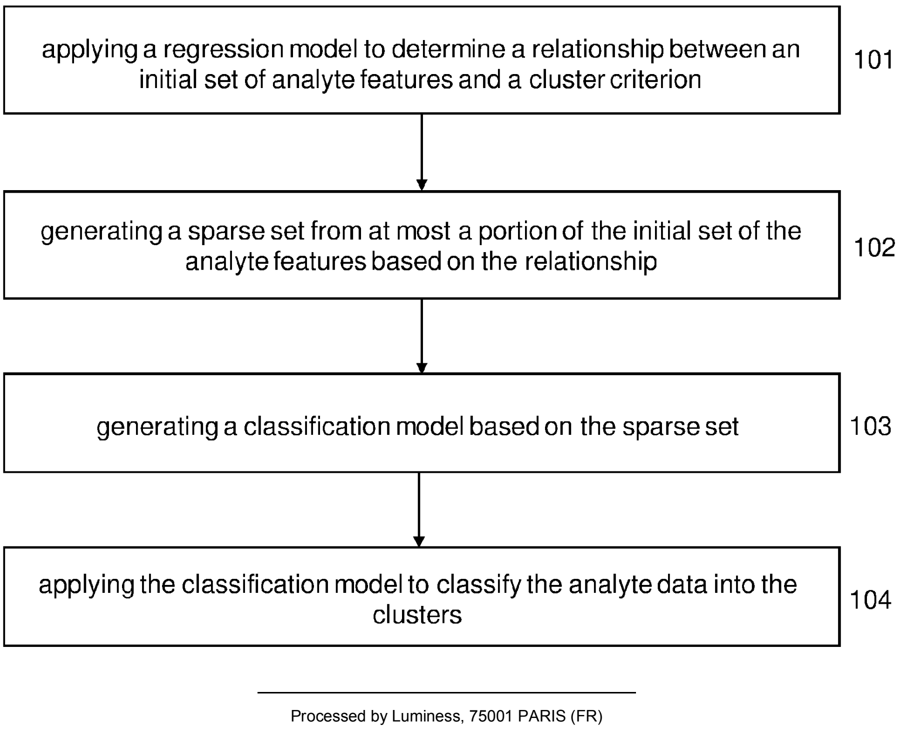


EP 4 462 101 A1

## Description

## CROSS-REFRENCE TO RELATED APPLICATION

Pursuant to 35 U.S.C. § 119(e), this application claims priority to the filing date of United States Provisional Patent Application Serial No. 63/465,057 filed May 9, 2023, the disclosure of which application is incorporated herein by reference in its entirety.

## INTRODUCTION

The characterization of analytes in biological fluids has become an important part of biological research, medical diagnoses and assessments of overall health and wellness of a patient. Detecting analytes in biological fluids, such as human blood or blood derived products, can provide results that may play a role in determining a treatment protocol of a patient having a variety of disease conditions.

Flow cytometry is a technique used to characterize and often times sort biological material, such as cells of a blood sample or particles of interest in another type of biological or chemical sample. A flow cytometer typically includes a sample reservoir for receiving a fluid sample, such as a blood sample, and a sheath reservoir containing a sheath fluid. The flow cytometer transports the particles (including cells) in the fluid sample as a cell stream to a flow cell, while also directing the sheath fluid to the flow cell. To characterize the components of the flow stream, the flow stream is irradiated with light. Variations in the materials in the flow stream, such as morphologies or the presence of fluorescent labels, may cause variations in the observed light and these variations allow for characterization and separation. To characterize the components in the flow stream, light must impinge on the flow stream and be collected. Light sources in flow cytometers can vary and may include one or more broad spectrum lamps, light emitting diodes as well as single wavelength lasers. The light source is aligned with the flow stream and an optical response from the illuminated particles is collected and quantified.

Isolation of biological particles has been achieved by adding a sorting or collection capability to flow cytometers. Particles in a segregated stream, detected as having one or more desired characteristics, are individually isolated from the sample stream by mechanical or electrical removal. A common flow sorting technique utilizes drop sorting in which a fluid stream containing linearly segregated particles is broken into drops. The drops containing particles of interest are electrically charged and deflected into a collection tube by passage through an electric field. Typically, the linearly segregated particles in the stream are characterized as they pass through an observation point situated just below the nozzle tip. Once a particle is identified as meeting one or more desired criteria, the time at which it will reach the drop break-off point and break from the stream in a drop can be predicted. Ideally, a brief charge is applied to the fluid stream just before the drop containing the selected particle breaks from the stream and then grounded immediately after the drop breaks off. The drop to be sorted maintains an electrical charge as it breaks off from the fluid stream, and all other drops are left un-charged.

The parameters measured using a flow cytometer typically include light at the excitation wavelength scattered by the particle in a narrow angle along a mostly forward direction, referred to as forward-scatter (FSC), the excitation light that is scattered by the particle in an orthogonal direction to the excitation laser, referred to as side-scatter (SSC), and the light emitted from fluorescent molecules in one or more detectors that measure signal over a range of spectral wavelengths, or by the fluorescent dye that is primarily detected in that specific detector or array of detectors. Different cell types can be identified by their light scatter characteristics and fluorescence emissions resulting from labeling various cell proteins or other constituents with fluorescent dye-labeled antibodies or other fluorescent probes.

Flow cytometers may further comprise means for recording the measured data and analyzing the data. For example, data storage and analysis may be carried out using a computer connected to the detection electronics. For example, the data can be stored in tabular form, where each row corresponds to data for one particle, and the columns correspond to each of the measured features. The use of standard file formats, such as an "FCS" file format, for storing data from a particle analyzer facilitates analyzing data using separate programs and/or machines. Using current analysis methods, the data typically are displayed in 1-dimensional histograms or 2-dimensional (2D) plots for ease of visualization, but other methods may be used to visualize multidimensional data.

While flow cytometer data generally contains numerous data points (i.e., events), it is often the case that only a certain portion of the flow cytometer data is of interest to the user. For example, it may be desirable to identify the best parameters to discriminate debris/small particles from single cells and multiplets. Debris are essentially pieces of cells that have been broken during processing. Multiplets are two or more cells that are joined together. Cellular debris can be considered as "junk" or data that users do not want to collect or process with further analyses. Multiplets are also events that are desirable to remove from analysis as the fluorescent signal obtained from these are double of what would be observed from single cells, i.e., they are outlier events. Removal of such debris and multiplets is often a first step performed in the analysis of flow data. In other words, doublet and clump exclusion is a critical preliminary step in establishing a pure sort. Doublets can confound purity and can lead to unwanted results or unnecessary expense when performing downstream functional and or genomic analyses.

## SUMMARY

The present disclosure provides improvements to the processes by which analyte data (e.g., flow cytometer data) is classified, e.g., in the process of removing data associated with undesirable analytes (e.g., debris, multiplets, etc.). It was found that manual gating (SSC-A vs FSC-A) for singlets can result in 40% of true non-singlets being wrongly classified as singlets. Furthermore, analyte classification often varies greatly between different users, thereby hindering generalizability and reproducibility of results. As such, a simplified process for data cleanup is desirable. Particularly, automated processes are needed for cleaning data that minimize the removal of events of interest which can result from the drawing of manual gates. Embodiments of the present disclosure satisfy these and other needs.

Aspects of the disclosure include computer-implemented methods of classifying analyte data. Methods of interest include, via a processor, applying a regression model to determine a relationship between an initial set of analyte features and a cluster criterion, generating a sparse set from at most a portion of the initial set of the analyte features based on the relationship, generating a classification model based on the sparse set, and applying the classification model to classify the analyte data into the clusters. In some embodiments, the analyte data is flow cytometer data. In some such embodiments, the cluster criterion is an association of the analyte data with a singlet. In certain cases, the clusters comprise a singlet cluster and a non-singlet cluster. In some versions, the analyte features comprise a size feature, imaging feature, a scatter feature, or any combination thereof. In some cases, the classification model comprises a mixture model, such as a Gaussian mixture model. In other cases, the classification model comprises a density-based spatial clustering of applications with noise (DBSCAN) algorithm. In still other cases, the classification model comprises a balanced iterative reducing and clustering using hierarchies (BIRCH) algorithm. In yet other cases, the classification model comprises a K-means clustering algorithm. In further cases, the classification model comprises a spectral clustering algorithm. In some cases, the initial set of analyte features comprises from 5 to 20 analyte features (e.g., 9 to 11 analyte features). In certain versions, the sparse set of analyte features comprises from 2 to 10 analyte features (e.g., 2 to 4 analyte features). In certain versions, classifying the analyte data comprises including 90% or more (e.g., 97% or more) of analyte data associated with the cluster criterion in a cluster associated with the cluster criterion. In some cases, classifying the analyte data comprises excluding 85% or more (e.g., 92% or more) of analyte data not associated with the cluster criterion from a cluster associated with the cluster criterion.

Aspects of the disclosure additionally include systems. Systems of interest include memory operably coupled to a processor, wherein the memory comprises instructions stored thereon, which when executed by the processor, cause the processor to carry out a method of the disclosure (e.g., as described above and herein). In other words, the processor is configured to apply a regression model to determine a relationship between an initial set of analyte features and a cluster criterion, generate a sparse set from at most a portion of the initial set of the analyte features based on the relationship, generate a classification model based on the sparse set, and apply the classification model to classify analyte data into the clusters. In some instances, the system is or comprises (e.g., is operably connected with) a flow cytometer. In some versions, the flow cytometer is an imaging-enabled flow cytometer. Methods of the disclosure may in some cases involve providing analyte data to a system of the disclosure, and receiving classified analyte data from said system.

Aspects of the disclosure also include non-transitory computer-readable storage media comprising instructions stored thereon for classifying analyte data by a method of the disclosure. In other words, the instructions are for classifying analyte data by applying a regression model to determine a relationship between an initial set of analyte features and a cluster criterion, generating a sparse set from at most a portion of the initial set of the analyte features based on the relationship, generating a classification model based on the sparse set, and applying the classification model to classify the analyte data into the clusters.

## BRIEF DESCRIPTION OF THE FIGURES

The disclosure may be best understood from the following detailed description when read in conjunction with the accompanying drawings. Included in the drawings are the following figures:

**FIG. 1** presents a flow chart for practicing a method of classifying analyte data according to certain embodiments.
**FIG. 2** presents a flow cytometric system according to certain embodiments.
**FIG. 3** depicts an image-enabled particle sorter according to certain embodiments.
**FIG. 4** depicts a functional block diagram of a particle analysis system according to certain embodiments.
**FIG. 5** depicts a depicts a functional block diagram for one example of a control system according to certain embodiments.

**FIG. 6A-6B** depict schematic drawings of a particle sorter system according to certain embodiments.

**FIG. 7** depicts aspects of a computer-controlled system according to certain embodiments.

**FIG. 8A-8B** depict flow charts for carrying out feature selection **(FIG. 8A)** and Gaussian mixture modeling **(FIG. 8B).**

**FIG. 9** depicts imaging features used in a manual gating method, and the distribution of lymphocytes, monocytes and high scatter events.

**FIG. 10A-10B** depict results from an imaging alone non-singlet exclusion method.

**FIG. 11A-11 B** depict results from a height vs. width plus imaging non-singlet exclusion method.

**FIG. 12A-12B** depict results from a height vs. area plus imaging non-singlet exclusion method.

**FIG. 13A-13B** compare non-singlet exclusion **(FIG. 13A)** and singlet preservation **(FIG. 13B)** among different methods.

**FIG. 14A-14B** present clustering outcomes.

**FIG. 15** shows a box-and whisker plot of singlet inclusion and non-singlet exclusion.

## DETAILED DESCRIPTION

Computer-implemented methods of classifying analyte data are provided. Methods of interest include applying a regression model to determine a relationship between an initial set of analyte features and a cluster criterion, generating a sparse set from at most a portion of the initial set of the analyte features based on the relationship, generating a classification model based on the sparse set, and applying the classification model to classify the analyte data into the clusters. Systems and non-transitory computer-readable storage media configured to carry out the subject methods are also provided.

Before the present disclosure is described in greater detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order which is logically possible.

While the system and method has or will be described for the sake of grammatical fluidity with functional explanations, it is to be expressly understood that the claims, unless expressly formulated under 35 U.S.C. §112, are not to be construed as necessarily limited in any way by the construction of "means" or "steps" limitations, but are to be accorded the full scope of the meaning and equivalents of the definition provided by the claims under the judicial doctrine of equivalents, and in the case where the claims are expressly formulated under 35 U.S.C. §112 are to be accorded full statutory equivalents under 35 U.S.C. §112.

## METHODS OF CLASSIFYING ANALYTE DATA

Aspects of the disclosure include computer-implemented methods of classifying analyte data into clusters. By "analyte data", it is meant data obtained by assessing a particular analyte for certain characteristics. By "classifying" the analyte data, it is meant designating analyte data (e.g., groups of analyte data) as belonging to a particular type out of one or more possible different types said data could belong to. Methods of the disclosure may in some cases be sufficient to improve analyte data classification relative to conventional classification methods, such as where analyte data is manually classified by a user (e.g., by drawing a gate on flow cytometer data). For example, the subject methods may in certain embodiments increase classification accuracy. Accuracy may be determined by assessing whether each analyte or data point/event associated therewith does in fact belong to the particular type with which it is classified. In certain cases, methods of the disclosure may increase classification accuracy relative to conventional methods (e.g., drawing a manual gate) by 1% or more, such as 5 % or more, such as 10% or more, such as 15% or more and including 20% or more. In embodiments, practicing the subject methods is sufficient to increase the speed and/or efficiency with which analyte data is classified relative to conventional methods (e.g., drawing a manual gate) such as by 1% or more such as 5% or more, such as 10% or more, such as 15% or more and including 20% or more. As noted above, methods of the disclosure are computer-implemented methods. In other words, method steps described herein may be carried out via a processor associated with a computer system. Any suitable processor may be used in the subject methods, such as those described below with respect to the systems of the disclosure.

In some cases, the analyte data is flow cytometer data. By "flow cytometer data" it is meant information regarding the characteristics of sample particles that has been collected by any number of detectors in a particle analyzer. As discussed herein, a "particle analyzer" is an analytical tool (e.g., flow cytometer) that enables the characterization of particles on the basis of certain (e.g., optical) parameters. By "particle", it is meant a discrete component of a biological sample such as a molecule, analyte-bound bead, individual cell, or the like. While the present disclosure is primarily described in terms of flow cytometer data, the applicability of the disclosure is not limited to flow cytometer data. In certain cases, the present disclosure may be applicable to other types of data, such as nucleic acid data.

Flow cytometer data may be received from any suitable source. In some embodiments, flow cytometer data is received from the memory of a storage device. In such embodiments, flow cytometer data may have been previously generated and saved in the memory of the storage device for subsequent recall and analysis. In other embodiments, the flow cytometer data is received in real time. Put another way, flow cytometer data generated during the operation of a flow cytometer may subsequently (e.g., immediately) populate the data-space (e.g., two-dimensional plot). In embodiments, the flow cytometer data is received from a forward scatter detector. A forward scatter detector may, in some instances, yield information regarding the overall size of a particle. In embodiments, the flow cytometer data is received from a side scatter detector. A side scatter detector may, in some instances, be configured to detect refracted and reflected light from the surfaces and internal structures of the particle, which tends to increase with increasing particle complexity of structure.

In certain embodiments, the particles are detected and uniquely identified by exposing the particles to excitation light and measuring the fluorescence of each particle in one or more detection channels, as desired. Fluorescence emitted in detection channels used to identify the particles and binding complexes associated therewith may be measured following excitation with a single light source, or may be measured separately following excitation with distinct light sources. If separate excitation light sources are used to excite the particle labels, the labels may be selected such that all the labels are excitable by each of the excitation light sources used. In embodiments, the flow cytometer data is received from a fluorescent light detector. A fluorescent light detector may, in some instances, be configured to detect fluorescence emissions from fluorescent molecules, e.g., labeled specific binding members (such as labeled antibodies that specifically bind to markers of interest) associated with the particle in the flow cell. In certain embodiments, methods include detecting fluorescence from the sample with one or more fluorescence detectors, such as 2 or more, such as 3 or more, such as 4 or more, such as 5 or more, such as 6 or more, such as 7 or more, such as 8 or more, such as 9 or more, such as 10 or more, such as 15 or more and including 25 or more fluorescence detectors. In embodiments, each of the fluorescence detectors is configured to generate a fluorescence data signal. Fluorescence from the sample may be detected by each fluorescence detector, independently, over one or more of the wavelength ranges of 200 nm - 1200 nm. In some instances, methods include detecting fluorescence from the sample over a range of wavelengths, such as from 200 nm to 1200 nm, such as from 300 nm to 1100 nm, such as from 400 nm to 1000 nm, such as from 500 nm to 900 nm and including from 600 nm to 800 nm. In other instances, methods include detecting fluorescence with each fluorescence detector at one or more specific wavelengths. For example, the fluorescence may be detected at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof, depending on the number of different fluorescence detectors in the subject light detection system. In certain embodiments, methods include detecting wavelengths of light which correspond to the fluorescence peak wavelength of certain fluorophores present in the sample. In embodiments, flow cytometer data is received from one or more light detectors (e.g., one or more detection channels), such as 2 or more, such as 3 or more, such as 4 or more, such as 5 or more, such as 6 or more and including 8 or more light detectors (e.g., 8 or more detection channels).

In some cases, methods may include classifying multiple sets of analyte data, such as 2 or more sets, 3 or more sets, 4 or more sets, 5 or more sets, 7 or more sets, 8 or more sets, 9 or more sets, and including 10 or more sets. In such cases, sets may be from the same source or from different sources. The number of data points (e.g., events, observations) classified by the subject methods may also vary. In some cases, the number of data points ranges from 1k to 100k, such as 10k to 80k, such as 20k to 60k and including 25k to 50k. In some embodiments, the number of data points is 1k or more, such as 5k or more, such as 10k or more, such as 15k or more, such as 20k or more, such as 25k or more, such as 30k or more, such as 35k or more, such as 40k or more, such as 45k or more, such as 50k or more, such as 55k or more, such as 60k or more, such as 65k or more, such as 70k or more, such as 75k or more, such as 80k or more, such as 85k or more, such as 90k or more, such as 95k or more and including 100k or more.

In some cases, prior to clustering the analyte data, methods include preprocessing the data, e.g., such that it is in a more suitable form for manipulation by different models. Any suitable preprocessing protocol may be employed. In some embodiments, methods include standardizing analyte features, e.g., such that they are centered around the mean and scaled to unit variance.

Methods of the disclosure include applying a regression model to determine a relationship between an initial set of analyte features and a cluster criterion. By "analyte features" it is meant one or more properties (e.g., optical, impedance, and/or temporal properties) associated with each individual analyte (e.g., particle) such that each analyte is present in the analyte data as a set of digitized feature values. Depending on the requirements of a given experiment, the number of analyte features present in the data may vary and can include, e.g., 10 features or more, such as 20 features or more, such as 30 features or more, such as 40 features or more, such as 50 features or more, and including 60 features or more. In certain instances, the analyte features are selected from size features, imaging features, and scatter features. In some such instances the analyte features are scatter features selected from side-scatter (SSC) features and forward-scatter (FSC) features. Where the analyte data is flow cytometer data, the analyte features may also be associated with and/or obtained from fluorescent light, axial light loss (ALL), and the like. Exemplary features include, but are not limited to, size, center of mass, short axis moment, diffusivity, long axis moment, radial moment, maximum intensity, and eccentricity. In embodiments, the analyte features comprise imaging features.

The "cluster criterion" discussed herein may be any suitable standard relative to which analyte data may be assessed and classified into clusters. For example, the cluster criterion may be an association of the analyte data with a certain parameter of interest. Analyte data may be considered to be "associated" with a parameter of interest if the analyte corresponding to the data can be said to correspond to said parameter, i.e., it is positive therefor. Such analyte data may be classified into a particular cluster, while analyte data that does not correspond to the cluster criterion may be classified into one or more other clusters (e.g., clusters that are negative for the parameter of interest). In some embodiments, analyte data that does not correspond to the cluster criterion may be classified into a single cluster (i.e., such that there are 2 total clusters). Alternatively, analyte data that does not correspond to the cluster criterion may be classified into multiple clusters according to some other criterion, such as 2 or more clusters, 3 or more clusters, 4 or more clusters, 5 or more clusters, 6 or more clusters, 7 or more clusters, 8 or more clusters, 9 or more clusters, and including 10 or more clusters. Cluster criteria may vary according to the nature of the analytes being observed and/or the nature of an experiment being performed. In some cases, the cluster criterion is an association of the analyte data with a singlet. In such cases, flow cytometer data associated with a singlet may be classified into a singlet cluster, while flow cytometer that is not associated with a singlet may be classified into one or more non-singlet clusters. Non-singlets that may be classified into the non-singlet cluster may include, but are not limited to multiplets/aggregates (e.g., doublets, triplets, quadruplets; quintuplets; and so on), debris (e.g., components of lysed cells), and the like. In accordance with the above, the non-singlets may be classified into a single non-singlet cluster, or may be further segmented into multiple non-singlets clusters (e.g., where one cluster corresponds to doublets, one to debris, and so on, as appropriate). In some cases, the non-singlet cluster comprises a doublet or an aggregate. Other cluster criteria may include association with a particular fluorescent marker which may, itself, be associated with a particular phenotype of analyte depending on the nature of the experiment being performed. In some embodiments, the cluster criterion is a size or shape of analytes.

An "initial" set of analyte features is a subset of analyte features from a universe of possible analyte features (e.g., ones that could be obtained from a given flow cytometer in an experiment having particular constraints) which have been found via a regression model to have a relationship with the cluster criterion such that the initial set is more useful for predicting the classification of analyte data into clusters according to the cluster criterion relative to other analyte features in the universe of possible analyte features. In some cases, the analyte features in the initial set are more predictive than the universe of possible analyte features when considered as a whole by 1% or more, such as 5%

or more, such as 10% or more, such as 15% or more, such as 20% or more, such as 25% or more, such as 30% or more, such as 35% or more, such as 40% or more and including 50% or more. The regression model may be any model configured to estimate the relationship between the analyte features and the cluster criterion. In some cases, the regression model employs linear regression. In other cases, the regression model employs nonlinear regression. Regression models may in some instances involve, e.g., Bayesian methods, percentage regression, scenario optimization, least absolute deviations, nonparametric regression, scenario optimization, distance metric learning, and the like.

The number of analyte features in the initial set may vary. In some cases, the initial set of analyte features comprises from 5 to 20 analyte features, such as 7 to 15 analyte features, and including 9 to 11 analyte features. In some cases, the initial set of analyte features comprises 5 analyte features, 6 analyte features, 7 analyte features, 8 analyte features, 9 analyte features, 10 analyte features, 11 analyte features, 12 analyte features, 13 analyte features, 14 analyte features, 15 analyte features, 16 analyte features, 17 analyte features, 18 analyte features, 19 analyte features, or 20 analyte features. The number of analyte features may also be described as a percentage of the number of analyte features in the universe of possible analyte features. In some cases, the initial set of analyte features comprises 10% or more of possible analyte features, such as 11% or more, 12% or more, 13% or more, 14% or more, 15% or more, 16% or more, 17% or more, 18% or more, 19% or more, 20% or more, 21% or more, 22% or more 23% or more, 24% or more, and including 25% or more. In some embodiments, the initial set comprises both imaging and non-imaging analyte features.

Methods of the disclosure additionally include generating a sparse set from at most a portion of the initial set of the analyte features based on the relationship. By "sparse set" it is meant a subset of the initial set of analyte features that has been further refined based on predictiveness relative to the cluster criterion. In other words, the sparse set optimizes a rate of inclusion of analyte data associated with the cluster criterion (e.g., singlets) from a cluster and a rate of exclusion of analyte data not associated with the cluster criterion (e.g., non-singlets). The number of analyte features in the sparse set may in some instances vary, and can range, e.g., from 2 to 10 analyte features, such as 2 to 5 analyte features, and including 2 to 4 analyte features. In some cases, the sparse set includes 2 analyte features, 3 analyte features, 4 analyte features, 5 analyte features, 6 analyte features, 7 analyte features, 8 analyte features, 9 analyte features or 10 analyte features. In some cases, the sparse set comprises 10% or more of the analyte features from the initial set, such as 15% or more, such as 20% or more, such as 25% or more, such as 30% or more, such as 35% or more and including 40% or more.

Protocols by which the sparse set is generated may in some cases vary. In some instances, generating the sparse set of analyte features includes iteratively tuning the analyte features of the initial set. In embodiments, the iterative tuning involves estimating experimental inclusion and exclusion rates relative to the cluster criterion. Put another way, the iterative tuning involves estimating a true inclusion rate of analyte data in a cluster that is associated with the cluster criterion and the exclusion of analyte data that is not associated with the cluster criterion from the same cluster. In a particular example involving singlets and non-singlets, the iterative tuning involves estimating an experimental true singlet inclusion rate and a true non-singlet exclusion rate. Tuning may be iterated until optimal rates are found. In some cases, generation of the sparse set involves iteratively testing different combinations of analyte features for accuracy (e.g., with respect to the true singlet inclusion rate and the true non-singlet exclusion rate), and the combination having the highest accuracy is taken as optimal. In some embodiments, the sparse set comprises both imaging and non-imaging analyte features.

In embodiments, the sparse set of analyte features may be generated based on training data. In some cases, the training data is used during the application of the regression model, the iterative tuning, or both. The training data may be received from any suitable source. In some embodiments, training data is received from the memory of a storage device. In such embodiments, training data may have been previously generated and saved in the memory of the storage device for subsequent recall and analysis. In embodiments, analyte data within the training dataset is of known classification. For example, in some cases where the training dataset includes flow cytometer data, each individual analyte may have been confirmed to correspond to one class or another by some other means. In certain instances, an expert user manually provides classifications to the training dataset. Such can include, e.g., manually drawing gates on a two-dimensional plot of flow cytometer data. Analyte features from the training dataset as well as these classifications may be provided for training purposes. In other words, the training data may be considered "ground truth" data. In some versions, such ground truth data is obtained by employing a particular stain or dye in a flow cytometer experiment that is known to correspond to an analyte characteristic of interest. The stain or dye may be selected depending on the nature of said characteristic. For example, where it is desirable to cluster singlets and non-singlets, ground truth data may be obtained using a DNA intercalating dye. Cells (at least of the eukaryotic variety) generally have a single nucleus containing DNA. Staining said DNA will thereby allow a user to reliably determine whether a given event/observation involves one cell (i.e., a singlet) or multiple cells (i.e., non-singlets). DNA intercalating dyes that may be employed include, but are not limited to, ethidium bromide, SYBR green, propidium iodide, acridine orange, DAPI and DRAQ5. In some embodiments, methods include training using a plurality of training datasets, such as 2 or more training datasets, such as 3 or more training datasets, such as 4 or more training datasets, and including 5 or more training datasets.

In addition to the above, methods include generating a classification model based on the sparse set. Any classification model suitable for clustering analyte data may be employed. In some cases, the classification model is a machine learning algorithm. In some versions, the classification model is or comprises a mixture model. Mixture models represent a combination of probability distributions. Each distribution may be a distinct subgroup or cluster within the data. These component distributions can be of different types, such as Gaussian (normal), exponential, or others, depending on the nature of the data being modeled. In a mixture model, each data point is assumed to have been generated by one of the component distributions, with the likelihood of being generated by each component determined by the model's parameters. The parameters typically include the weights (or proportions) of each component and the parameters (e.g., mean ($\mu$), co-variance ($\Sigma$)) of each component distribution. Details regarding mixture models may be found, e.g., in McNicholas, P. D. (2016). *Mixture model-based classification*; incorporated by reference herein.

In certain embodiments, the mixture model comprises a Gaussian mixture model. In such embodiments, methods involve assuming that the analyte data comprises a mixture of Gaussians (i.e., Gaussian distributions). In one exemplary version, methods include initializing a mean $\mu_c$, co-variance $\Sigma_c$, and fraction per cluster $\Pi_c$, and calculating the probability that each datapoint in the analyte data belongs to cluster c:

$$r_{ic} = \frac{\pi_c N(x_i \mid \mu_c, \Sigma_c)}{\sum_{k=1}^{K} \pi_k N(x_i \mid \mu_k, \Sigma_k)}$$

where $N(x \mid \mu\ \Sigma)$ describes the multivariate Gaussian with:

$$N(x_i, \mu_c, \Sigma_c) = \frac{1}{(2\pi)^{\frac{n}{2}} |\Sigma_c|^{\frac{1}{2}}} exp\left(-\frac{1}{2}(x_i - \mu_c)^T \Sigma_c^{-1} (x_i - \mu_c)\right)$$

For each cluster c, methods may include calculating the total weight $m_c$ and updating the parameters mean $\mu_c$, co-variance $\Sigma_c$, and fraction per cluster $\Pi_c$. The probability that each datapoint belongs to a cluster c may be calculated using formulae:

$$m_c = \sum_i r_{ic}$$

$$\pi_c = \frac{m_c}{m}$$

$$\mu_c = \frac{1}{m_c} \sum_i r_{ic} x_i$$

$$\Sigma_c = \frac{1}{m_c} \sum_i r_{ic} (x_i - \mu_c)^T (x_i - \mu_c)$$

Methods according to some embodiments also include iteratively repeating the above steps until a log-likelihood function of the model converges:

$$\ln p(X \mid \pi, \mu, \Sigma) = \sum_{i=1}^{K} \ln\left(\sum_{k=1}^{K} \pi_k N(x_i \mid \mu_k, \Sigma_k)\right)$$

Each datapoint is thereby assigned to a cluster.

In some cases, the classification model comprises a density-based spatial clustering of applications with noise (DBSCAN) algorithm. DBSCAN groups together data points by density, with points having a higher density forming a cluster. Details regarding DBSCAN may be found in, e.g., Ester et al. (1996) Proceedings of the Second International Conference on Knowledge Discovery and Data. 96(34):226-231; incorporated by reference herein. In some cases, the classification model comprises a K-means clustering algorithm. K-means clustering involves partitioning observations into clusters in which each observation belongs to the cluster with the nearest mean (e.g., centroid). Details regarding K-means clustering may be found in, e.g., Lloyd, Stuart P. (1967) IEEE Transactions on Information Theory. 28(2): 129-137; incorporated by reference herein. In other cases, the classification model comprises a balanced iterative reducing and clustering using hierarchies (BIRCH) algorithm. BIRCH is an unsupervised data mining algorithm used for hierarchical clustering. Details regarding BIRCH may be found in, e.g., Zhang et al. (1996) ACM sigmod record. 25(2): 103-114. In certain implementations, BIRCH may be employed to supplement one or more of the other algorithms discussed herein. For example, in some embodiments, BIRCH is used to accelerate K-means clustering. In further embodiments, BIRCH is used to accelerate Gaussian mixture modeling as described herein. In certain versions, the classification model comprises a spectral clustering algorithm. Spectral clustering involves the use of eigenvalues of a similarity matrix. Details regarding spectral clustering may be found in, e.g., Von Luxburg, U. (2007) Statistics and computing 17: 395-416; incorporated by reference herein.

After the classification model is generated, methods of the disclosure include applying the classification model to classify the analyte data into the clusters. Following the classification of the analyte data, methods according to some versions may include assessing the classification model, e.g., by comparing the classifications to ground truth data. In some cases, classifying the analyte data via the methods described herein comprises including 90% or more of the analyte data associated with a cluster criterion in the cluster associated with the cluster criterion, such as 90% or more, and including 97% or more. In addition, classifying the analyte data via methods described herein may involve excluding 85% or more of analyte data not associated with the cluster criterion from a cluster associated with the cluster criterion, such as 90% or more, and including 92% or more. In some embodiments, methods include generating one or more population clusters based on the analyte features in the sample. As used herein, a "population", or "subpopulation" of analytes, such as cells, nucleic acids or other particles, generally refers to a group of analytes that possess properties (e.g., optical, impedance, or temporal properties) with respect to one or more measured parameters such that measured parameter data form a cluster in the data space. In embodiments, data is comprised of signals from any given number of different parameters, such as, for instance 2 or more, 3 or more, 4 or more, 5 or more, 6 or more, 7 or more, 8 or more, 9 or more, 10 or more, and including 20 or more. Thus, populations are recognized as clusters in the data. Conversely, each data cluster generally is interpreted as corresponding to a population of a particular type of cell or analyte, although clusters that correspond to noise or background typically also are observed. A cluster may be defined in a subset of the dimensions, e.g., with respect to a subset of the measured parameters, which corresponds to populations that differ in only a subset of the measured parameters or features extracted from the measurements of the cell, particle or nucleic acid.

In embodiments, methods include receiving data, calculating parameters of each analyte, and clustering together analytes based on the calculated parameters. For example, where the data is flow cytometer data, an experiment may include particles labeled by several fluorophores or fluorescently labeled antibodies, and groups of particles may be defined by populations corresponding to one or more fluorescent measurements. In the example, a first group may be defined by a certain range of light scattering for a first fluorophore, and a second group may be defined by a certain range of light scattering for a second fluorophore. If the first and second fluorophores are represented on an x and y axis, respectively, two different color-coded populations might appear to define each group of particles, if the information was to be graphically displayed. Any number of analytes may be assigned to a cluster, including 5 or more analytes, such as 10 or more analytes, such as 50 or more analytes, such as 100 or more analytes, such as 500 analytes and including 1000 analytes. In certain embodiments, the method groups together in a cluster rare events (e.g., rare cells in a sample, such as cancer cells) detected in the sample. In these embodiments, the analyte clusters generated may include 10 or fewer assigned analytes, such as 9 or fewer and including 5 or fewer assigned analytes.

**FIG. 1** presents a flowchart for practicing methods according to embodiments of the disclosure. As shown in **FIG. 1,** step 101 includes applying a regression model to determine a relationship between an initial set of analyte features and a cluster criterion. Step 102 involves generating a sparse set from at most a portion of the initial set of the analyte features based on the relationship. Step 103 involves generating a classification model based on the sparse set. Step 104 involves applying the classification model to classify the analyte data into the clusters.

In certain cases, methods of the disclosure may be carried out in conjunction with methods described in U.S. Provisional Patent Application No. 63/569,559, filed March 25, 2024 (Atty. Dkt. No. BECT-367PRV); the disclosure of which is incorporated by reference herein in its entirety. In such cases, computer-implemented methods may include categorizing the analyte data based on analyte features associated therewith by generating a predicted class for the analyte data using a decision tree ensemble, and refining the categorized analyte data based on the analyte features and the predicted class using a distance-based classification model to classify the analyte data.

In some instances, the sample analyzed in the instant methods is a biological sample. The term "biological sample" is used in its conventional sense to refer to a whole organism, plant, fungi or a subset of animal tissues, cells or component parts which may in certain instances be found in blood, mucus, lymphatic fluid, synovial fluid, cerebrospinal fluid, saliva, bronchoalveolar lavage, amniotic fluid, amniotic cord blood, urine, vaginal fluid and semen. As such, a "biological sample" refers to both the native organism or a subset of its tissues as well as to a homogenate, lysate or extract prepared from the organism or a subset of its tissues, including but not limited to, for example, plasma, serum, spinal fluid, lymph fluid, sections of the skin, respiratory, gastrointestinal, cardiovascular, and genitourinary tracts, tears, saliva, milk, blood cells, tumors, organs. Biological samples may be any type of organismic tissue, including both healthy and diseased tissue (e.g., cancerous, malignant, necrotic, etc.). In certain embodiments, the biological sample is a liquid sample, such as blood or derivative thereof, e.g., plasma, tears, urine, semen, etc., where in some instances the sample is a blood sample, including whole blood, such as blood obtained from venipuncture or fingerstick (where the blood may or may not be combined with any reagents prior to assay, such as preservatives, anticoagulants, etc.).

In certain embodiments the source of the sample is a "mammal" or "mammalian", where these terms are used broadly to describe organisms which are within the class Mammalia, including the orders carnivore (e.g., dogs and cats), Rodentia (e.g., mice, guinea pigs, and rats), and primates (e.g., humans, chimpanzees, and monkeys). In some instances, the subjects are humans. The methods may be applied to samples obtained from human subjects of both genders and at any stage of development (i.e., neonates, infant, juvenile, adolescent, adult), where in certain embodiments the human subject is a juvenile, adolescent or adult. While the present disclosure may be applied to samples from a human subject, it is to be understood that the methods may also be carried-out on samples from other animal subjects (that is, in "non-human subjects") such as, but not limited to, birds, mice, rats, dogs, cats, livestock and horses.

Cells of interest may be targeted for characterized according to a variety of parameters, such as a phenotypic characteristic identified via the attachment of a particular fluorescent label to cells of interest. In some embodiments, the system is configured to deflect analyzed droplets that are determined to include a target cell. A variety of cells may be characterized using the subject methods. Target cells of interest include, but are not limited to, stem cells, T cells, dendritic cells, B Cells, granulocytes, leukemia cells, lymphoma cells, virus cells (e.g., HIV cells), NK cells, macrophages, monocytes, fibroblasts, epithelial cells, endothelial cells, and erythroid cells. Target cells of interest include cells that have a convenient cell surface marker or antigen that may be captured or labelled by a convenient affinity agent or conjugate thereof. For example, the target cell may include a cell surface antigen such as CD11b, CD123, CD14, CD15, CD16, CD19, CD193, CD2, CD25, CD27, CD3, CD335, CD36, CD4, CD43, CD45RO, CD56, CD61, CD7, CD8, CD34, CD1c, CD23, CD304, CD235a, T cell receptor alpha/beta, T cell receptor gamma/delta, CD253, CD95, CD20, CD105, CD117, CD120b, Notch4, Lgr5 (N-Terminal), SSEA-3, TRA-1-60 Antigen, Disialoganglioside GD2 and CD71. In some embodiments, the target cell is selected from HIV containing cell, a Treg cell, an antigen-specific T -cell populations, tumor cells or hematopoietic progenitor cells (CD34+) from whole blood, bone marrow or cord blood.

In practicing the subject methods, an amount of an initial fluidic sample is injected into the flow cytometer. The amount of sample injected into the particle sorting module may vary, for example, ranging from 0.001 mL to 1000 mL, such as from 0.005 mL to 900 mL, such as from 0.01 mL to 800 mL, such as from 0.05 mL to 700 mL, such as from 0.1 mL to 600 mL, such as from 0.5 mL to 500 mL, such as from 1 mL to 400 mL, such as from 2 mL to 300 mL and including from 5 mL to 100 mL of sample.

Methods according to embodiments of the present disclosure include counting and optionally sorting labeled particles (e.g., target cells) in a sample. In practicing the subject methods, the fluidic sample including the particles is first introduced into a flow nozzle of the system. Upon exit from the flow nozzle, the particles are passed substantially one at a time through the sample interrogation region where each of the particles is irradiated to a source of light and measurements of light scatter parameters and, in some instances, fluorescent emissions as desired (e.g., two or more light scatter parameters and measurements of one or more fluorescent emissions) are separately recorded for each particle. Depending on the properties of the flow stream being interrogated, 0.001 mm or more of the flow stream may be irradiated with light, such as 0.005 mm or more, such as 0.01 mm or more, such as 0.05 mm or more, such as 0.1 mm or more, such as 0.5 mm or more and including 1 mm or more of the flow stream may be irradiated with light. In certain embodiments, methods include irradiating a planar cross-section of the flow stream in the sample interrogation region, such as with a laser (as described above). In other embodiments, methods include irradiating a predetermined length of the flow stream in the sample interrogation region, such as corresponding to the irradiation profile of a diffuse laser beam or lamp.

In certain embodiments, methods include irradiating the flow stream at or near the flow cell nozzle orifice. For example, methods may include irradiating the flow stream at a position about 0.001 mm or more from the nozzle orifice, such as 0.005 mm or more, such as 0.01 mm or more, such as 0.05 mm or more, such as 0.1 mm or more, such as 0.5 mm or more and including 1 mm or more from the nozzle orifice. In certain embodiments, methods include irradiating the flow stream immediately adjacent to the flow cell nozzle orifice.

In embodiments of the method, detectors, such as photomultiplier tubes (PMT), are used to record light that passes through each particle (in certain cases referred to as forward light scatter), light that is reflected orthogonal to the direction of the flow of the particles through the sensing region (in some cases referred to as orthogonal or side light scatter) and fluorescent light emitted from the particles, if it is labeled with fluorescent marker(s), as the particle passes through the sensing region and is illuminated by the energy source. Each of forward light scatter (FSC), side-scatter (SSC), and fluorescence emissions include a separate parameter for each particle (or each "event"). Thus, for example, two, three or four parameters can be collected (and recorded) from a particle labeled with two different fluorescence markers. The data recorded for each particle is analyzed in real time or stored in a data storage and analysis means, such as a computer, as desired.

In certain embodiments, the particles are detected and uniquely identified by exposing the particles to excitation light and measuring the fluorescence of each particle in one or more detection channels, as desired. Fluorescence emitted in detection channels used to identify the particles and binding complexes associated therewith may be measured following excitation with a single light source, or may be measured separately following excitation with distinct light sources. If separate excitation light sources are used to excite the particle labels, the labels may be selected such that all the labels are excitable by each of the excitation light sources used.

Methods in certain embodiments also include data acquisition, analysis and recording, such as with a computer, wherein multiple data channels record data from each detector for the light scatter and fluorescence emitted by each particle as it passes through the sample interrogation region of the particle sorting module. In these embodiments, analysis includes classifying and counting particles such that each particle is present as a set of digitized parameter values. The subject systems may be set to trigger on a selected parameter in order to distinguish the particles of interest from background and noise. "Trigger" refers to a preset threshold for detection of a parameter and may be used as a means for detecting passage of a particle through the light source. Detection of an event that exceeds the threshold for the selected parameter triggers acquisition of light scatter and fluorescence data for the particle. Data is not acquired for particles or other components in the medium being assayed which cause a response below the threshold. The trigger parameter may be the detection of forward-scattered light caused by passage of a particle through the light beam. The flow cytometer then detects and collects the light scatter and fluorescence data for the particle.

A particular subpopulation of interest is then further analyzed by "gating" based on the data collected for the entire population. To select an appropriate gate, the data is plotted so as to obtain the best separation of subpopulations possible. This procedure may be performed by plotting forward light scatter (FSC) vs. side (i.e., orthogonal) light scatter (SSC) on a two dimensional dot plot. A subpopulation of particles is then selected (i.e., those cells within the gate) and particles that are not within the gate are excluded. Where desired, the gate may be selected by drawing a line around the desired subpopulation using a cursor on a computer screen. Only those particles within the gate are then further analyzed by plotting the other parameters for these particles, such as fluorescence. Where desired, the above analysis may be configured to yield counts of the particles of interest in the sample.

Methods of interest may further include employing particles in research, laboratory testing, or therapy. In some embodiments, the subject methods include obtaining individual cells prepared from a target fluidic or tissue biological sample. For example, the subject methods include obtaining cells from fluidic or tissue samples to be used as a research or diagnostic specimen for diseases such as cancer. Likewise, the subject methods include obtaining cells from fluidic or tissue samples to be used in therapy. A cell therapy protocol is a protocol in which viable cellular material including, e.g., cells and tissues, may be prepared and introduced into a subject as a therapeutic treatment. Conditions that may be treated by the administration of the flow cytometrically sorted sample include, but are not limited to, blood disorders, immune system disorders, organ damage, etc.

A typical cell therapy protocol may include the following steps: sample collection, cell isolation, genetic modification, culture, and expansion in vitro, cell harvesting, sample volume reduction and washing, bio-preservation, storage, and introduction of cells into a subject. The protocol may begin with the collection of viable cells and tissues from source tissues of a subject to produce a sample of cells and/or tissues. The sample may be collected via any suitable procedure that includes, e.g., administering a cell mobilizing agent to a subject, drawing blood from a subject, removing bone marrow from a subject, etc. After collecting the sample, cell enrichment may occur via several methods including, e.g., centrifugation based methods, filter based methods, elutriation, magnetic separation methods, fluorescence-activated cell sorting (FACS), and the like. In some cases, the enriched cells may be genetically modified by any convenient method, e.g., nuclease mediated gene editing. The genetically modified cells can be cultured, activated, and expanded in vitro. In some cases, the cells are preserved, e.g., cryopreserved, and stored for future use where the cells are thawed and then administered to a patient, e.g., the cells may be infused in the patient.

## SYSTEMS

Aspects of the disclosure also include systems. Systems of interest include memory operably coupled to a processor, wherein the memory comprises instructions stored thereon, which when executed by the processor, cause the processor to carry out methods of the disclosure, that is, apply a regression model to determine a relationship between an initial set of analyte features and a cluster criterion, generate a sparse set from at most a portion of the initial set of the analyte features based on the relationship, generate a classification model based on the sparse set, and apply the classification model to classify analyte data into the clusters.

Systems may include a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor, or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as Java, Perl, C++, Python, other high level or low level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques. In some embodiments, the processor includes analog electronics which provide feedback control, such as for example negative feedback control.

The system memory may be any of a variety of known or future memory storage devices. Examples include any commonly available random access memory (RAM), magnetic medium such as a resident hard disk or tape, an optical medium such as a read and write compact disc, flash memory devices, or other memory storage device. The memory storage device may be any of a variety of known or future devices, including a compact disk drive, a tape drive, or a diskette drive. Such types of memory storage devices typically read from, and/or write to, a program storage medium (not shown) such as a compact disk. Any of these program storage media, or others now in use or that may later be developed, may be considered a computer program product. As will be appreciated, these program storage media typically store a computer software program and/or data. Computer software programs, also called computer control logic, typically are stored in system memory and/or the program storage device used in conjunction with the memory storage device.

In some embodiments, a computer program product is described comprising a computer usable medium having control logic (computer software program, including program code) stored therein. The control logic, when executed by the processor the computer, causes the processor to perform functions described herein. In other embodiments, some functions are implemented primarily in hardware using, for example, a hardware state machine. Implementation of the hardware state machine so as to perform the functions described herein will be apparent to those skilled in the relevant arts.

The subject programmable logic may be implemented in any of a variety of devices such as specifically programmed event processing computers, wireless communication devices, integrated circuit devices, or the like. In some embodiments, the programable logic may be executed by a specifically programmed processor, which may include one or more processors, such as one or more digital signal processors (DSPs), configurable microprocessors, application specific integrated circuits (ASICs), field programmable gate arrays (FPGAs), or other equivalent integrated or discrete logic circuitry. A combination of computing devices, e.g., a combination of a DSP and a microprocessor, a plurality of microprocessors, one or more microprocessors in conjunction with a DSP core, or any other such configuration in at least partial data connectivity may implement one or more of the features described herein.

Memory may be any suitable device in which the processor can store and retrieve data, such as magnetic, optical, or solid-state storage devices (including magnetic or optical disks or tape or RAM, or any other suitable device, either fixed or portable). The processor may include a general-purpose digital microprocessor suitably programmed from a computer readable medium carrying necessary program code. Programming can be provided remotely to processor through a communication channel, or previously saved in a computer program product such as memory or some other portable or fixed computer readable storage medium using any of those devices in connection with memory. For example, a magnetic or optical disk may carry the programming, and can be read by a disk writer/reader. Systems of the disclosure also include programming, e.g., in the form of computer program products, algorithms for use in practicing the methods as described above. Programming according to the present disclosure can be recorded on computer readable media, e.g., any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; portable flash drive; and hybrids of these categories such as magnetic/optical storage media.

The processor may also have access to a communication channel to communicate with a user at a remote location. By remote location is meant the user is not directly in contact with the system and relays input information to an input manager from an external device, such as a computer connected to a Wide Area Network ("WAN"), telephone network, satellite network, or any other suitable communication channel, including a mobile telephone (i.e., smartphone).

In some embodiments, systems according to the present disclosure may be configured to include a communication interface. In some embodiments, the communication interface includes a receiver and/or transmitter for communicating with a network and/or another device. The communication interface can be configured for wired or wireless communication, including, but not limited to, radio frequency (RF) communication (e.g., Radio-Frequency Identification (RFID), Zigbee communication protocols, Wi-Fi, infrared, wireless Universal Serial Bus (USB), Ultra Wide Band (UWB), Bluetooth® communication protocols, and cellular communication, such as code division multiple access (CDMA) or Global System for Mobile communications (GSM).

In one embodiment, the communication interface is configured to include one or more communication ports, e.g., physical ports or interfaces such as a USB port, a USB-C port, an RS-232 port, or any other suitable electrical connection port to allow data communication between the subject systems and other external devices such as a computer terminal (for example, at a physician's office or in hospital environment) that is configured for similar complementary data communication.

In one embodiment, the communication interface is configured for infrared communication, Bluetooth® communication, or any other suitable wireless communication protocol to enable the subject systems to communicate with other devices such as computer terminals and/or networks, communication enabled mobile telephones, personal digital assistants, or any other communication devices which the user may use in conjunction.

In one embodiment, the communication interface is configured to provide a connection for data transfer utilizing Internet Protocol (IP) through a cell phone network, Short Message Service (SMS), wireless connection to a personal computer (PC) on a Local Area Network (LAN) which is connected to the internet, or Wi-Fi connection to the internet at a Wi-Fi hotspot.

In one embodiment, the subject systems are configured to wirelessly communicate with a server device via the communication interface, e.g., using a common standard such as 802.11 or Bluetooth® RF protocol, or an IrDA infrared protocol. The server device may be another portable device, such as a smart phone, Personal Digital Assistant (PDA) or notebook computer; or a larger device such as a desktop computer, appliance, etc. In some embodiments, the server device has a display, such as a liquid crystal display (LCD), as well as an input device, such as buttons, a keyboard, mouse or touch-screen.

In some embodiments, the communication interface is configured to automatically or semi-automatically communicate data stored in the subject systems, e.g., in an optional data storage unit, with a network or server device using one or more of the communication protocols and/or mechanisms described above.

Output controllers may include controllers for any of a variety of known display devices for presenting information to a user, whether a human or a machine, whether local or remote. If one of the display devices provides visual information, this information typically may be logically and/or physically organized as an array of picture elements. A graphical user interface (GUI) controller may include any of a variety of known or future software programs for providing graphical input and output interfaces between the system and a user, and for processing user inputs. The functional elements of the computer may communicate with each other via system bus. Some of these communications may be accomplished in alternative embodiments using network or other types of remote communications. The output manager may also provide information generated by the processing module to a user at a remote location, e.g., over the Internet, phone or satellite network, in accordance with known techniques. The presentation of data by the output manager may be implemented in accordance with a variety of known techniques. As some examples, data may include SQL, HTML or XML documents, email or other files, or data in other forms. The data may include Internet URL addresses so that a user may retrieve additional SQL, HTML, XML, or other documents or data from remote sources. The one or more platforms present in the subject systems may be any type of known computer platform or a type to be developed in the future, although they typically will be of a class of computer commonly referred to as servers. However, they may also be a main-frame computer, a workstation, or other computer type. They may be connected via any known or future type of cabling or other communication system including wireless systems, either networked or otherwise. They may be co-located or they may be physically separated. Various operating systems may be employed on any of the computer platforms, possibly depending on the type and/or make of computer platform chosen. Appropriate operating systems include Windows® NT®, Windows® XP, Windows® 7, Windows® 8, Windows® 10, iOS®, macOS®, Linux®, Ubuntu®, Fedora®, OS/400®, i5/OS®, IBM i®, Android™, SGI IRIX®, Oracle Solaris® and others.

In embodiments, systems further comprise a flow cytometer operably connected to the processor. The subject flow cytometers generally include a flow cell. Flow cells of interest include a cuvette configured to transport particles in a flow stream. As discussed herein, a "flow cell" is described in its conventional sense to refer to a component containing a flow channel for a liquid flow stream for transporting particles in a sheath fluid. Cuvettes of interest have a passage (i.e., flow channel) running therethrough. The flow stream for which the flow channel is configured may include a liquid sample injected from a sample tube. In certain instances, the flow cell includes a light-accessible flow channel. The cuvette may be comprised of, e.g., quartz, glass, clear plastic, and the like. In some embodiments, cuvettes are formed from silica, such as fused silica. In some cases, the flow cell is configured for irradiation with light from a light source at one or more interrogation points. The "interrogation point" discussed herein refers to a region within the flow cell in which the particle is irradiated by light from the light source, e.g., for analysis. The size of the interrogation point may vary as desired. For example, where 0 μm represents the optical axis of light emitted by the light source, the interrogation point may range from -50 μm to 50 μm, such as -25 μm to 40 μm, and including -15 μm to 30 μm. Depending on certain considerations (e.g., the number and arrangement of lasers), multiple irradiation points may exist within the flow cells.

In some embodiments, the flow cell includes, or is configured for use with, a sample injection port configured to provide a sample to the flow cell. In embodiments, the sample injection system is configured to provide suitable flow of sample to the flow cell inner chamber (i.e., flow channel). Depending on the desired characteristics of the flow stream, the rate of sample conveyed to the flow cell chamber by the sample injection port may be1 μL/min or more, such as 2 μL/min or more, such as 3 μL/min or more, such as 5 μL/min or more, such as 10 μL/min or more, such as 15 μL/min or more, such as 25 μL/min or more, such as 50 μL/min or more and including 100 μL/min or more, where in some instances the rate of sample conveyed to the flow cell chamber by the sample injection port is 1 μL/sec or more, such as 2 μL/sec or more, such as 3 μL/sec or more, such as 5 μL/sec or more, such as 10 μL/sec or more, such as 15 μL/sec or more, such as 25 μL/sec or more, such as 50 μL/sec or more and including 100 μL/sec or more.

The sample injection port may be an orifice positioned in a wall of the inner chamber or may be a conduit positioned at the proximal end of the inner chamber. Where the sample injection port is an orifice positioned in a wall of the inner chamber, the sample injection port orifice may be any suitable shape where cross-sectional shapes of interest include, but are not limited to: rectilinear cross-sectional shapes, e.g., squares, rectangles, trapezoids, triangles, hexagons, etc., curvilinear cross-sectional shapes, e.g., circles, ovals, etc., as well as irregular shapes, e.g., a parabolic bottom portion coupled to a planar top portion. In certain embodiments, the sample injection port has a circular orifice. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

In certain instances, the sample injection port is a conduit positioned at a proximal end of the flow cell inner chamber. For example, the sample injection port may be a conduit positioned to have the orifice of the sample injection port in line with the flow cell orifice. Where the sample injection port is a conduit positioned in line with the flow cell orifice, the cross-sectional shape of the sample injection tube may be any suitable shape where cross-sectional shapes of interest include, but are not limited to: rectilinear cross-sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The orifice of the conduit may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm. The shape of the tip of the sample injection port may be the same or different from the cross-section shape of the sample injection tube. For example, the orifice of the sample injection port may include a beveled tip having a bevel angle ranging from 1° to 10°, such as from 2° to 9°, such as from 3° to 8°, such as from 4° to 7° and including a bevel angle of 5°.

In some embodiments, the flow cell also includes a sheath fluid injection port configured to provide a sheath fluid to the flow cell. In embodiments, the sheath fluid injection system is configured to provide a flow of sheath fluid to the flow cell inner chamber, for example in conjunction with the sample to produce a laminated flow stream of sheath fluid surrounding the sample flow stream. Depending on the desired characteristics of the flow stream, the rate of sheath fluid conveyed to the flow cell chamber by the may be 25 μL/sec or more, such as 50 μL/sec or more, such as 75 μL/sec or more, such as 100 μL/sec or more, such as 250 μL/sec or more, such as 500 μL/sec or more, such as 750 μL/sec or more, such as 1000 μL/sec or more and including 2500 μL/sec or more.

In some embodiments, the sheath fluid injection port is an orifice positioned in a wall of the inner chamber. The sheath fluid injection port orifice may be any suitable shape where cross-sectional shapes of interest include, but are not limited to: rectilinear cross-sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The size of the sheath fluid injection port orifice may vary depending on shape, in certain instances, having an opening ranging from 0.1 mm to 5.0 mm, e.g., 0.2 mm to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

Flow cytometers of the present disclosure include a light source configured to irradiate the particles in the flow stream at an interrogation point within the flow cell. The number of light sources in the flow cytometers may vary. In some embodiments, flow cytometers include a single light source. Alternatively, flow cytometers may in some instances include a plurality of light sources. In some such instances, the number of light sources ranges from 2 to 10, such as 2 to 5, and including 2 to 4. Any convenient light source may be employed as the light source described herein. In some embodiments, the light source is a laser. In embodiments, the laser may be any convenient laser, such as a continuous wave laser. For example, the laser may be a diode laser, such as an ultraviolet diode laser, a visible diode laser and a near-infrared diode laser. In other embodiments, the laser may be a helium-neon (HeNe) laser. In some instances, the laser is a gas laser, such as a helium-neon laser, argon laser, krypton laser, xenon laser, nitrogen laser, $CO_2$ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In other instances, the subject flow cytometers include a dye laser, such as a stilbene, coumarin or rhodamine laser. In yet other instances, lasers of interest include a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, the subject flow cytometers include a solid-state laser, such as a ruby laser, an Nd:YAG laser, NdCrYAG laser, Er:YAG laser, Nd:YLF laser, Nd:$YVO_4$ laser, Nd:$YCa_4O(BO_3)_3$ laser, Nd:YCOB laser, titanium sapphire laser, thulim YAG laser, ytterbium YAG laser, $ytterbium_2O_3$ laser or cerium doped lasers and combinations thereof.

Laser light sources according to certain embodiments may also include one or more optical adjustment components. In certain embodiments, the optical adjustment component is located between the light source and the flow cell, and may include any device that is capable of changing the spatial width of irradiation or some other characteristic of irradiation from the light source, such as for example, irradiation direction, wavelength, beam width, beam intensity and focal spot. Optical adjustment protocols may include any convenient device which adjusts one or more characteristics of the light source, including but not limited to lenses, mirrors, filters, fiber optics, wavelength separators, pinholes, slits, collimating protocols and combinations thereof. In certain embodiments, flow cytometers of interest include one or more focusing lenses. The focusing lens, in one example, may be a de-magnifying lens. In still other embodiments, flow cytometers of interest include fiber optics.

The light source may be positioned any suitable distance from the flow cell, such as where the light source and the flow cell are separated by 0.005 mm or more, such as 0.01 mm or more, such as 0.05 mm or more, such as 0.1 mm or more, such as 0.5 mm or more, such as 1 mm or more, such as 5 mm or more, such as 10 mm or more, such as 25 mm or more and including at a distance of 100 mm or more. In addition, the light source may be positioned at any suitable angle relative to the flow cell, such as at an angle ranging from 10 degrees to 90 degrees, such as from 15 degrees to 85 degrees, such as from 20 degrees to 80 degrees, such as from 25 degrees to 75 degrees and including from 30 degrees to 60 degrees, for example at a 90 degree angle.

In some embodiments, light sources of interest include a plurality of lasers configured to provide laser light for discrete irradiation of the flow stream, such as 2 lasers or more, such as 3 lasers or more, such as 4 lasers or more, such as 5 lasers or more, such as 10 lasers or more, and including 15 lasers or more configured to provide laser light for discrete irradiation of the flow stream. Depending on the desired wavelengths of light for irradiating the flow stream, each laser may have a specific wavelength that varies from 200 nm to 1500 nm, such as from 250 nm to 1250 nm, such as from 300 nm to 1000 nm, such as from 350 nm to 900 nm and including from 400 nm to 800 nm. In certain embodiments, lasers of interest may include one or more of a 405 nm laser, a 488 nm laser, a 561 nm laser and a 635 nm laser.

In certain embodiments, the light source is a light beam generator that is configured to generate two or more beams of frequency shifted light. In some instances, the light beam generator includes a laser, a radiofrequency generator configured to apply radiofrequency drive signals to an acousto-optic device to generate two or more angularly deflected laser beams. In these embodiments, the laser may be a pulsed lasers or continuous wave laser. For example lasers in light beam generators of interest may be a gas laser, such as a helium-neon laser, argon laser, krypton laser, xenon laser, nitrogen laser, CO2 laser, CO laser, argon-fluorine (ArF) excimer laser, krypton-fluorine (KrF) excimer laser, xenon chlorine (XeCl) excimer laser or xenon-fluorine (XeF) excimer laser or a combination thereof; a dye laser, such as a stilbene, coumarin or rhodamine laser; a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof; a solid-state laser, such as a ruby laser, an Nd:YAG laser, NdCrYAG laser, Er:YAG laser, Nd:YLF laser, $Nd:YVO_4$ laser, $Nd:YCa_4O(BO_3)_3$ laser, Nd:YCOB laser, titanium sapphire laser, thulim YAG laser, ytterbium YAG laser, $ytterbium_2O_3$ laser or cerium doped lasers and combinations thereof.

The acousto-optic device may be any convenient acousto-optic protocol configured to frequency shift laser light using applied acoustic waves. In certain embodiments, the acousto-optic device is an acousto-optic deflector. The acousto-optic device in the subject system is configured to generate angularly deflected laser beams from the light from the laser and the applied radiofrequency drive signals. The radiofrequency drive signals may be applied to the acousto-optic device with any suitable radiofrequency drive signal source, such as a direct digital synthesizer (DDS), arbitrary waveform generator (AWG), or electrical pulse generator.

In embodiments, a controller is configured to apply radiofrequency drive signals to the acousto-optic device to produce the desired number of angularly deflected laser beams in the output laser beam, such as being configured to apply 3 or more radiofrequency drive signals, such as 4 or more radiofrequency drive signals, such as 5 or more radiofrequency drive signals, such as 6 or more radiofrequency drive signals, such as 7 or more radiofrequency drive signals, such as 8 or more radiofrequency drive signals, such as 9 or more radiofrequency drive signals, such as 10 or more radiofrequency drive signals, such as 15 or more radiofrequency drive signals, such as 25 or more radiofrequency drive signals, such as 50 or more radiofrequency drive signals and including being configured to apply 100 or more radiofrequency drive signals.

In some instances, to produce an intensity profile of the angularly deflected laser beams in the output laser beam, the controller is configured to apply radiofrequency drive signals having an amplitude that varies such as from about 0.001 V to about 500 V, such as from about 0.005 V to about 400 V, such as from about 0.01 V to about 300 V, such as from about 0.05 V to about 200 V, such as from about 0.1 V to about 100 V, such as from about 0.5 V to about 75 V, such as from about 1 V to 50 V, such as from about 2 V to 40 V, such as from 3 V to about 30 V and including from about 5 V to about 25 V. Each applied radiofrequency drive signal has, in some embodiments, a frequency of from about 0.001 MHz to about 500 MHz, such as from about 0.005 MHz to about 400 MHz, such as from about 0.01 MHz to about 300 MHz, such as from about 0.05 MHz to about 200 MHz, such as from about 0.1 MHz to about 100 MHz, such as from about 0.5 MHz to about 90 MHz, such as from about 1 MHz to about 75 MHz, such as from about 2 MHz to about 70 MHz, such as from about 3 MHz to about 65 MHz, such as from about 4 MHz to about 60 MHz and including from about 5 MHz to about 50 MHz.

In certain embodiments, the controller has a processor having memory operably coupled to the processor such that the memory includes instructions stored thereon, which when executed by the processor, cause the processor to produce an output laser beam with angularly deflected laser beams having a desired intensity profile. For example, the memory may include instructions to produce two or more angularly deflected laser beams with the same intensities, such as 3 or more, such as 4 or more, such as 5 or more, such as 10 or more, such as 25 or more, such as 50 or more and including memory may include instructions to produce 100 or more angularly deflected laser beams with the same intensities. In other embodiments, the memory may include instructions to produce two or more angularly deflected laser beams with different intensities, such as 3 or more, such as 4 or more, such as 5 or more, such as 10 or more, such as 25 or more, such as 50 or more and including memory may include instructions to produce 100 or more angularly deflected laser beams with different intensities.

In certain embodiments, the controller has a processor having memory operably coupled to the processor such that the memory includes instructions stored thereon, which when executed by the processor, cause the processor to produce an output laser beam having increasing intensity from the edges to the center of the output laser beam along the horizontal axis. In these instances, the intensity of the angularly deflected laser beam at the center of the output beam may range from 0.1 % to about 99% of the intensity of the angularly deflected laser beams at the edge of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the edge of the output laser beam along the horizontal axis. In other embodiments, the controller has a processor having memory operably coupled to the processor such that the memory includes instructions stored thereon, which when executed by the processor, cause the processor to produce an output laser beam having an increasing intensity from the edges to the center of the output laser beam along the horizontal axis. In these instances, the intensity of the angularly deflected laser beam at the edges of the output beam may range from 0.1% to about 99% of the intensity of the angularly deflected laser beams at the center of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the center of the output laser beam along the horizontal axis. In yet other embodiments, the controller has a processor having memory operably coupled to the processor such that the memory includes instructions stored thereon, which when executed by the processor, cause the processor to produce an output laser beam having an intensity profile with a Gaussian distribution along the horizontal axis. In still other embodiments, the controller has a processor having memory operably coupled to the processor such that the memory includes instructions stored thereon, which when executed by the processor, cause the processor to produce an output laser beam having a top hat intensity profile along the horizontal axis.

In embodiments, light beam generators of interest may be configured to produce angularly deflected laser beams in the output laser beam that are spatially separated. Depending on the applied radiofrequency drive signals and desired irradiation profile of the output laser beam, the angularly deflected laser beams may be separated by 0.001 μm or more, such as by 0.005 μm or more, such as by 0.01 μm or more, such as by 0.05 μm or more, such as by 0.1 μm or more, such as by 0.5 μm or more, such as by 1 μm or more, such as by 5 μm or more, such as by 10 μm or more, such as by 100 μm or more, such as by 500 μm or more, such as by 1000 μm or more and including by 5000 μm or more. In some embodiments, systems are configured to produce angularly deflected laser beams in the output laser beam that overlap, such as with an adjacent angularly deflected laser beam along a horizontal axis of the output laser beam. The overlap between adjacent angularly deflected laser beams (such as overlap of beam spots) may be an overlap of 0.001 μm or more, such as an overlap of 0.005 μm or more, such as an overlap of 0.01 μm or more, such as an overlap of 0.05 μm or more, such as an overlap of 0.1 μm or more, such as an overlap of 0.5 μm or more, such as an overlap of 1 μm or more, such as an overlap of 5 μm or more, such as an overlap of 10 μm or more and including an overlap of 100 μm or more.

In certain instances, light beam generators configured to generate two or more beams of frequency shifted light include laser excitation modules as described in U.S. Patent Nos. 9,423,353; 9,784,661 and 10,006,852 and U.S. Patent Publication Nos. 2017/0133857 and 2017/0350803, the disclosures of which are herein incorporated by reference.

In addition, flow cytometers include a detector configured to collect light emitted by the irradiated particles. The light detectors are configured to detect particle-modulated light conveyed by the fiber optic light collection elements and generate signals based on a characteristic of that light (e.g., intensity). For example, the one or more particle-modulated light detector(s) may include one or more side-scattered light detectors for detecting side-scatter wavelengths of light (i.e., light refracted and reflected from the surfaces and internal structures of the particle). In some embodiments, flow cytometers include a single side-scattered light detector. In other embodiments, flow cytometers include multiple side-scattered light detectors, such as 2 or more, such as 3 or more, such as 4 or more, and including 5 or more.

Any convenient detector for detecting collected light may be used in the side-scattered light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 $cm^2$ to 10 $cm^2$, such as from 0.05 $cm^2$ to 9 $cm^2$, such as from 0.1 $cm^2$ to 8 $cm^2$, such as from 0.5 $cm^2$ to 7 $cm^2$ and including from 1 $cm^2$ to 5 $cm^2$.

In embodiments, the subject flow cytometers also include a fluorescent light detector configured to detect one or more fluorescent wavelengths of light. In other embodiments, flow cytometers include multiple fluorescent light detectors such as 2 or more, such as 3 or more, such as 4 or more, 5 or more, 10 or more, 15 or more, and including 20 or more.

Any convenient detector for detecting collected light may be used in the fluorescent light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 $cm^2$ to 10 $cm^2$, such as from 0.05 $cm^2$ to 9 $cm^2$, such as from, such as from 0.1 $cm^2$ to 8 $cm^2$, such as from 0.5 $cm^2$ to 7 $cm^2$ and including from 1 $cm^2$ to 5 $cm^2$.

Where the subject flow cytometers include multiple fluorescent light detectors, each fluorescent light detector may be the same, or the collection of fluorescent light detectors may be a combination of different types of detectors. For example, where the subject flow cytometers include two fluorescent light detectors, in some embodiments the first fluorescent light detector is a CCD-type device and the second fluorescent light detector (or imaging sensor) is a CMOS-type device. In other embodiments, both the first and second fluorescent light detectors are CCD-type devices. In yet other embodiments, both the first and second fluorescent light detectors are CMOS-type devices. In still other embodiments, the first fluorescent light detector is a CCD-type device and the second fluorescent light detector is a photomultiplier tube (PMT). In still other embodiments, the first fluorescent light detector is a CMOS-type device and the second fluorescent light detector is a photomultiplier tube. In yet other embodiments, both the first and second fluorescent light detectors are photomultiplier tubes.

In embodiments of the present disclosure, fluorescent light detectors of interest are configured to measure collected light at one or more wavelengths, such as at 2 or more wavelengths, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring light emitted by a sample in the flow stream at 400 or more different wavelengths. In some embodiments, 2 or more detectors in the modules as described herein are configured to measure the same or overlapping wavelengths of collected light.

In some embodiments, fluorescent light detectors of interest are configured to measure collected light over a range of wavelengths (e.g., 200 nm - 1000 nm). In certain embodiments, detectors of interest are configured to collect spectra of light over a range of wavelengths. For example, flow cytometers may include one or more detectors configured to collect spectra of light over one or more of the wavelength ranges of 200 nm - 1000 nm. In yet other embodiments, detectors of interest are configured to measure light emitted by a sample in the flow stream at one or more specific wavelengths. For example, modules may include one or more detectors configured to measure light at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof. In certain embodiments, one or more detectors may be configured to be paired with specific fluorophores, such as those used with the sample in a fluorescence assay.

Flow cytometers may include any suitable mechanism(s) for providing sheath fluid and sample fluid to the sample fluid input coupler and sheath fluid input coupler. For example, the sample fluid input coupler may be fluidically connected to a sample fluid line (e.g., tubing) fluidically connected to a sample fluid reservoir. Similarly, the sheath fluid input coupler may be fluidically connected to a sheath fluid line fluidically connected to a sheath fluid reservoir. Similarly, flow cytometers may include any suitable mechanism(s) for managing waste from the flow stream. The fluidic output coupler may be fluidically connected to a waste line fluidically connected to a waste reservoir. Fluid management systems that may be adapted for use in the subject flow cytometers are provided in U.S. Patent Application Publication No. 2022/0341838, the disclosure of which is incorporated by reference herein in its entirety.

Suitable flow cytometry systems may include, but are not limited to those described in Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); Practical Flow Cytometry, 3rd ed., Wiley-Liss (1995); Virgo, et al. (2012) Ann Clin Biochem. Jan;49(pt 1): 17-28; Linden, et. al., Semin Throm Hemost. 2004 Oct;30(5):502-11; Alison, et al. J Pathol, 2010 Dec; 222(4):335-344; and Herbig, et al. (2007) Crit Rev Ther Drug Carrier Syst. 24(3):203-255; the disclosures of which are incorporated herein by reference. In certain instances, flow cytometry systems of interest include BD Biosciences FACSCanto™ flow cytometer, BD Biosciences FACSCanto™ II flow cytometer, BD Accuri™ flow cytometer, BD Accuri™ C6 Plus flow cytometer, BD Biosciences FACSCelesta™ flow cytometer, BD Biosciences FACSLyric™ flow cytometer, BD Biosciences FACSVerse™ flow cytometer, BD Biosciences FACSymphony™ flow cytometer, BD Biosciences LSRFortessa™ flow cytometer, BD Biosciences LSRFortessa™ X-20 flow cytometer, BD Biosciences FACSPresto™ flow cytometer, BD Biosciences FACSVia™ flow cytometer and BD Biosciences FACSCalibur™ cell sorter, a BD Biosciences FACSCount™ cell sorter, BD Biosciences FACSLyric™ cell sorter, BD Biosciences Via™ cell sorter, BD Biosciences Influx™ cell sorter, BD Biosciences Jazz™ cell sorter, BD Biosciences Aria™ cell sorter, BD Biosciences FACSAria™ II cell sorter, BD Biosciences FACSAria™ III cell sorter, BD Biosciences FACSAria™ Fusion cell sorter and BD Biosciences FACSMelody™ cell sorter, BD Biosciences FACSymphony™ S6 cell sorter, BD Biosciences FACSDiscover™ cell sorter, or the like.

In some embodiments, the subject systems are flow cytometric systems, such those described in U.S. Patent Nos. 10,663,476; 10,620,111; 10,613,017; 10,605,713; 10,585,031; 10,578,542; 10,578,469; 10,481,074; 10,302,545; 10,145,793; 10,113,967; 10,006,852; 9,952,076; 9,933,341; 9,726,527; 9,453,789; 9,200,334; 9,097,640; 9,095,494; 9,092,034; 8,975,595; 8,753,573; 8,233,146; 8,140,300; 7,544,326; 7,201,875; 7,129,505; 6,821,740; 6,813,017; 6,809,804; 6,372,506; 5,700,692; 5,643,796; 5,627,040; 5,620,842; 5,602,039; 4,987,086; 4,498,766; the disclosures of which are herein incorporated by reference in their entirety.

In some embodiments, the flow cytometer is configured as an imaging flow cytometer. For example, in certain instances, the subject systems are flow cytometry systems configured for imaging particles in a flow stream by fluorescence imaging using radiofrequency tagged emission (FIRE), such as those described in Diebold, et al. Nature Photonics Vol. 7(10); 806-810 (2013) as well as described in U.S. Patent Nos. 9,423,353; 9,784,661 and 10,006,852 and U.S. Patent Publication Nos. 2017/0133857 and 2017/0350803, the disclosures of which are herein incorporated by reference. In some embodiments where the flow cytometer is a particle sorter, the particle sorter is an image enabled particle sorter. Image enabled particle sorters are described in U.S. Provisional Patent Application Nos. 63/431,803 and 63/465,057; the disclosures of which are herein incorporated by reference in their entirety.

**FIG. 2** shows a system 200 for flow cytometry in accordance with an illustrative embodiment of the present disclosure. System 200 includes a laser 201 configured to irradiate particles 211 in flow stream 214 at interrogation point 215 within flow cell 210. While the example of **FIG. 2** shows a single laser, it is understood that multiple lasers could also be used. The laser beam from laser 201 is directed to focusing lens 202 which focuses the beam onto the portion of a fluid stream where particles 211 of a sample are located, within the flow cell 210. The flow cell 210 is part of a fluidics system which directs particles, typically one at a time, in a stream to the focused laser beam for interrogation. Alternatively, where the flow cytometer is a stream-in-air cytometer, a nozzle top may be employed.

As shown in **FIG. 2,** flow cell 210 is fluidically connected to sheath fluid reservoir 203 comprising a sheath fluid and sample fluid reservoir 204 comprising a sample fluid. Sheath fluid from sheath fluid reservoir 203 is provided to at least one sheath fluid injection port 208 via conduit (i.e., sheath fluid line) 207. In addition, sample fluid containing particles 211 from sample fluid reservoir 204 is provided to sample injection port 206 via conduit (i.e., sample fluid line) 205. Sample injection port 206 is fluidically connected to sample injector 213 (e.g., sample injection needle) which is configured to introduce particles 211 into the interior of flow cell 210. Particles 211 are hydrodynamically focused via sheath fluid entering from sheath fluid injection port 208 such that flow stream 214 forms downstream of tapered portion 212 of flow cell 210. Particles emitting at the distal end of flow cell 210 may be disposed of and/or collected via any suitable protocol. For example, depending on the type of flow cytometry being performed, particles may be collected at the distal end of flow cell 210, e.g., via a waste line. Alternatively, particles may be sorted.

The light from the laser beam(s) interacts with the particles 211 in the sample by diffraction, refraction, reflection, scattering, and absorption with re-emission at various different wavelengths depending on the characteristics of the particle such as its size, internal structure, and the presence of one or more fluorescent molecules attached to or naturally present on or in the particle. The fluorescence emissions as well as the diffracted light, refracted light, reflected light, and scattered light may be routed to one or more detectors. In particular, forward scattered light (FSC) is routed to forward-scattered light detector 223. The forward-scattered light detector 223 is positioned slightly off axis from the direct beam through the flow cell 210 and is configured to detect diffracted light, the excitation light that travels through or around the particle in mostly a forward direction. The intensity of the light detected by the forward-scattered light detector 223 is dependent on the overall size of the particle. The forward-scatter detector can include, e.g., a photodiode. Positioned between forward-scattered light detector 223 are optical filter 221a and scatter bar 222. Optical filter 221a may be configured to filter out at least one wavelength of non-FSC light, while scatter bar 222 may be configured to prevent the incident beam from laser 201 (i.e., non-scattered light) from being detected by forward-scattered light detector 223.

In addition, side-scattered light (SSC) is detected by side-scattered light detector 224. In other words, side-scattered light detector 224 is configured to detect refracted and reflected light from the surfaces and internal structures of the particles 211 that tend to increase with increasing particle complexity of structure. In the example of **FIG. 2,** flow cytometer 200 includes dichroic mirror 220a configured to reflect SSC light to side-scattered light detector 224 while passing non-SSC (e.g., fluorescent) light. Optical filter 221b is configured to prevent at least one wavelength of non-SSC light from being detected by side-scattered light detector 224. Also shown are fluorescent light detectors 225a-225c which are each configured to detect different wavelengths of fluorescent light. For example, dichroic mirror 220b may be configured to reflect fluorescent light (FL) corresponding to a first wavelength (or range of wavelengths) to fluorescent light detector 225a while passing other wavelengths of light. Optical filter 221c may be configured to prevent at least one wavelength of light that does not correspond to the first wavelength (or range of wavelengths) from being detected by fluorescent light detector 225a. Similarly, dichroic mirror 220c is configured to reflect FL light corresponding to a second wavelength (or range of wavelengths) to fluorescent light detector 225b while passing a third wavelength of light (or range of wavelengths) for detection by fluorescent light detector 225c. Optical filter 221d is configured to prevent at least one wavelength of light that does not correspond to the second wavelength (or range of wavelengths) from being detected by fluorescent light detector 225b. In addition, Optical filter 221e is configured to prevent at least one wavelength of light that does not correspond to the third wavelength (or range of wavelengths) from being detected by fluorescent light detector 225c.

One of skill in the art will recognize that a flow cytometer in accordance with an embodiment of the present disclosure is not limited to the flow cytometer depicted in **FIG. 2,** but can include any flow cytometer known in the art. For example, a flow cytometer may have any number of lasers, beam splitters, filters, and detectors at various wavelengths and in various different configurations. For example, while the embodiment of **FIG. 2** shows 3 fluorescent light detectors for illustrative purposes, it is understood that any suitable number of fluorescent light detectors may be employed.

In operation, cytometer operation is controlled by a controller/processor 290, and the measurement data from the detectors can be stored in the memory 295 and processed by the controller/processor 290. Although not shown explicitly, the controller/processor 290 is coupled to the detectors to receive the output signals therefrom, and may also be coupled to electrical and electromechanical components of the flow cytometer to control the laser 201, fluid flow parameters, and the like. Input/output (I/O) capabilities 297 may be provided also in the system. The memory 295, controller/processor 290, and I/O 297 may be entirely provided as an integral part of the flow cytometer. In such an embodiment, a display may also form part of the I/O capabilities 297 for presenting experimental data to users of the cytometer 200. Alternatively, some or all of the memory 295 and controller/processor 290 and I/O capabilities may be part of one or more external devices such as a general purpose computer. In some embodiments, some or all of the memory 295 and controller/processor 290 can be in wireless or wired communication with the cytometer 210. The controller/processor 290 in conjunction with the memory 295 and the I/O 297 can be configured to perform various functions related to the preparation and analysis of a flow cytometer experiment.

Different fluorescent molecules in a fluorochrome panel used for a flow cytometer experiment will emit light in their own characteristic wavelength bands. The particular fluorescent labels used for an experiment and their associated fluorescent emission bands may be selected to generally coincide with the filter windows of the detectors. The I/O 297 can be configured to receive data regarding a flow cytometer experiment having a panel of fluorescent labels and a plurality of cell populations having a plurality of markers, each cell population having a subset of the plurality of markers. The I/O 297 can also be configured to receive biological data assigning one or more markers to one or more cell populations, marker density data, emission spectrum data, data assigning labels to one or more markers, and cytometer configuration data. Flow cytometer experiment data, such as label spectral characteristics and flow cytometer configuration data can also be stored in the memory 295. The controller/processor 290 can be configured to evaluate one or more assignments of labels to markers.

In some embodiments, the subject systems are particle sorting systems that are configured to sort particles with an enclosed particle sorting module, such as those described in U.S. Patent Publication No. 2017/0299493, filed on March 28, 2017, the disclosure of which is incorporated herein by reference. In certain embodiments, particles (e.g., cells) of the sample are sorted using a sort decision module having a plurality of sort decision units, such as those described in U.S. Patent Publication No. 2020/0256781, filed on December 23, 2019, the disclosure of which is incorporated herein by reference. In some embodiments, systems for sorting components of a sample include a particle sorting module having deflection plates, such as described in U.S. Patent Publication No. 2017/0299493, filed on March 28, 2017, the disclosure of which is incorporated herein by reference.

In certain embodiments, systems are a fluorescence imaging using radiofrequency tagged emission image-enabled particle sorter, such as depicted in **FIG. 3.** Particle sorter 300 includes a light irradiation component 300a which includes light source 301 (e.g., 488 nm laser) which generates output beam of light 301a that is split with beamsplitter 302 into beams 302a and 302b. Light beam 302a is propagated through acousto-optic device (e.g., an acousto-optic deflector, AOD) 303 to generate an output beam 303a having one or more angularly deflected beams of light. In some instances, output beam 303a generated from acousto-optic device 303 includes a local oscillator beam and a plurality of radiofrequency comb beams. Light beam 302b is propagated through acousto-optic device (e.g., an acousto-optic deflector, AOD) 304 to generate an output beam 304a having one or more angularly deflected beams of light. In some instances, output beam 304a generated from acousto-optic device 304 includes a local oscillator beam and a plurality of radiofrequency comb beams. Output beams 303a and 304a generated from acousto-optic devices 303 and 304, respectively are combined with beamsplitter 305 to generate output beam 305a which is conveyed through an optical component 306 (e.g., an objective lens) to irradiate particles in flow cell 307. In certain embodiments, acousto-optic device 303 (AOD) splits a single laser beam into an array of beamlets, each having different optical frequency and angle. Second AOD 304 tunes the optical frequency of a reference beam, which is then overlapped with the array of beamlets at beam combiner 305. In certain embodiments, the light irradiation system having a light source and acousto-optic device can also include those described in Schraivogel, et al. ("High-speed fluorescence image-enabled cell sorting" Science (2022), 375 (6578): 315-320) and United States Patent Publication No. 2021/0404943, the disclosure of which is herein incorporated by reference.

Output beam 305a irradiates sample particles 308 propagating through flow cell 307 (e.g., with sheath fluid 309) at irradiation region 310. As shown in irradiation region 310, a plurality of beams (e.g., angularly deflected radiofrequency shifted beams of light depicted as dots across irradiation region 310) overlaps with a reference local oscillator beam (depicted as the shaded line across irradiation region 310). Due to their differing optical frequencies, the overlapping beams exhibit a beating behavior, which causes each beamlet to carry a sinusoidal modulation at a distinct frequency $f_{1-n}$.

Light from the irradiated sample is conveyed to light detection system 300b that includes a plurality of photodetectors. Light detection system 300b includes forward scattered light photodetector 311 for generating forward scatter images 311a and a side scattered light photodetector 312 for generating side scatter images 312a. Light detection system 300b also includes brightfield photodetector 313 for generating light loss images 313a. In some embodiments, forward scatter detector 311 and side scatter detector 312 are photodiodes (e.g., avalanche photodiodes, APDs). In some instances, brightfield photodetector 313 is a photomultiplier tube (PMT). Fluorescence from the irradiated sample is also detected with fluorescence photodetectors 314-317. In some instances, photodetectors 314-317 are photomultiplier tubes. Light from the irradiated sample is directed to the side scatter detection channel 312 and fluorescence detection channels 314-317 through beamsplitter 320. Light detection system 300b includes bandpass optical components 321, 322, 323 and 324 (e.g., dichroic mirrors) for propagating predetermined wavelength of light to photodetectors 314-317. In some instances, optical component 321 is a 534 nm/40 nm bandpass. In some instances, optical component 322 is a 586 nm/42 nm bandpass. In some instances, optical component 323 is a 700 nm/54 nm bandpass. In some instances, optical component 324 is a 783 nm/56 nm bandpass. The first number represents the center of a spectral band. The second number provides a range of the spectral band. Thus, a 510/20 filter extends 10 nm on each side of the center of the spectral band, or from 500 nm to 520 nm.

Data signals generated in response to light detected in scattered light detection channels 311 and 312, brightfield light detection channel 313 and fluorescence detection channels 314-317 are processed by real-time digital processing with processors 350 and 351. Images 311a-317a can be generated in each light detection channel based on the data signals generated in processors 350 and 351. Image-enabled sorting is performed in response to a sort signal generated in sort trigger 352. Sorting component 300c includes deflection plates 331 for deflecting particles into sample containers 332 or to waste stream 333. In some instances, sort component 300c is configured to sort particles with an enclosed particle sorting module, such as those described in U.S. Patent Publication No. 2017/0299493, filed on March 28, 2017, the disclosure of which is incorporated herein by reference. In certain embodiments, sorting component 300c includes a sort decision module having a plurality of sort decision units, such as those described in U.S. Patent Publication No. 2020/0256781, the disclosure of which is incorporated herein by reference.

In some embodiments, systems are particle analyzers where the particle analysis system 401 **(FIG. 4)** can be used to analyze and characterize particles, with or without physically sorting the particles into collection vessels. **FIG. 4** shows a functional block diagram of a particle analysis system for computational based sample analysis and particle characterization. In some embodiments, the particle analysis system 401 is a flow system. The particle analysis system 401 includes a fluidics system 402. The fluidics system 402 can include or be coupled with a sample tube 405 and a moving fluid column within the sample tube in which particles 403 (e.g. cells) of a sample move along a common sample path 409.

The particle analysis system 401 includes a detection system 404 configured to collect a signal from each particle as it passes one or more detection stations along the common sample path. A detection station 408 generally refers to a monitored area 407 of the common sample path. Detection can, in some implementations, include detecting light or one or more other properties of the particles 403 as they pass through a monitored area 407. In **FIG. 4,** one detection station 408 with one monitored area 407 is shown. Some implementations of the particle analysis system 401 can include multiple detection stations. Furthermore, some detection stations can monitor more than one area.

Each signal is assigned a signal value to form a data point for each particle. As described above, this data can be referred to as event data. The data point can be a multidimensional data point including values for respective properties measured for a particle. The detection system 404 is configured to collect a succession of such data points in a first-time interval.

The particle analysis system 401 can also include a control system 406. The control system 406 can include one or more processors, an amplitude control circuit and/or a frequency control circuit. The control system shown can be operationally associated with the fluidics system 402. The control system can be configured to generate a calculated signal frequency for at least a portion of the first-time interval based on a Poisson distribution and the number of data points collected by the detection system 404 during the first time interval. The control system 406 can be further configured to generate an experimental signal frequency based on the number of data points in the portion of the first time interval. The control system 406 can additionally compare the experimental signal frequency with that of a calculated signal frequency or a predetermined signal frequency.

**FIG. 5** shows a functional block diagram for one example of a particle analyzer control system, such as an analytics controller (i.e., processor) 500, for analyzing and displaying biological events. An analytics controller 500 can be configured to implement a variety of processes for controlling graphic display of biological events.

A particle analyzer or sorting system 502 can be configured to acquire biological event data. For example, a flow cytometer can generate flow cytometric event data. The particle analyzer 502 can be configured to provide biological event data to the analytics controller 500. A data communication channel can be included between the particle analyzer or sorting system 502 and the analytics controller 500. The biological event data can be provided to the analytics controller 500 via the data communication channel. Analytics controller 500 may be a processor configured to carry out methods of the invention, e.g., by applying a regression model to determine a relationship between an initial set of analyte features and a cluster criterion, generating a sparse set from at most a portion of the initial set of the analyte features based on the relationship, generating a classification model based on the sparse set, and applying the classification model to classify the analyte data into the clusters.

The analytics controller 500 can be configured to receive biological event data from the particle analyzer or sorting system 502. The biological event data received from the particle analyzer or sorting system 502 can include flow cytometric event data. The analytics controller 500 can be configured to provide a graphical display including a first plot of biological event data to a display device 506. The analytics controller 500 can be further configured to render a region of interest as a gate around a population of biological event data shown by the display device 506, overlaid upon the first plot, for example. In some embodiments, the gate can be a logical combination of one or more graphical regions of interest drawn upon a single parameter histogram or bivariate plot. In some embodiments, the display can be used to display particle parameters or saturated detector data.

The analytics controller 500 can be further configured to display the biological event data on the display device 506 within the gate differently from other events in the biological event data outside of the gate. For example, the analytics controller 500 can be configured to render the color of biological event data contained within the gate to be distinct from the color of biological event data outside of the gate. The display device 506 can be implemented as a monitor, a tablet computer, a smartphone, or other electronic device configured to present graphical interfaces.

The analytics controller 500 can be configured to receive a gate selection signal identifying the gate from a first input device. For example, the first input device can be implemented as a mouse 510. The mouse 510 can initiate a gate selection signal to the analytics controller 500 identifying the gate to be displayed on or manipulated via the display device 506 (e.g., by clicking on or in the desired gate when the cursor is positioned there). In some implementations, the first device can be implemented as the keyboard 508 or other means for providing an input signal to the analytics controller 500 such as a touchscreen, a stylus, an optical detector, or a voice recognition system. Some input devices can include multiple inputting functions. In such implementations, the inputting functions can each be considered an input device. For example, as shown in **FIG. 5,** the mouse 510 can include a right mouse button and a left mouse button, each of which can generate a triggering event.

The triggering event can cause the analytics controller 500 to alter the manner in which the data is displayed, which portions of the data is actually displayed on the display device 506, and/or provide input to further processing such as selection of a population of interest for particle sorting.

In some embodiments, the analytics controller 500 can be configured to detect when gate selection is initiated by the mouse 510. The analytics controller 500 can be further configured to automatically modify plot visualization to facilitate the gating process. The modification can be based on the specific distribution of biological event data received by the analytics controller 500.

The analytics controller 500 can be connected to a storage device 504. The storage device 504 can be configured to receive and store biological event data from the analytics controller 500. The storage device 504 can also be configured to receive and store flow cytometric event data from the analytics controller 500. The storage device 504 can be further configured to allow retrieval of biological event data, such as flow cytometric event data, by the analytics controller 500.

A display device 506 can be configured to receive display data from the analytics controller 500. The display data can comprise plots of biological event data and gates outlining sections of the plots. The display device 506 can be further configured to alter the information presented according to input received from the analytics controller 500 in conjunction with input from the particle analyzer 502, the storage device 504, the keyboard 508, and/or the mouse 510.

In some implementations, the analytics controller 500 can generate a user interface to receive example events for sorting. For example, the user interface can include a control for receiving example events or example images. The example events or images or an example gate can be provided prior to collection of event data for a sample, or based on an initial set of events for a portion of the sample.

**FIG. 6A** is a schematic drawing of a particle sorter system 600 (e.g., the particle analyzer or sorting system 502) in accordance with one embodiment presented herein. In some embodiments, the particle sorter system 600 is a cell sorter system. As shown in **FIG. 6A,** a drop formation transducer 602 (e.g., piezo-oscillator) is coupled to a fluid conduit 601, which can be coupled to, can include, or can be, a nozzle 603. Within the fluid conduit 601, sheath fluid 604 hydrodynamically focuses a sample fluid 606 comprising particles 609 into a moving fluid column 608 (e.g., a stream). Within the moving fluid column 608, particles 609 (e.g., cells) are lined up in single file to cross a monitored area 611 (e.g., where laser-stream intersect), irradiated by an irradiation source 612 (e.g., a laser). Vibration of the drop formation transducer 602 causes moving fluid column 608 to break into a plurality of drops 610, some of which contain particles 609.

In operation, a detection station 614 (e.g., an event detector) identifies when a particle of interest (or cell of interest) crosses the monitored area 611. Detection station 614 feeds into a timing circuit 628, which in turn feeds into a flash charge circuit 630. At a drop break off point, informed by a timed drop delay ($\Delta t$), a flash charge can be applied to the moving fluid column 608 such that a drop of interest carries a charge. The drop of interest can include one or more particles or cells to be sorted. The charged drop can then be sorted by activating deflection plates (not shown) to deflect the drop into a vessel such as a collection tube or a multi- well or microwell sample plate where a well or microwell can be associated with drops of particular interest. As shown in **FIG. 6A,** the drops can be collected in a drain receptacle 638.

A detection system 616 (e.g., a drop boundary detector) serves to automatically determine the phase of a drop drive signal when a particle of interest passes the monitored area 611. An exemplary drop boundary detector is described in U.S. Pat. No. 7,679,039, which is incorporated herein by reference in its entirety. The detection system 616 allows the instrument to accurately calculate the place of each detected particle in a drop. The detection system 616 can feed into an amplitude signal 620 and/or phase 618 signal, which in turn feeds (via amplifier 622) into an amplitude control circuit 626 and/or frequency control circuit 624. The amplitude control circuit 626 and/or frequency control circuit 624, in turn, controls the drop formation transducer 602. The amplitude control circuit 626 and/or frequency control circuit 624 can be included in a control system.

In some implementations, sort electronics (e.g., the detection system 616, the detection station 614 and a processor 640) can be coupled with a memory configured to store the detected events and a sort decision based thereon. The sort decision can be included in the event data for a particle. In some implementations, the detection system 616 and the detection station 614 can be implemented as a single detection unit or communicatively coupled such that an event measurement can be collected by one of the detection system 616 or the detection station 614 and provided to the non-collecting element.

**FIG. 6B** is a schematic drawing of a particle sorter system, in accordance with one embodiment presented herein. The particle sorter system 600 shown in **FIG. 6B,** includes deflection plates 652 and 654. A charge can be applied via a stream-charging wire in a barb. This creates a stream of droplets 610 containing particles 610 for analysis. The particles can be illuminated with one or more light sources (e.g., lasers) to generate light scatter and fluorescence information. The information for a particle is analyzed such as by sorting electronics or other detection system (not shown in FIG. 6B). The deflection plates 652 and 654 can be independently controlled to attract or repel the charged droplet to guide the droplet toward a destination collection receptacle (e.g., one of 672, 674, 676, or 678). As shown in **FIG. 6B,** the deflection plates 652 and 654 can be controlled to direct a particle along a first path 662 toward the receptacle 674 or along a second path 668 toward the receptacle 678. If the particle is not of interest (e.g., does not exhibit scatter or illumination information within a specified sort range), deflection plates may allow the particle to continue along a flow path 664. Such uncharged droplets may pass into a waste receptacle such as via aspirator 670.

The sorting electronics can be included to initiate collection of measurements, receive fluorescence signals for particles, and determine how to adjust the deflection plates to cause sorting of the particles. Example implementations of the embodiment shown in **FIG. 6B** include the BD FACSAria™ line of flow cytometers commercially provided by Becton, Dickinson and Company (Franklin Lakes, NJ).

**FIG. 7** depicts a general architecture of an example computing device 700 according to certain embodiments. The general architecture of the computing device 700 depicted in **FIG. 7** includes an arrangement of computer hardware and software components. It is not necessary, however, that all of these generally conventional elements be shown in order to provide an enabling disclosure. As illustrated, the computing device 700 includes a processing unit 710, a network interface 720, a computer readable medium drive 730, an input/output device interface 740, a display 750, and an input device 760, all of which may communicate with one another by way of a communication bus. The network interface 720 may provide connectivity to one or more networks or computing systems. The processing unit 710 may thus receive information and instructions from other computing systems or services via a network. The processing unit 710 may also communicate to and from memory 770 and further provide output information for an optional display 750 via the input/output device interface 740. For example, an analysis software (e.g., data analysis software or program such as FlowJo®) stored as executable instructions in the non-transitory memory of the analysis system can display the flow cytometry event data to a user. The input/output device interface 740 may also accept input from the optional input device 760, such as a keyboard, mouse, digital pen, microphone, touch screen, gesture recognition system, voice recognition system, gamepad, accelerometer, gyroscope, or other input device.

The memory 770 may contain computer program instructions (grouped as modules or components in some embodiments) that the processing unit 710 executes in order to implement one or more embodiments. The memory 770 generally includes RAM, ROM and/or other persistent, auxiliary or non-transitory computer-readable media. The memory 770 may store an operating system 772 that provides computer program instructions for use by the processing unit 710 in the general administration and operation of the computing device 700. Data may be stored in data storage device 790. The memory 770 may further include computer program instructions and other information for implementing aspects of the present disclosure.

## KITS

Aspects of the present disclosure further include kits, where kits include storage media such as a magneto-optical disk, CD-ROM, CD-R, magnetic tape, non-volatile memory card, ROM, DVD-ROM, Blue-ray disk, solid state disk, and network attached storage (NAS). Any of these program storage media, or others now in use or that may later be developed, may be included in the subject kits. In embodiments, the program storage media include instructions for classifying flow cytometer data. In embodiments, the instructions contained on computer readable media provided in the subject kits, or a portion thereof, can be implemented as software components of a software for analyzing data. In these embodiments, computer-controlled systems according to the instant disclosure may function as a software "plugin" for an existing software package (e.g., FlowJo®).

In addition to the above components, the subject kits may further include (in some embodiments) instructions. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

## UTILITY

The subject particle analyzers, methods and computer systems find use in a variety of applications where it is desirable to analyze and, optionally, sort particle components in a sample in a fluid medium, such as a biological sample, and then store sorted products, e.g., for later use, such as therapeutic use. The present disclosure particularly finds use where it is desirable to classify flow cytometer data. For example, the subject particle analyzers, methods and computer systems may be employed to facilitate the determination of a suitable gate for a particular population or subpopulation of flow cytometer data, especially in data sets where such suitable gates are not readily apparent. Embodiments of the disclosure also find use where it is desirable to provide a flow cytometer with improved cell sorting accuracy, enhanced particle collection, particle charging efficiency, more accurate particle charging and enhanced particle deflection during cell sorting.

Embodiments of the disclosure find use in applications where cells prepared from a biological sample may be desired for research, laboratory testing or for use in therapy. In some embodiments, the subject methods and devices may facilitate obtaining and/ or analyzing individual cells prepared from a target fluidic or tissue biological sample. For example, the subject methods and systems facilitate obtaining cells from fluidic or tissue samples to be used as a research or diagnostic specimen for diseases such as cancer. Likewise, the subject methods and systems may facilitate obtaining cells from fluidic or tissue samples to be used in therapy.

The following is presented by way of example and not by way of limitation:

## EXPERIMENTAL

### EXPERIMENT 1

The inclusion of cellular doublets in a targeted single cell sort has obvious consequences in that the non-target cells contaminate the sort product and can lead to misleading or undesirable results in downstream applications. Doublets that consist of a cell and an un-lysed red blood cell, large exosome, apoptotic body or platelet can also impact the results of a sort. The inclusion and or exclusion of these events in a sort and the impact they may have on the quality of the sort product was explored.

In summary, blood mononuclear cells (PBMC's) were prepared by ficol separation from 5 healthy donors. Sample preparations were counterstained with DRAQ5, a membrane permeable, DNA intercalating dye that is compatible with BD CellView™ Image Technology. Singlet, doublet and clump frequency was tracked via DNA content as traditional scatter-based doublet discrimination strategies were compared with an image-based approach. Using DNA content as a ground truth for singlet vs. non-singlet events, each gate along the workflow was compared. Traditional scatter-based approaches such as width vs. height and area vs. height were performed alongside an image-based approach using the features of eccentricity and radial moment. The image-based approach was evaluated alone and in conjunction with scatter-based methods. Singlet preservation was also evaluated as a significant loss of singlets would be an undesirable consequence of any successful doublet exclusion strategy.

*Sample Preparation and Data Acquisition*

Whole blood was collected in standard EDTA sample collection tubes. Peripheral blood mononuclear cells (PBMCs) were prepared using a standard ficol density gradient centrifugation protocol. Blood from 5 healthy donors was processed in parallel. PBMCs were washed and stained with Draq5 (BD Biosciences cat#564902). Cells were acquired on a BD FACSDiscover™ S8 with CellView™ Image Technology prototype. Data was analyzed in FlowJo™ software (BD Biosciences).

*Manual Gating Techniques*

Doublet discrimination gating based on scatter (SSC and FSC) was done without the use of imaging in any way as it was tried to capture practices employed by users on non-imaging enabled cytometers. The use of imaging was not limited to numerical image derived features but also employed pop-up mouse-over imaging to guide gate placement.

*Machine Learning (ML) Algorithm Techniques*

One training dataset of 25K events was used to find a regression relationship between predictors and target (DRAQ5 DNA stain indicator). A top ten feature list was generated and then iteratively fine-tuned to arrive at a sparse 3-feature set (**FIG. 8A**). Underlying distribution was assumed to be a multi-modal Gaussian mixture and unsupervised clustering was performed on 375K events (10 datasets) (**FIG. 8B**).

*Results*

The imaging features used in the manual gating method were eccentricity and radial moment. Eccentricity is a ratio of the shortest to longest axis moments within the image in a given scatter or fluorescent channel, while radial moment is the average distance of all signal-positive pixels from the centroid (relative center) within the image in a given scatter or fluorescent channel. In **FIG. 9**, the distribution of lymphocytes, monocytes and high scatter events are shown in the scatter plot and the imaging features plot. Next, non-singlet exclusion methods based on imaging features and scatter were compared. As shown in **FIG. 10A**, use of DRAQ5 (DNA stain) enabled enumeration of absolute numbers of singlets and non-singlets within each gate. The number of ground truth singlets and non-singlets (based on DNA content) inside and outside each gate are shown as pie charts. As shown in **FIG. 10B**, 83% of non-singlets were excluded and 95% of total singlets were preserved using a non-singlet exclusion method using imaging alone. Results from a non-singlet exclusion method using height vs. width in addition to imaging are shown in **FIG. 11A-11B**. Such a workflow combines scatter and imaging-based approaches to doublet discrimination. In the right-most plot in **FIG. 11A**, a more aggressive gate configured to exclude more non-singlets is also shown. Metrics from this gate are shown in the bottom plot of **FIG. 11B**, while results from the other gate are shown in the top plot of **FIG. 11B**. Results from a non-singlet exclusion method using height vs. area plus imaging are shown in **FIG. 12A-12B**. This workflow also combines scatter and imaging-based approaches to doublet discrimination. As shown in **FIG. 12B**, 91% of non-singlets were excluded while 92% of total singlets were preserved. A bar graph of the percentage of total non-singlets excluded by each method is shown in **FIG. 13A** (n=5 donors). A bar graph of the percentage of singlets preserved by each is shown in **FIG. 13B**. Data was normalized for each donor to the absolute number of non-singlets/singlets as measured by DNA content.

Clustering outcomes for the machine learning-based algorithm method were plotted. **FIG. 14A** shows clustering outcomes for GT singlets (magenta), GT non-singlets (blue), algorithm identified non-singlets (that are true singlets) (orange) and algorithm identified singlets (that are true non-singlets) (light blue). **FIG. 14B** shows all algorithm identified singlets (violet) and algorithm identified non-singlets (light blue). A box-and-whisker plot depicted in **FIG. 15** demonstrates that median true singlet identification rate is ~97% (blue) and true doublet exclusion rate is ~92% (orange). The S.D. of singlets distribution is 0.52 and S.D. of doublets distribution is 1.72.

*Conclusions*

Doublet discrimination based on scatter excluded approximately 60% of doublets (H vs. W and H vs. A were approximately equivalent) and preserved approximately 95% of singlets. Doublet discrimination based on imaging features alone excluded 80% of doublets and preserved 95% of singlets. Combining imaging and scatter parameters, 90% of doublets were excluded and 90% of singlets were preserved. An aggressive imaging gate was able to exclude 97% of doublets, but only preserved 78% of true singlets. An ML based algorithm can include 97% of true singlets and exclude 92% of true non-singlets. A combination of imaging and non-imaging parameters was powerful for singlet vs non-singlet discrimination. The underlying distribution of events closely followed a multi-modal Gaussian assumption. Gaussian clustering was superior to K-means, Spectral, DBSCAN and BIRCH clustering methods for this application.

**E**XPERIMENT **2**

An initial feature list was generated using the DRAQ5 DNA stain indicator, R3 (700)-A channel as the target and all other non-detector channels as predictors. The regression relationship between predictors and target yielded a top-10 feature list. This list was iteratively tuned to arrive at an empirical list consisting of 3 channels, namely: 'Eccentricity (LightLoss (Imaging))', 'LightLoss (Violet)-A' and 'Size (LightLoss (Imaging))'. The final feature list was then used to cluster available datasets consisting of two groups, 25K events and 50K events, each containing data from 5 cell donors. Gaussian mixture modelling was applied to cluster datasets described above. Gaussian mixture modelling assumes that the data distribution is a mixture of Gaussians. For the purpose of this study, the assumption was limited to 2 gaussian models, each for singlet and non-singlet populations. Several clustering techniques including DBSCAN, BIRCH, K-means, and Spectral were applied but the Gaussian mixture model explained the data best and was selected for high specificity and sensitivity. Clustered data was compared against ground truth DRAQ5 DNA stain indicator, R3 (700)-A channel distribution. Performance metrics measured were a) singlet inclusion rate, and b) non-singlet exclusion rate and are shown in **Table 1** and **Table 2**, below, for the data at 25k events and for the data at 50k events, respectively.

**Table 1:** Data at 25K events (5 donors)

| Donor_ ID | True singlets included (%) | True non-singlets excluded (%) | Number of True singlets included | Actual number of singlets | Number of true non-singlets excluded | Actual number of non-singlets |
|---|---|---|---|---|---|---|
| 1 | 96.90 | 92.27 | 25521 | 26161 | 2852 | 2212 |
| 2 | 97.98 | 91.96 | 25191 | 25480 | 3088 | 2799 |
| 3 | 97.23 | 92.52 | 25864 | 26365 | 3548 | 3047 |
| 4 | 98.53 | 88.02 | 27739 | 27959 | 1806 | 1586 |
| 5 | 97.46 | 91.83 | 25253 | 25694 | 3036 | 2595 |
| **MEAN** | **97.62** | **91.32** | | | | |
| **STD** | **0.64** | **1.86** | | | | |

**Table 2:** Data at 50K events (5 donors)

| Donor_ ID | True singlets included (%) | True non-singlets excluded (%) | Number of True singlets included | Actual number of singlets | Number of true non-singlets excluded | Actual number of non-singlets |
|---|---|---|---|---|---|---|
| 1 | 97.22 | 92.15 | 51255 | 52395 | 5138 | 3998 |
| 2 | 97.83 | 93.37 | 50117 | 50940 | 5049 | 4226 |
| 3 | 97.22 | 92.67 | 51232 | 52330 | 5941 | 4843 |
| 4 | 98.33 | 88.85 | 55584 | 56076 | 4492 | 4000 |
| 5 | 97.67 | 92.24 | 50503 | 51403 | 4754 | 3854 |
| **MEAN** | **97.654** | **91.856** | | | | |
| **STD** | **0.465005** | **1.748193** | | | | |

Notwithstanding the appended claims, the disclosure is also defined by the following clauses:

1. A computer-implemented method for classifying analyte data into clusters, the method comprising, via a processor:

   applying a regression model to determine a relationship between an initial set of analyte features and a cluster criterion;
   generating a sparse set from at most a portion of the initial set of the analyte features based on the relationship;
   generating a classification model based on the sparse set; and
   applying the classification model to classify the analyte data into the clusters.

2. The computer-implemented method according to Clause 1, wherein the analyte data is flow cytometer data.
3. The computer-implemented method according to Clause 2, wherein the cluster criterion is an association of the analyte data with a singlet.
4. The computer-implemented method according to Clause 3, wherein the clusters comprise a singlet cluster and a non-singlet cluster.
5. The computer-implemented method according to Clause 4, wherein the non-singlet cluster comprises a doublet or an aggregate.
6. The computer-implemented method according to any one of Clauses 2 to 5, wherein the analyte features comprise a size feature, imaging feature, a scatter feature, or any combination thereof.
7. The computer-implemented method according to Clause 6, wherein the analyte features comprise imaging features.
8. The computer-implemented method according to any one of the preceding clauses, wherein the classification model comprises a mixture model.
9. The computer-implemented method according Clause 8, wherein the mixture model comprises a Gaussian mixture model.
10. The computer implemented method according to any one of Clauses 1 to 7, wherein the classification model comprises a density-based spatial clustering algorithm.
11. The computer implemented method according to Clause 10, wherein the density-based spatial clustering algorithm comprises a density-based spatial clustering of applications with noise (DBSCAN) algorithm.
12. The computer implemented method according to any one of Clauses 1 to 7, wherein the classification model comprises a balanced iterative reducing and clustering using hierarchies (BIRCH) algorithm.
13. The computer implemented method according to any one of Clauses 1 to 7, wherein the classification model comprises a K-means clustering algorithm.
14. The computer implemented method according to any one of Clauses 1 to 7, wherein the classification model comprises a spectral clustering algorithm.
15. The computer-implemented method according to any one of the preceding clauses, wherein the initial set of analyte features comprises from 5 to 20 analyte features.
16. The computer-implemented method according to Clause 15, wherein the initial set of analyte features comprises from 7 to 15 analyte features.
17. The computer-implemented method according to Clause 16, wherein the initial set of analyte features comprises from 9 to 11 analyte features.

18. The computer-implemented method according to any one of the preceding clauses, wherein the initial set of analyte features comprises 10% or more of possible analyte features.

19. The computer-implemented method according to Clause 18, wherein the initial set of analyte features comprises 15% or more of possible analyte features.

20. The computer-implemented method according to Clause 19, wherein the initial set of analyte features comprises 20% or more of possible analyte features.

21. The computer-implemented method according to any one of the preceding clauses, wherein the sparse set of analyte features comprises from 2 to 10 analyte features.

22. The computer-implemented method according to Clause 21, wherein the sparse set of analyte features comprises from 2 to 5 analyte features.

23. The computer-implemented method according to Clause 22, wherein the sparse set of analyte features comprises from 2 to 4 analyte features.

24. The computer-implemented method according to any one of the preceding clauses, wherein classifying the analyte data comprises including 90% or more of analyte data associated with the cluster criterion in a cluster associated with the cluster criterion.

25. The computer-implemented method according to Clause 24, wherein classifying the analyte data comprises including 95% or more of the analyte data associated with the cluster criterion in the cluster associated with the cluster criterion.

26. The computer-implemented method according to Clause 25, wherein classifying the analyte data comprises including 97% or more of the analyte data associated with the cluster criterion in the cluster associated with the cluster criterion.

27. The computer-implemented method according to any one of Clauses 1 to 23, wherein classifying the analyte data comprises excluding 85% or more of analyte data not associated with the cluster criterion from a cluster associated with the cluster criterion.

28. The computer-implemented method according to Clause 27, wherein classifying the analyte data comprises excluding 90% or more of the analyte data not associated with the cluster criterion from the cluster associated with the cluster criterion.

29. The computer-implemented method according to Clause 28, wherein classifying the analyte data comprises excluding 92% or more of the analyte data not associated with the cluster criterion from the cluster associated with the cluster criterion.

30. A system comprising memory operably coupled to a processor, wherein the memory comprises instructions stored thereon, which when executed by the processor, cause the processor to:

apply a regression model to determine a relationship between an initial set of analyte features and a cluster criterion;
generate a sparse set from at most a portion of the initial set of the analyte features based on the relationship;
generate a classification model based on the sparse set; and
apply the classification model to classify analyte data into the clusters.

31. The system according to Clause 30, wherein the analyte data is flow cytometer data.

32. The system according to Clause 31, wherein the cluster criterion is an association of the flow cytometer data with a singlet.

33. The system according to Clause 32, wherein the clusters comprise a singlet cluster and a non-singlet cluster.

34. The system according to Clause 33, wherein the non-singlet cluster comprises a doublet or an aggregate.

35. The system according to any one of Clauses 31 to 34, wherein the analyte features comprise a size feature, imaging feature, a scatter feature, or any combination thereof.

36. The system according to Clause 35, wherein the analyte features comprise imaging features.

37. The system according to any one of Clauses 30 to 36, wherein the classification model comprises a mixture model.

38. The system according to Clause 37, wherein the mixture model comprises a Gaussian mixture model.

39. The system according to any one of Clauses 30 to 36, wherein the classification model comprises a density-based spatial clustering algorithm.

40. The system according to Clause 39, wherein the density-based spatial clustering algorithm comprises a density-based spatial clustering of applications with noise (DBSCAN) algorithm.

41. The system according to any one of Clauses 30 to 36, wherein the classification model comprises a balanced iterative reducing and clustering using hierarchies (BIRCH) algorithm.

42. The system according to any one of Clauses 30 to 36, wherein the classification model comprises a K-means clustering algorithm.

43. The system according to any one of Clauses 30 to 36, wherein the classification model comprises a spectral clustering algorithm.

44. The system according to any one of Clauses 30 to 43, wherein the initial set of analyte features comprises from 5 to 20 analyte features.

45. The system according to Clause 44, wherein the initial set of analyte features comprises from 7 to 15 analyte features.

46. The system according to Clause 45, wherein the initial set of analyte features comprises from 9 to 11 analyte features.

47. The system according to any one of Clauses 30 to 46, wherein the initial set of analyte features comprises 10% or more of possible analyte features.

48. The system according to Clause 47, wherein the initial set of analyte features comprises 15% or more of possible analyte features.

49. The system according to Clause 48, wherein the initial set of analyte features comprises 20% or more of possible analyte features.

50. The system according to any one of Clauses 30 to 49, wherein the sparse set of analyte features comprises from 2 to 10 analyte features.

51. The system according to Clause 50, wherein the sparse set of analyte features comprises from 2 to 5 analyte features.

52. The system according to Clause 51, wherein the sparse set of analyte features comprises from 2 to 4 analyte features.

53. The system according to any one of Clauses 30 to 52, wherein classifying the analyte data comprises including 90% or more of analyte data associated with the cluster criterion in a cluster associated with the cluster criterion.

54. The system according to Clause 53, wherein classifying the analyte data comprises including 95% or more of the analyte data associated with the cluster criterion in the cluster associated with the cluster criterion.

55. The system according to Clause 54, wherein classifying the analyte data comprises including 97% or more of the analyte data associated with the cluster criterion in the cluster associated with the cluster criterion.

56. The system according to Clauses 30 to 52, wherein classifying the analyte data comprises excluding 85% or more of analyte data not associated with the cluster criterion from a cluster associated with the cluster criterion.

57. The system according to Clause 56, wherein classifying the analyte data comprises excluding 90% or more of the analyte data not associated with the cluster criterion from the cluster associated with the cluster criterion.

58. The system according to Clause 57, wherein classifying the analyte data comprises excluding 92% or more of the analyte data not associated with the cluster criterion from the cluster associated with the cluster criterion.

59. The system according to any one of Clauses 30 to 58, further comprising a display configured to output the classified analyte data.

60. The system according to any one of Clauses 30 to 59, further comprising a flow cytometer operably connected to the processor.

61. A non-transitory computer-readable storage medium comprising instructions stored thereon for classifying analyte data into clusters by a method comprising:

applying a regression model to determine a relationship between an initial set of analyte features and a cluster criterion;
generating a sparse set from at most a portion of the initial set of the analyte features based on the relationship;
generating a classification model based on the sparse set; and
applying the classification model to classify the analyte data into the clusters.

62. The non-transitory computer-readable storage medium according to Clause 61, wherein the analyte data is flow cytometer data.

63. The non-transitory computer-readable storage medium according to Clause 62, wherein the cluster criterion is an association of the analyte data with a singlet.

64. The non-transitory computer-readable storage medium according to Clause 63, wherein the clusters comprise a singlet cluster and a non-singlet cluster.

65. The non-transitory computer-readable storage medium according to Clause 64, wherein the non-singlet cluster comprises a doublet or an aggregate.

66. The non-transitory computer-readable storage medium according to any one of Clauses 62 to 65, wherein the analyte features comprise a size feature, imaging feature, a scatter feature, or any combination thereof.

67. The non-transitory computer-readable storage medium according to Clause 66, wherein the analyte features comprise imaging features.

68. The non-transitory computer-readable storage medium according to any one of Clauses 61 to 67, wherein the classification model comprises a mixture model.

69. The non-transitory computer-readable storage medium according to Clause 68, wherein the mixture model comprises a Gaussian mixture model.

70. The non-transitory computer-readable storage medium according to any one of Clauses 61 to 67, wherein the classification model comprises a density-based spatial clustering algorithm.

71. The non-transitory computer-readable storage medium according to Clause 70, wherein the density-based spatial clustering algorithm comprises a density-based spatial clustering of applications with noise (DBSCAN) algorithm.

72. The non-transitory computer-readable storage medium according to any one of Clauses 61 to 67, wherein the classification model comprises a balanced iterative reducing and clustering using hierarchies (BIRCH) algorithm.

73. The non-transitory computer-readable storage medium according to any one of Clauses 61 to 67, wherein the classification model comprises a K-means clustering algorithm.

74. The non-transitory computer-readable storage medium according to any one of Clauses 61 to 67, wherein the classification model comprises a spectral clustering algorithm.

75. The non-transitory computer-readable storage medium according to any one of Clauses 61 to 74, wherein the initial set of analyte features comprises from 5 to 20 analyte features.

76. The non-transitory computer-readable storage medium according to Clause 75, wherein the initial set of analyte features comprises from 7 to 15 analyte features.

77. The non-transitory computer-readable storage medium according to Clause 76, wherein the initial set of analyte features comprises from 9 to 11 analyte features.

78. The non-transitory computer-readable storage medium according to any one of Clauses 61 to 77, wherein the initial set of analyte features comprises 10% or more of possible analyte features.

79. The non-transitory computer-readable storage medium according to Clause 78, wherein the initial set of analyte features comprises 15% or more of possible analyte features.

80. The non-transitory computer-readable storage medium according to Clause 79, wherein the initial set of analyte features comprises 20% or more of possible analyte features.

81. The non-transitory computer-readable storage medium according to any one of Clauses 61 to 80, wherein the sparse set of analyte features comprises from 2 to 10 analyte features.

82. The non-transitory computer-readable storage medium according to Clause 81, wherein the sparse set of analyte features comprises from 2 to 5 analyte features.

83. The non-transitory computer-readable storage medium according to Clause 82, wherein the sparse set of analyte features comprises from 2 to 4 analyte features.

84. The non-transitory computer-readable storage medium according to any one of Clauses 61 to 83, wherein classifying the analyte data comprises including 90% or more of analyte data associated with the cluster criterion in a cluster associated with the cluster criterion.

85. The non-transitory computer-readable storage medium according to Clause 84, wherein classifying the analyte data comprises including 95% or more of the analyte data associated with the cluster criterion in the cluster associated with the cluster criterion.

86. The non-transitory computer-readable storage medium according to Clause 85, wherein classifying the analyte data comprises including 97% or more of the analyte data associated with the cluster criterion in the cluster associated with the cluster criterion.

87. The non-transitory computer-readable storage medium according to any one of Clauses 61 to 83, wherein classifying the analyte data comprises excluding 85% or more of analyte data not associated with the cluster criterion from a cluster associated with the cluster criterion.

88. The non-transitory computer-readable storage medium according to Clause 87, wherein classifying the analyte data comprises excluding 90% or more of the analyte data not associated with the cluster criterion from the cluster associated with the cluster criterion.

89. The non-transitory computer-readable storage medium according to Clause 88, wherein classifying the analyte data comprises excluding 92% or more of the analyte data not associated with the cluster criterion from the cluster associated with the cluster criterion.

90. A computer-implemented method of classifying analyte data into clusters, the method comprising:

(a) providing analyte data to a system comprising instructions stored thereon, which when executed by the processor, cause the processor to:

apply a regression model to determine a relationship between an initial set of analyte features and a cluster criterion;
generate a sparse set from at most a portion of the initial set of the analyte features based on the relationship;
generate a classification model based on the sparse set; and

apply the classification model to classify the analyte data into the clusters; and

(b) receiving the classified analyte data from the processor.

91. The computer-implemented method according to Clause 90, wherein the analyte data is flow cytometer data.

92. The computer-implemented method according to Clause 91, wherein the cluster criterion is an association of the analyte data with a singlet.

93. The computer-implemented method according to Clause 92, wherein the clusters comprise a singlet cluster and a non-singlet cluster.

94. The computer-implemented method according to Clause 93, wherein the non-singlet cluster comprises a doublet or an aggregate.

95. The computer-implemented method according to any one of Clauses 91 to 94, wherein the analyte features comprise a size feature, imaging feature, a scatter feature, or any combination thereof.

96. The computer-implemented method according to Clause 95, wherein the analyte features comprise imaging features.

97. The computer-implemented method according to any one of Clauses 90 to 96, wherein the classification model comprises a mixture model.

98. The computer-implemented method according to any one of Clauses 90 to 97, wherein the mixture model comprises a Gaussian mixture model.

99. The computer-implemented method according to any one of Clauses 90 to 96, wherein the classification model comprises a density-based spatial clustering algorithm.

100. The computer-implemented method according to Clause 99, wherein the density-based spatial clustering algorithm comprises a density-based spatial clustering of applications with noise (DBSCAN) algorithm.

101. The computer-implemented method according to any one of Clauses 90 to 96, wherein the classification model comprises a balanced iterative reducing and clustering using hierarchies (BIRCH) algorithm.

102. The computer-implemented method according to any one of Clauses 90 to 96, wherein the classification model comprises a K-means clustering algorithm.

103. The computer-implemented method according to any one of Clauses 90 to 96, wherein the classification model comprises a spectral clustering algorithm.

104. The computer-implemented method according to any one of Clauses 90 to 103, wherein the initial set of analyte features comprises from 5 to 20 analyte features.

105. The computer-implemented method according to Clause 104, wherein the initial set of analyte features comprises from 7 to 15 analyte features.

106. The computer-implemented method according to Clause 105, wherein the initial set of analyte features comprises from 9 to 11 analyte features.

107. The computer-implemented method according to any one of Clauses 90 to 106, wherein the initial set of analyte features comprises 10% or more of possible analyte features.

108. The computer-implemented method according to Clause 107, wherein the initial set of analyte features comprises 15% or more of possible analyte features.

109. The computer-implemented method according to Clause 108, wherein the initial set of analyte features comprises 20% or more of possible analyte features.

110. The computer-implemented method according to any one of Clauses 90 to 109, wherein the sparse set of analyte features comprises from 2 to 10 analyte features.

111. The computer-implemented method according to Clause 110, wherein the sparse set of analyte features comprises from 2 to 5 analyte features.

112. The computer-implemented method according to Clause 111, wherein the sparse set of analyte features comprises from 2 to 4 analyte features.

113. The computer-implemented method according to any one of Clauses 90 to 112, wherein classifying the analyte data comprises including 90% or more of analyte data associated with the cluster criterion in a cluster associated with the cluster criterion.

114. The computer-implemented method according to Clause 113, wherein classifying the analyte data comprises including 95% or more of the analyte data associated with the cluster criterion in the cluster associated with the cluster criterion.

115. The computer-implemented method according to Clause 114, wherein classifying the analyte data comprises including 97% or more of the analyte data associated with the cluster criterion in the cluster associated with the cluster criterion.

116. The computer-implemented method according to any one of Clauses 90 to 112, wherein classifying the analyte data comprises excluding 85% or more of analyte data not associated with the cluster criterion from a cluster associated with the cluster criterion.

117. The computer-implemented method according to Clause 116, wherein classifying the analyte data comprises excluding 90% or more of the analyte data not associated with the cluster criterion from the cluster associated with the cluster criterion.

118. The computer-implemented method according to Clause 117, wherein classifying the analyte data comprises excluding 92% or more of the analyte data not associated with the cluster criterion from the cluster associated with the cluster criterion.

119. The computer-implemented method according to any one of Clauses 90 to 118, wherein the method comprises receiving the classified analyte data on a display.

Although the foregoing disclosure has been described in some detail by way of illustration and example for purposes of clarity of understanding, it is readily apparent to those of ordinary skill in the art in light of the teachings of this disclosure that some changes and modifications may be made thereto without departing from the spirit or scope of the appended claims.

Accordingly, the preceding merely illustrates the principles of the disclosure. It will be appreciated that those skilled in the art will be able to devise various arrangements which, although not explicitly described or shown herein, embody the principles of the disclosure and are included within its spirit and scope. Furthermore, all examples and conditional language recited herein are principally intended to aid the reader in understanding the principles of the disclosure and the concepts contributed by the disclosure to furthering the art, and are to be construed as being without limitation to such specifically recited examples and conditions. Moreover, all statements herein reciting principles, aspects, and embodiments of the disclosure as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

The scope of the present disclosure, therefore, is not intended to be limited to the exemplary embodiments shown and described herein. Rather, the scope and spirit of present disclosure is embodied by the appended claims. In the claims, 35 U.S.C. §112(f) or 35 U.S.C. §112(6) is expressly defined as being invoked for a limitation in the claim only when the exact phrase "means for" or the exact phrase "step for" is recited at the beginning of such limitation in the claim; if such exact phrase is not used in a limitation in the claim, then 35 U.S.C. § 112 (f) or 35 U.S.C. §112(6) is not invoked.

## Claims

**1.** A computer-implemented method for classifying analyte data into clusters, the method comprising, via a processor:

applying a regression model to determine a relationship between an initial set of analyte features and a cluster criterion;
generating a sparse set from at most a portion of the initial set of the analyte features based on the relationship;
generating a classification model based on the sparse set; and
applying the classification model to classify the analyte data into the clusters.

**2.** The computer-implemented method according to Claim 1, wherein the analyte data is flow cytometer data.

**3.** The computer-implemented method according to Claim 2, wherein the cluster criterion is an association of the analyte data with a singlet.

**4.** The computer-implemented method according to Claim 3, wherein the clusters comprise a singlet cluster and a non-singlet cluster.

**5.** The computer-implemented method according to Claim 4, wherein the non-singlet cluster comprises a doublet or an aggregate.

**6.** The computer-implemented method according to any one of Claims 2 to 5, wherein the analyte features comprise a size feature, imaging feature, a scatter feature, or any combination thereof.

**7.** The computer-implemented method according to Claim 6, wherein the analyte features comprise imaging features.

**8.** The computer-implemented method according to any one of the preceding claims, wherein the classification model comprises a mixture model, preferably a Gaussian mixture model, or a density-based spatial clustering of applications with noise (DBSCAN) algorithm, or a balanced iterative reducing and clustering using hierarchies (BIRCH) algorithm, or a K-means clustering algorithm, or a spectral clustering algorithm.

**9.** The computer-implemented method according to any one of the preceding claims, wherein the initial set of analyte features comprises from 5 to 20 analyte features.

**10.** The computer-implemented method according to any one of the preceding claims, wherein the initial set of analyte features comprises 10% or more of possible analyte features.

**11.** The computer-implemented method according to any one of the preceding claims, wherein the sparse set of analyte features comprises from 2 to 10 analyte features.

**12.** The computer-implemented method according to any one of the preceding claims, wherein classifying the analyte data comprises including 90% or more of analyte data associated with the cluster criterion in a cluster associated with the cluster criterion.

**13.** A system comprising memory operably coupled to a processor, wherein the memory comprises instructions stored thereon, which when executed by the processor, cause the processor to:

apply a regression model to determine a relationship between an initial set of analyte features and a cluster criterion;
generate a sparse set from at most a portion of the initial set of the analyte features based on the relationship;
generate a classification model based on the sparse set; and
apply the classification model to classify analyte data into the clusters.

**14.** A non-transitory computer-readable storage medium comprising instructions stored thereon for classifying analyte data into clusters by a method comprising:

applying a regression model to determine a relationship between an initial set of analyte features and a cluster criterion;
generating a sparse set from at most a portion of the initial set of the analyte features based on the relationship;
generating a classification model based on the sparse set; and
applying the classification model to classify the analyte data into the clusters.

**15.** A computer-implemented method of classifying analyte data into clusters, the method comprising:

(a) providing analyte data to a system comprising instructions stored thereon, which when executed by the processor, cause the processor to:

apply a regression model to determine a relationship between an initial set of analyte features and a cluster criterion;
generate a sparse set from at most a portion of the initial set of the analyte features based on the relationship;
generate a classification model based on the sparse set; and
apply the classification model to classify the analyte data into the clusters; and

(b) receiving the classified analyte data from the processor.

FIG. 1

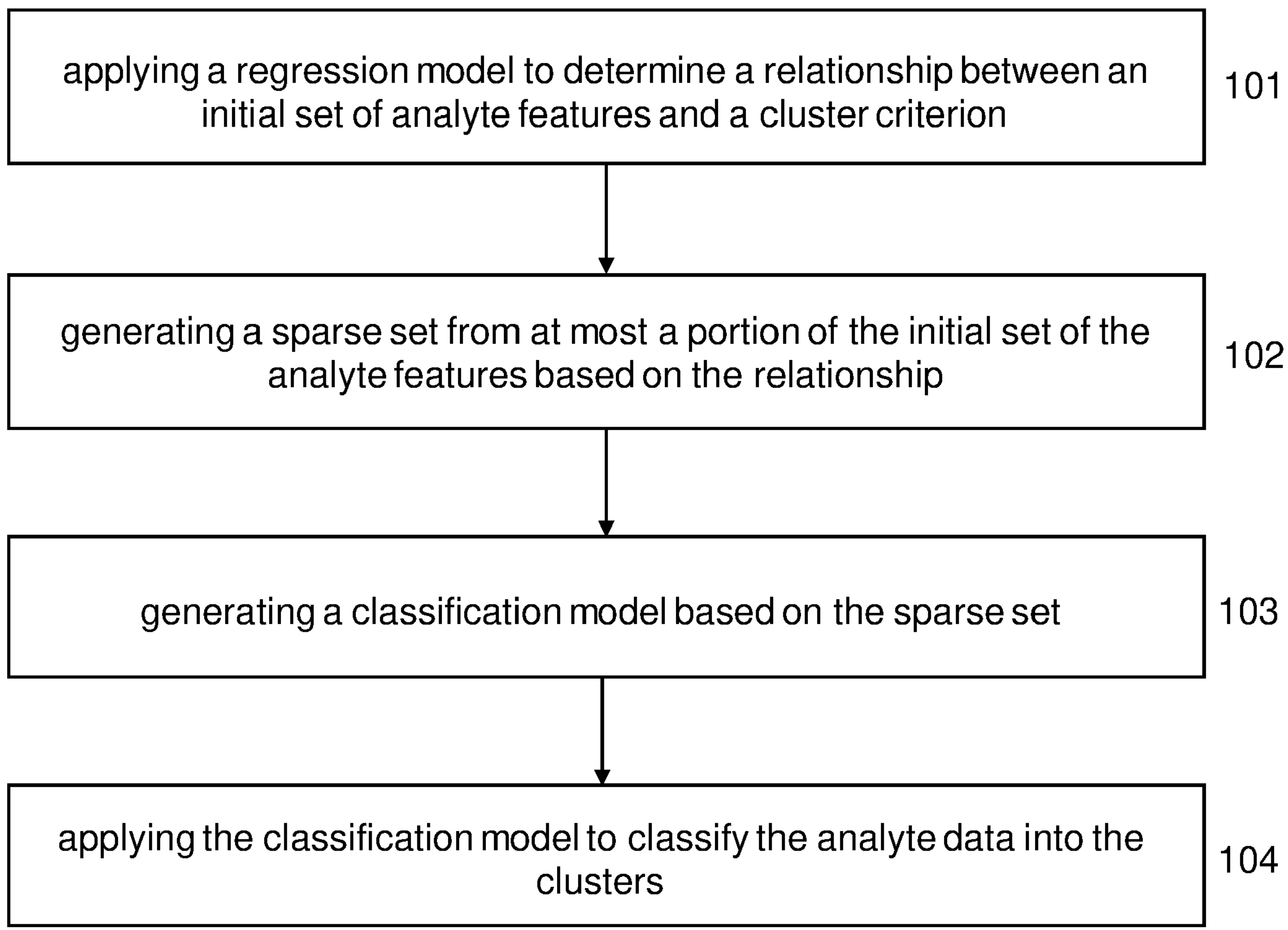
applying a regression model to determine a relationship between an initial set of analyte features and a cluster criterion
101
generating a sparse set from at most a portion of the initial set of the analyte features based on the relationship
102
generating a classification model based on the sparse set
103
applying the classification model to classify the analyte data into the clusters
104

FIG. 2

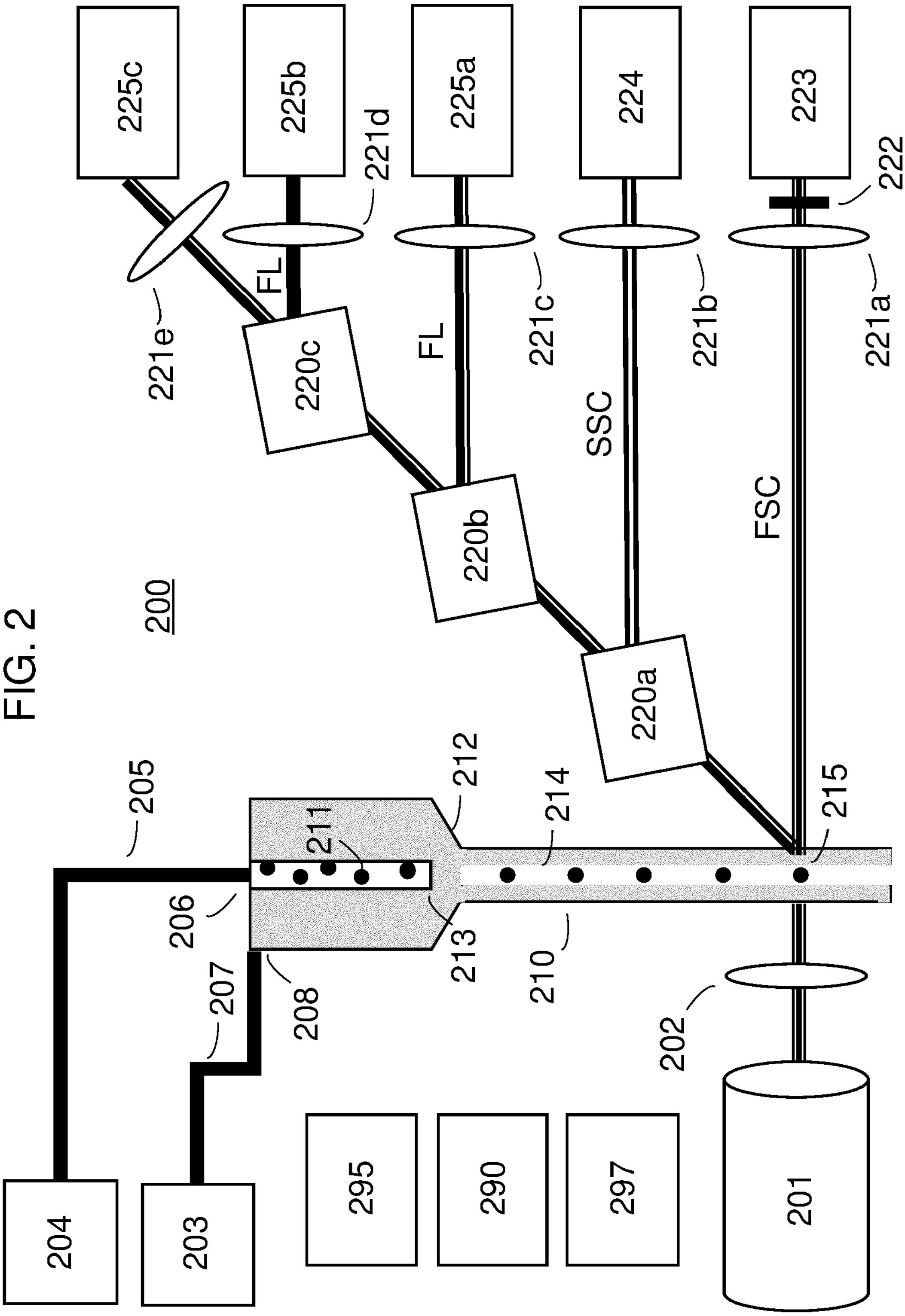
200
201
202
203
204
205
206
207
208
210
211
212
213
214
215
220a
220b
220c
221a
221b
221c
221d
221e
222
223
224
225a
225b
225c
290
295
297
FSC
SSC
FL
FL

FIG. 3

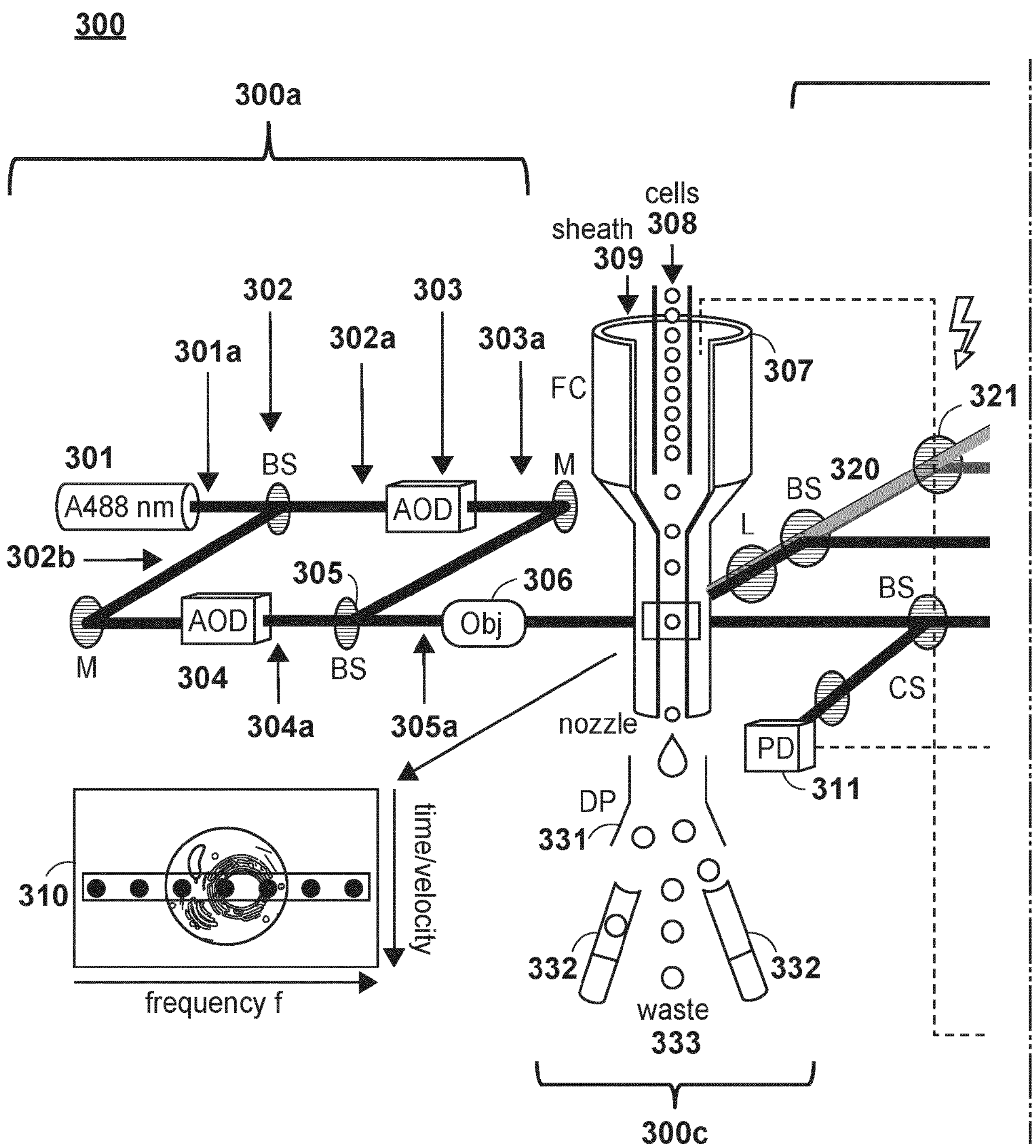
300
300a
cells
308
sheath
309
302
303
301a
302a
303a
307
FC
321
301
BS
M
320
BS
A488 nm
AOD
L
302b
305
306
BS
AOD
Obj
M
304
BS
304a
305a
nozzle
CS
PD
311
DP
331
time/velocity
310
332
332
waste
333
frequency f
300c

FIG. 3 (Cont.)

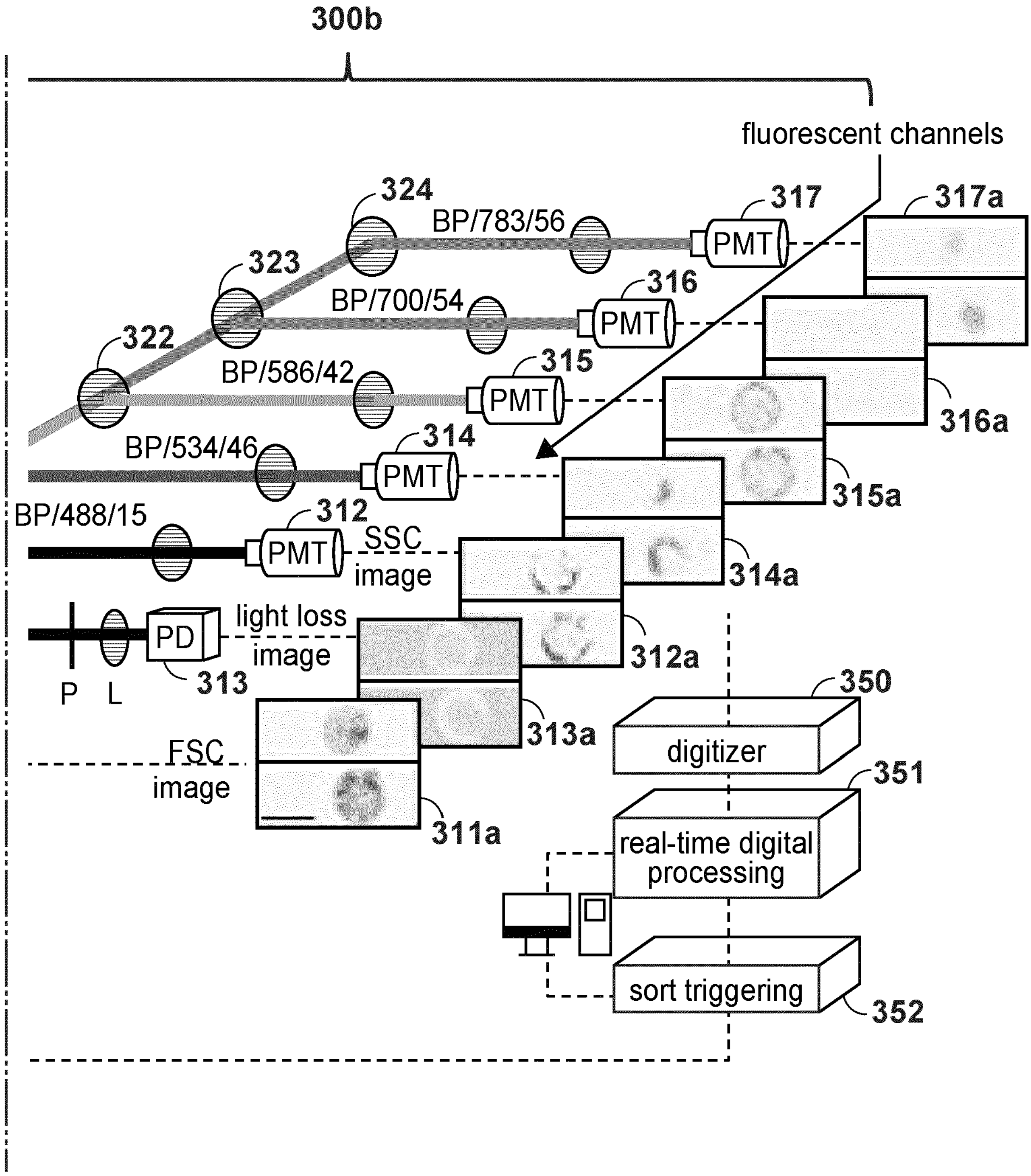
300b
fluorescent channels
324
323
322
317
317a
BP/783/56
PMT
316
BP/700/54
PMT
316a
315
BP/586/42
PMT
315a
314
BP/534/46
PMT
314a
BP/488/15
312
PMT
SSC
image
312a
light loss
image
PD
313
P
L
313a
FSC
image
311a
350
digitizer
351
real-time digital
processing
sort triggering
352

FIG. 4

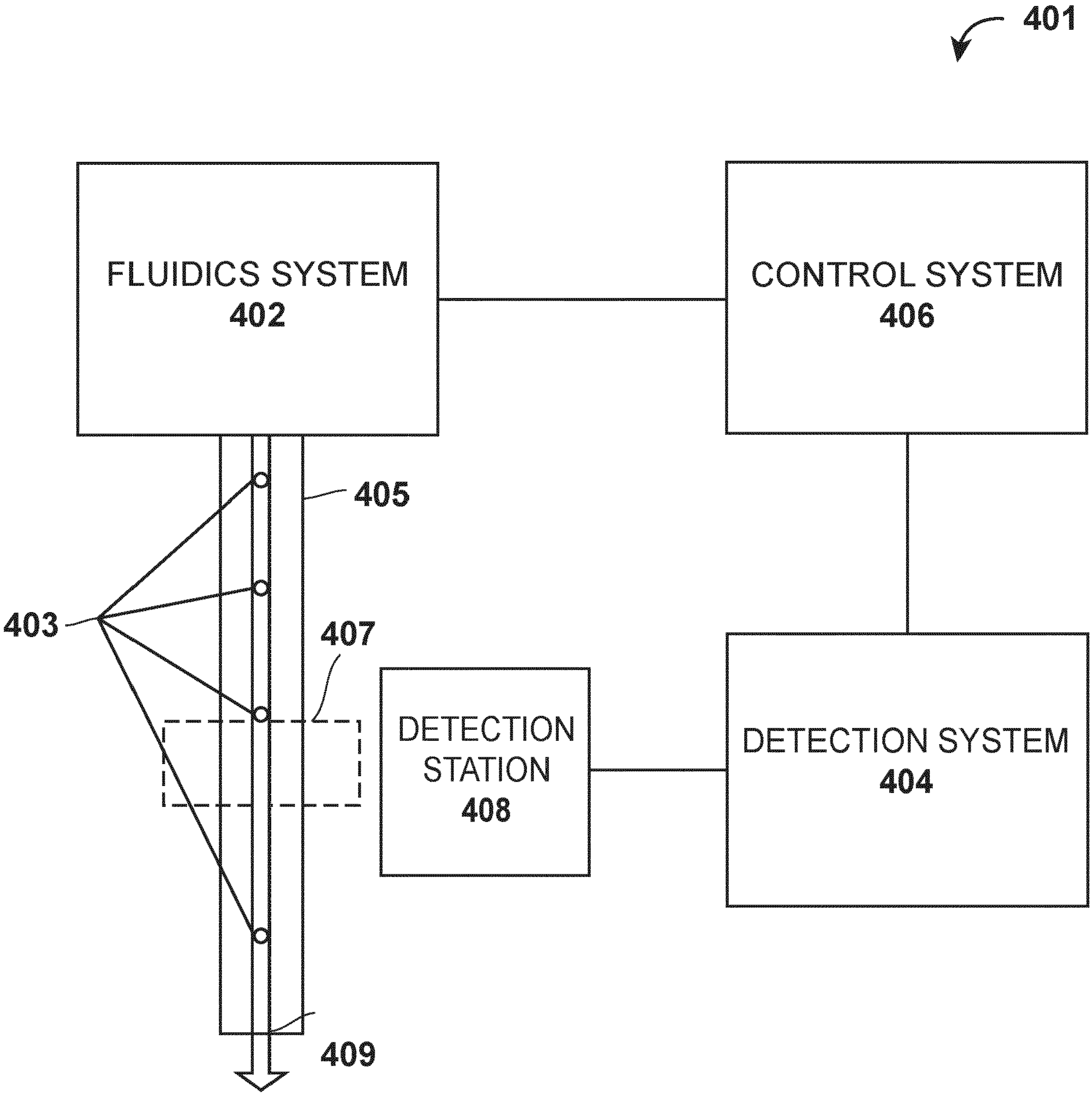
401
FLUIDICS SYSTEM
402
CONTROL SYSTEM
406
405
403
407
DETECTION
STATION
408
DETECTION SYSTEM
404
409

FIG. 5

500

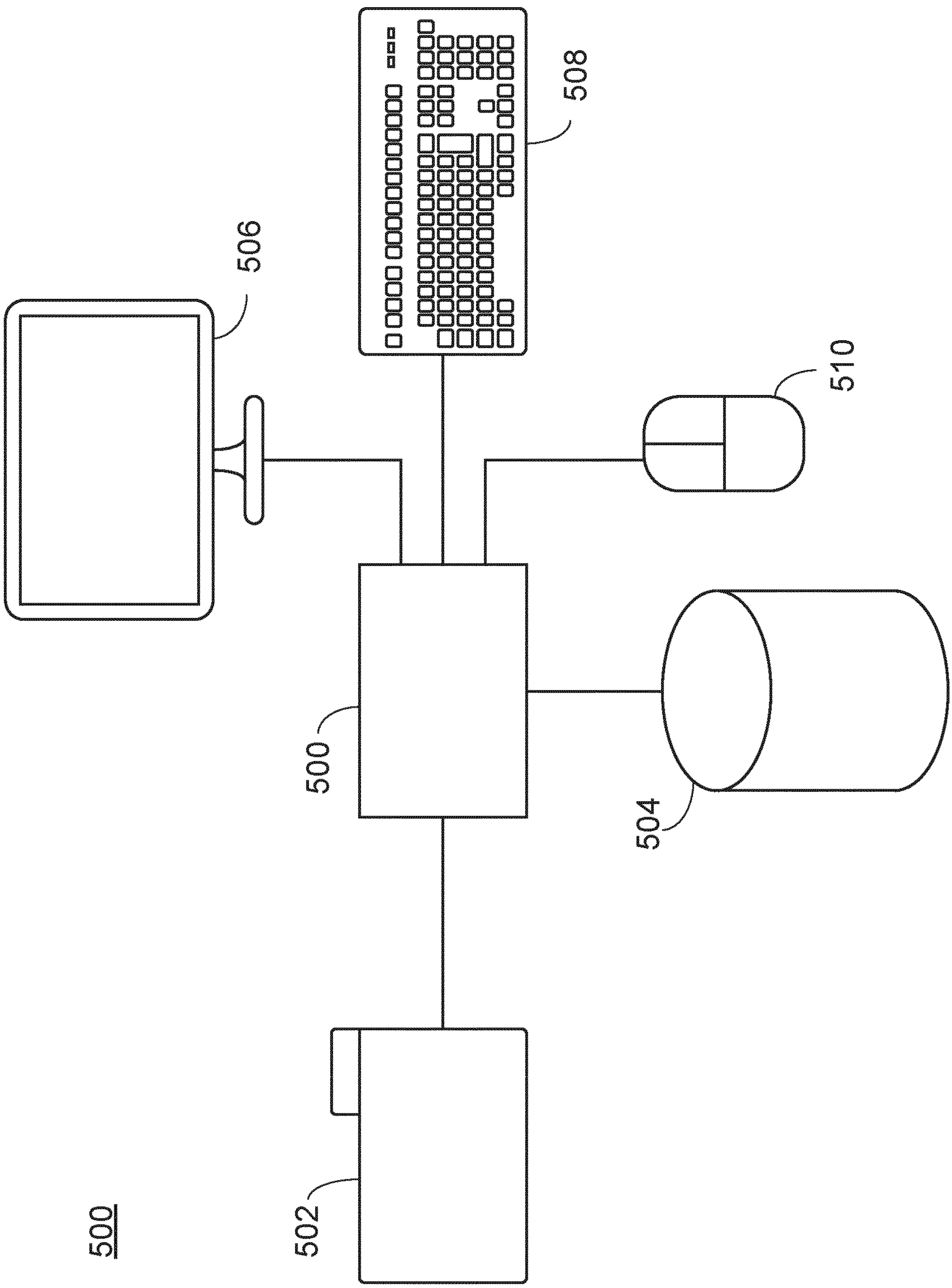

FIG. 6A

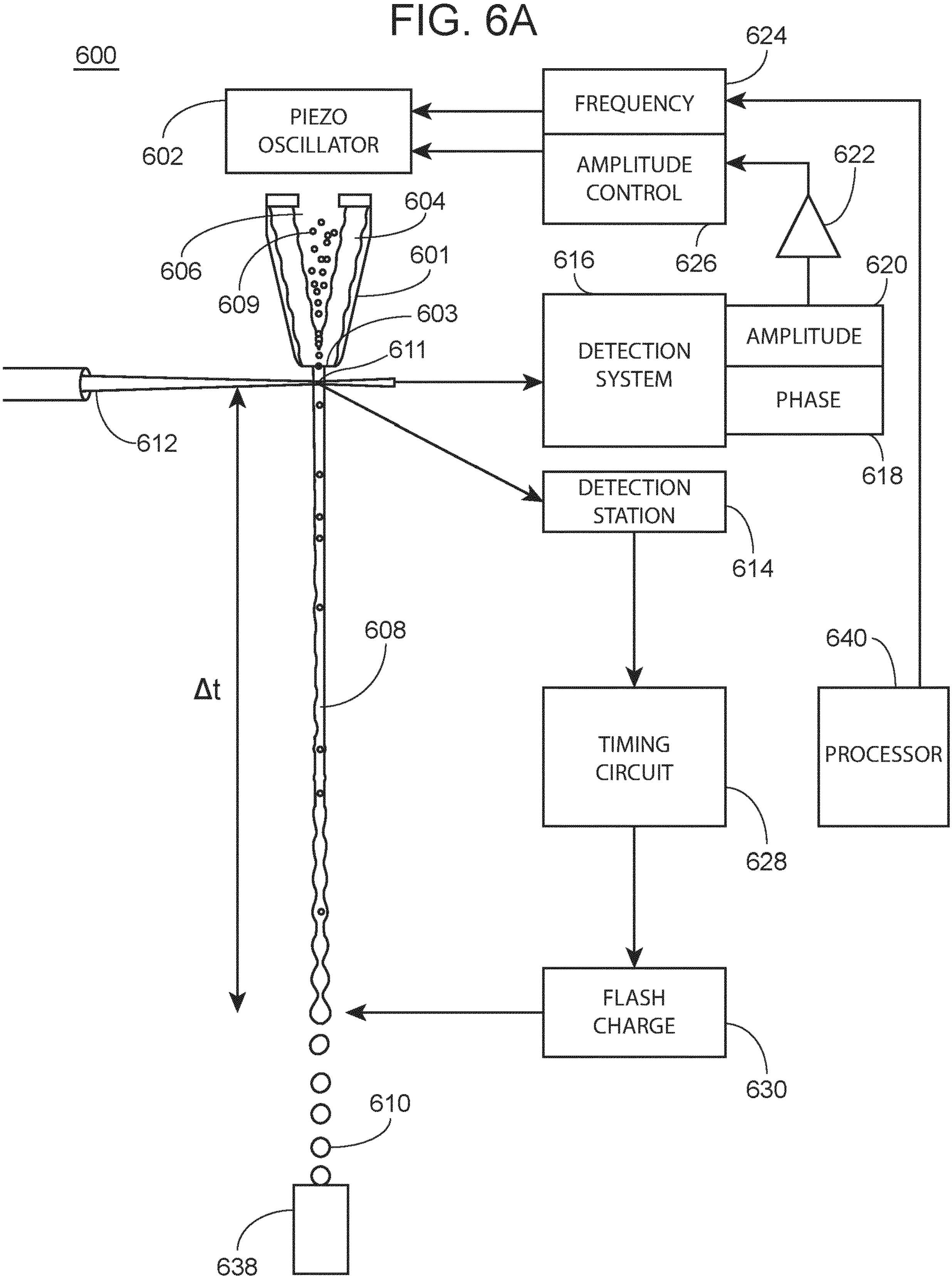
600
PIEZO OSCILLATOR
602
FREQUENCY
AMPLITUDE CONTROL
624
622
626
604
606
601
609
603
611
616
620
DETECTION SYSTEM
AMPLITUDE
PHASE
618
612
DETECTION STATION
614
608
Δt
640
TIMING CIRCUIT
PROCESSOR
628
FLASH CHARGE
630
610
638

FIG. 6B

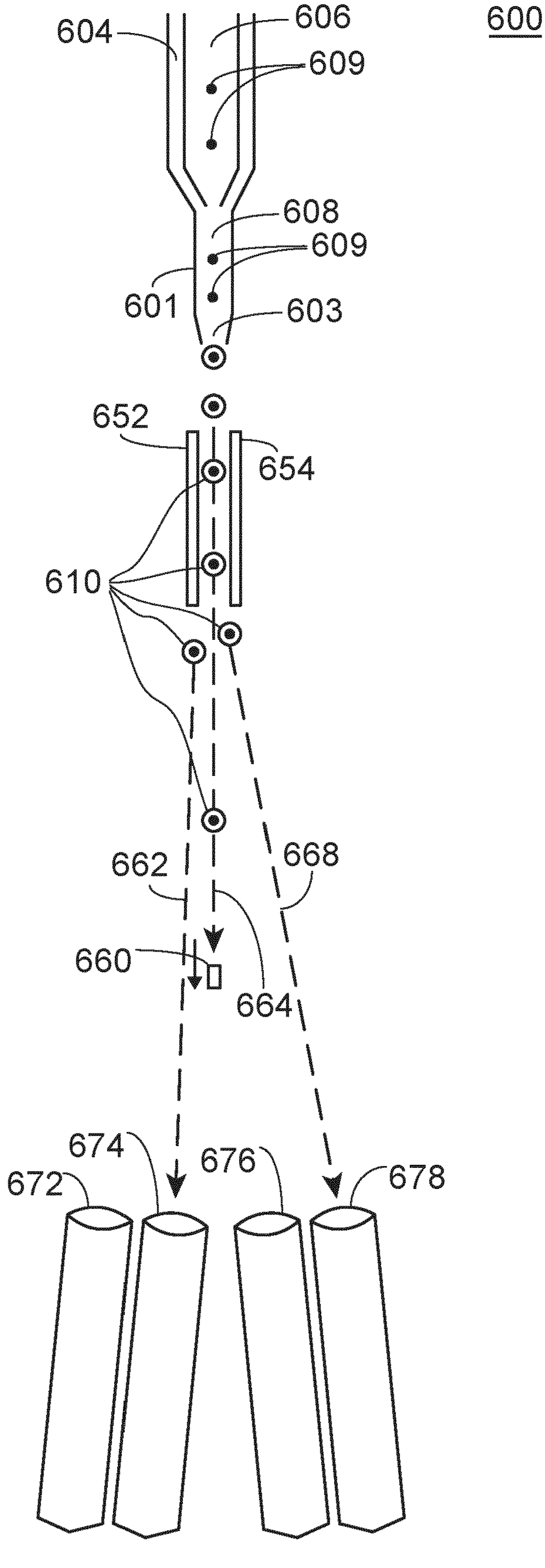
600
604
606
609
608
609
601
603
652
654
610
662
668
660
664
674
676
672
678

FIG. 7

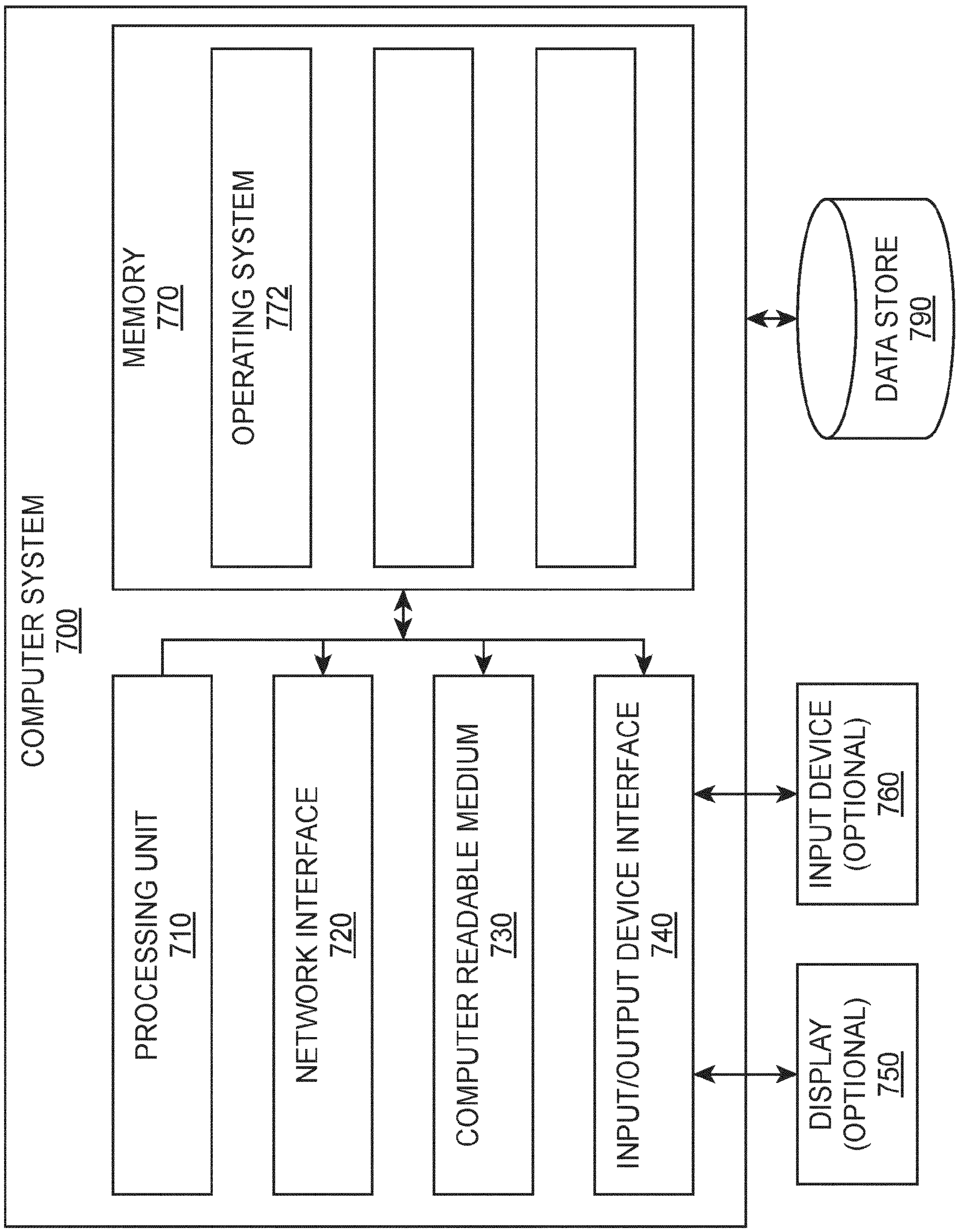
COMPUTER SYSTEM
700
MEMORY
770
OPERATING SYSTEM
772
PROCESSING UNIT
710
NETWORK INTERFACE
720
COMPUTER READABLE MEDIUM
730
INPUT/OUTPUT DEVICE INTERFACE
740
DATA STORE
790
INPUT DEVICE
(OPTIONAL)
760
DISPLAY
(OPTIONAL)
750

FIG. 8A

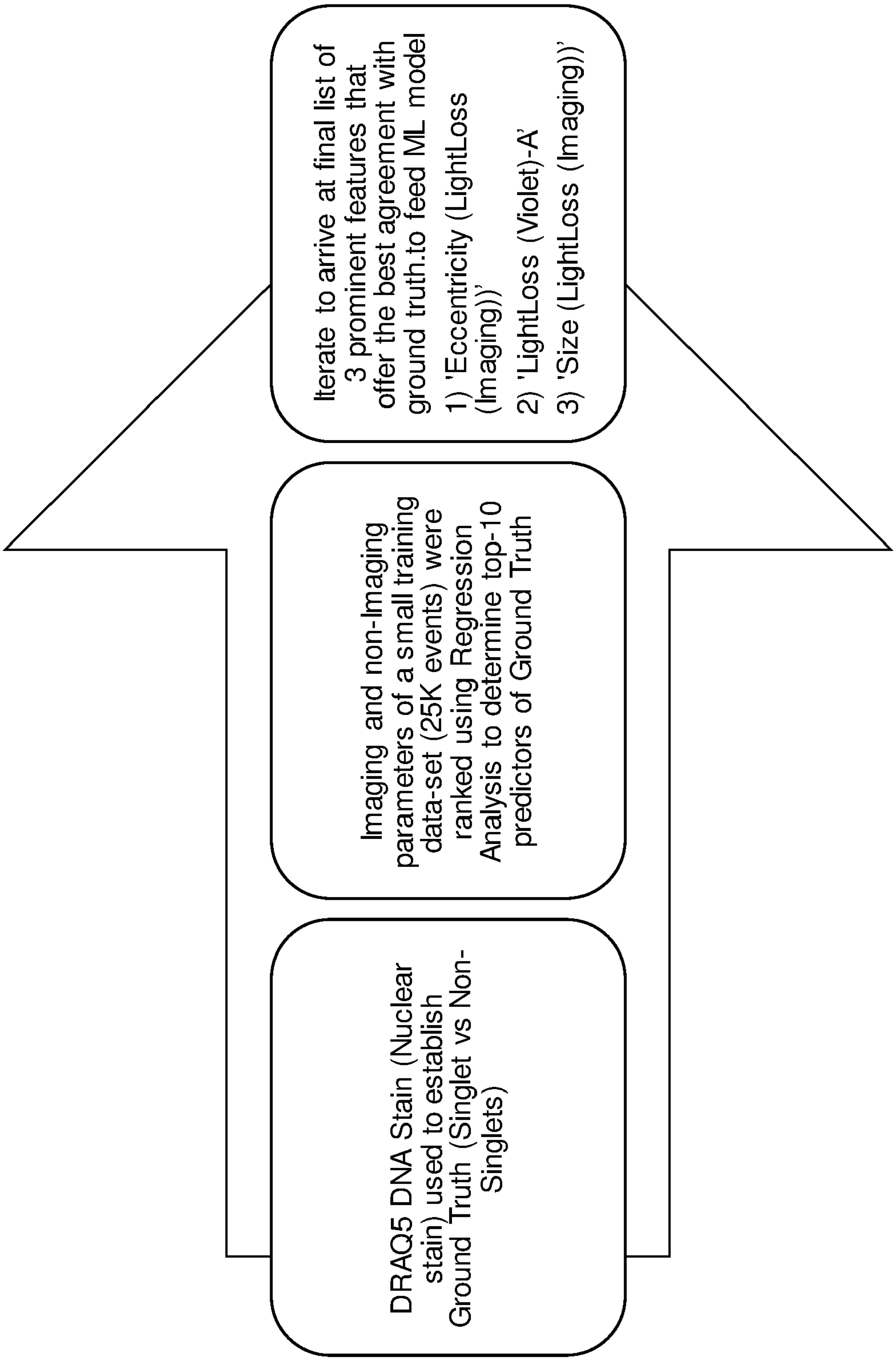
DRAQ5 DNA Stain (Nuclear stain) used to establish Ground Truth (Singlet vs Non-Singlets)
Imaging and non-Imaging parameters of a small training data-set (25K events) were ranked using Regression Analysis to determine top-10 predictors of Ground Truth
Iterate to arrive at final list of 3 prominent features that offer the best agreement with ground truth.to feed ML model
1) 'Eccentricity (LightLoss (Imaging))'
2) 'LightLoss (Violet)-A'
3) 'Size (LightLoss (Imaging))'

FIG. 8B

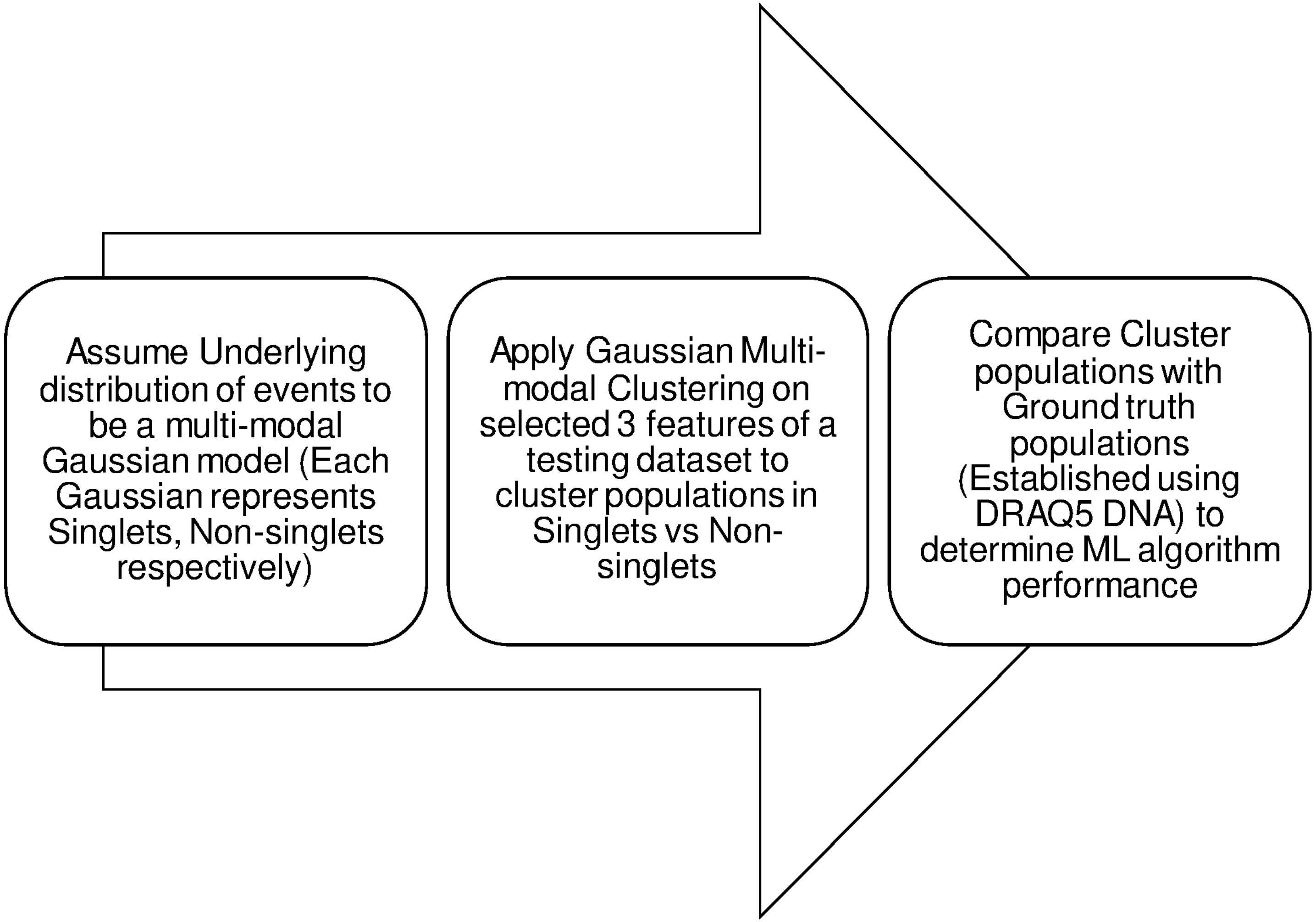
Assume Underlying distribution of events to be a multi-modal Gaussian model (Each Gaussian represents Singlets, Non-singlets respectively)
Apply Gaussian Multi-modal Clustering on selected 3 features of a testing dataset to cluster populations in Singlets vs Non-singlets
Compare Cluster populations with Ground truth populations (Established using DRAQ5 DNA) to determine ML algorithm performance

FIG. 9

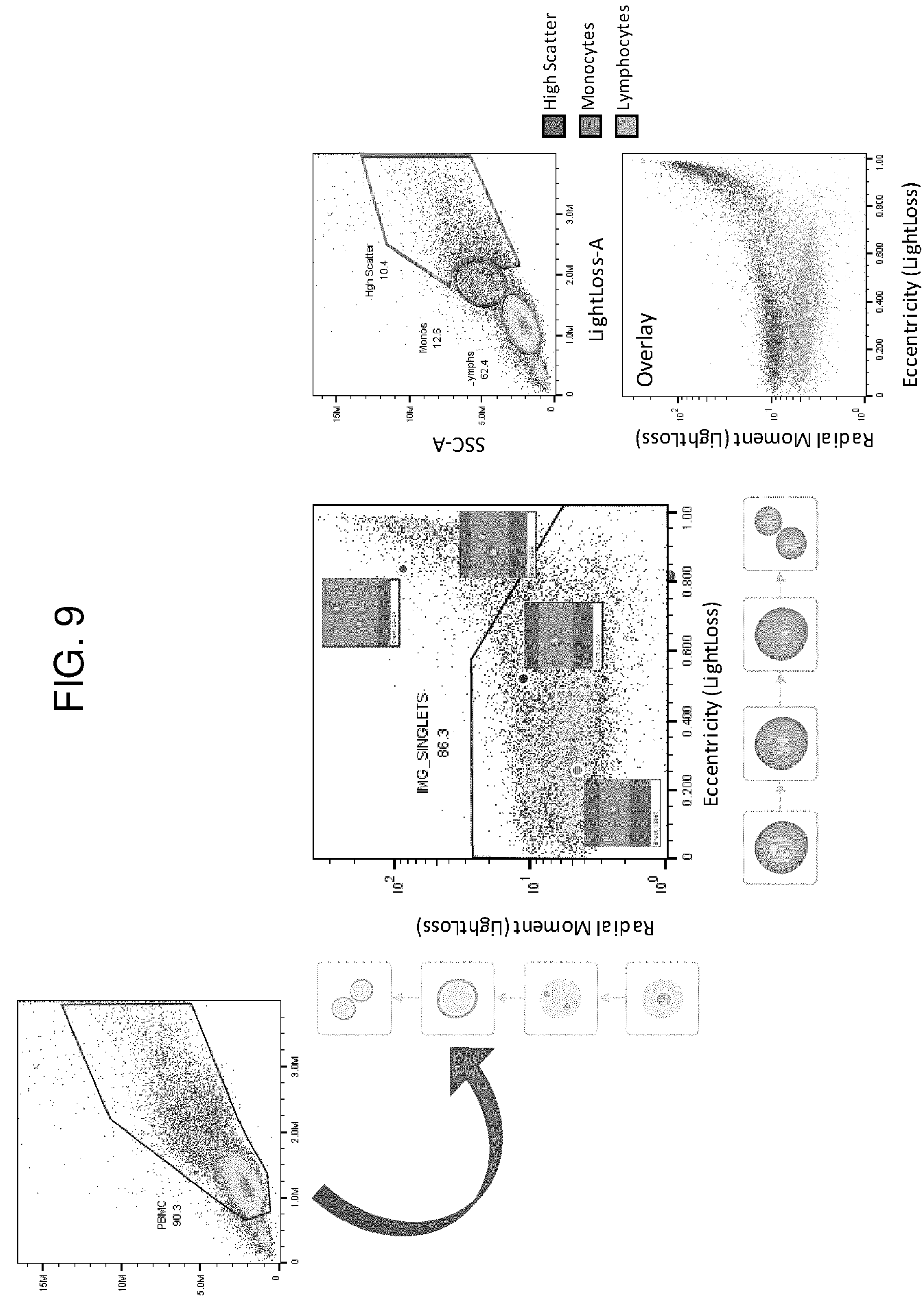
PBMC
90.3
IMG_SINGLETS
86.3
Radial Moment (LightLoss)
Eccentricity (LightLoss)
SSC-A
LightLoss-A
Hgh Scatter
10.4
Monos
12.6
Lymphs
62.4
Overlay
High Scatter
Monocytes
Lymphocytes

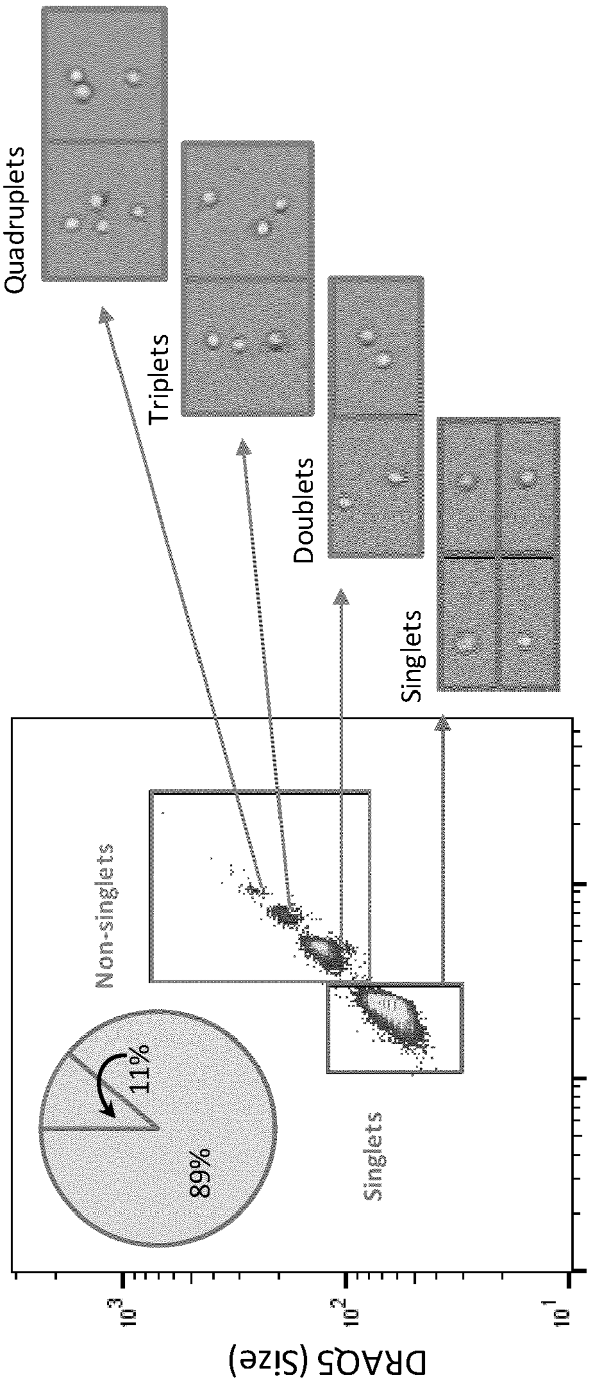
Quadruplets
Triplets
Doublets
Singlets
Non-singlets
Singlets
11%
89%
DRAQ5 (Size)
DRAQ5-A

FIG. 10A

FIG. 10B

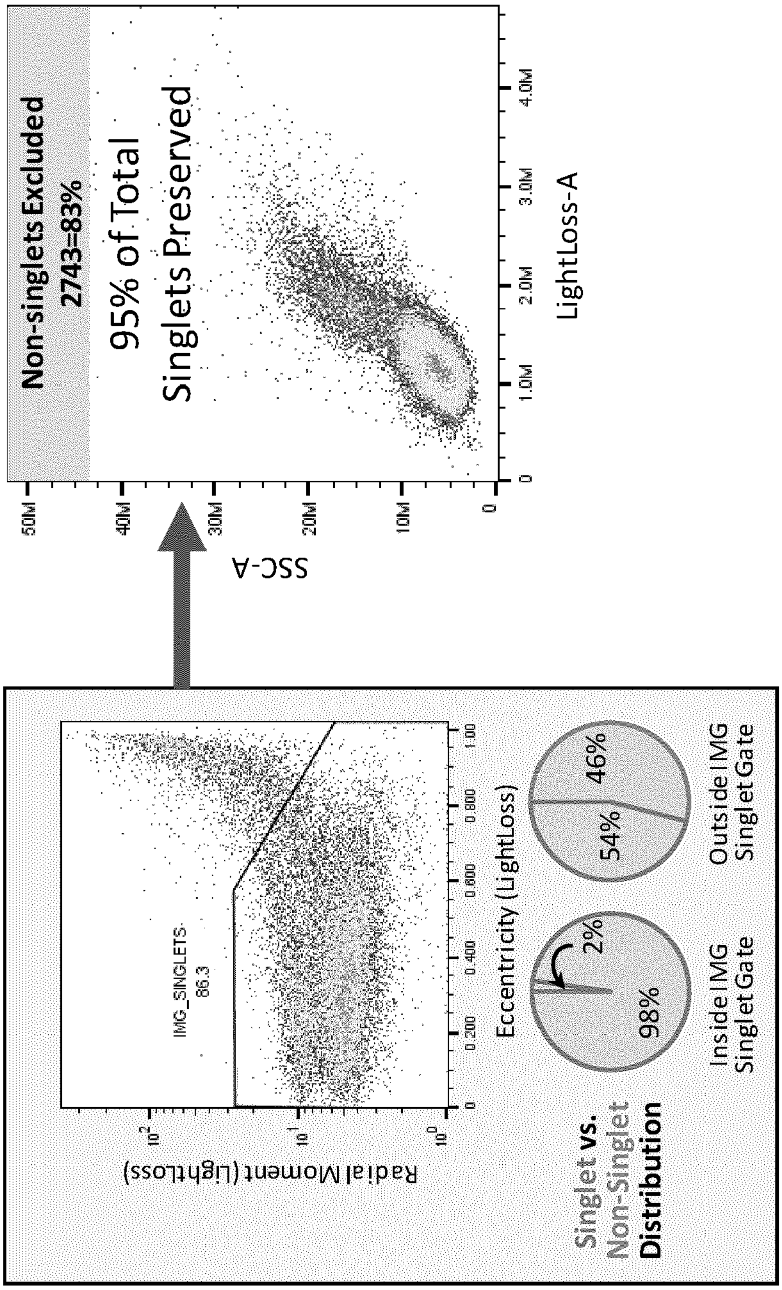
Radial Moment (LightLoss)
Eccentricity (LightLoss)
IMG_SINGLETS
86.3
Singlet vs. Non-Singlet Distribution
98%
2%
Inside IMG Singlet Gate
54%
46%
Outside IMG Singlet Gate
Non-singlets Excluded
2743=83%
95% of Total Singlets Preserved
SSC-A
LightLoss-A

FIG. 11A

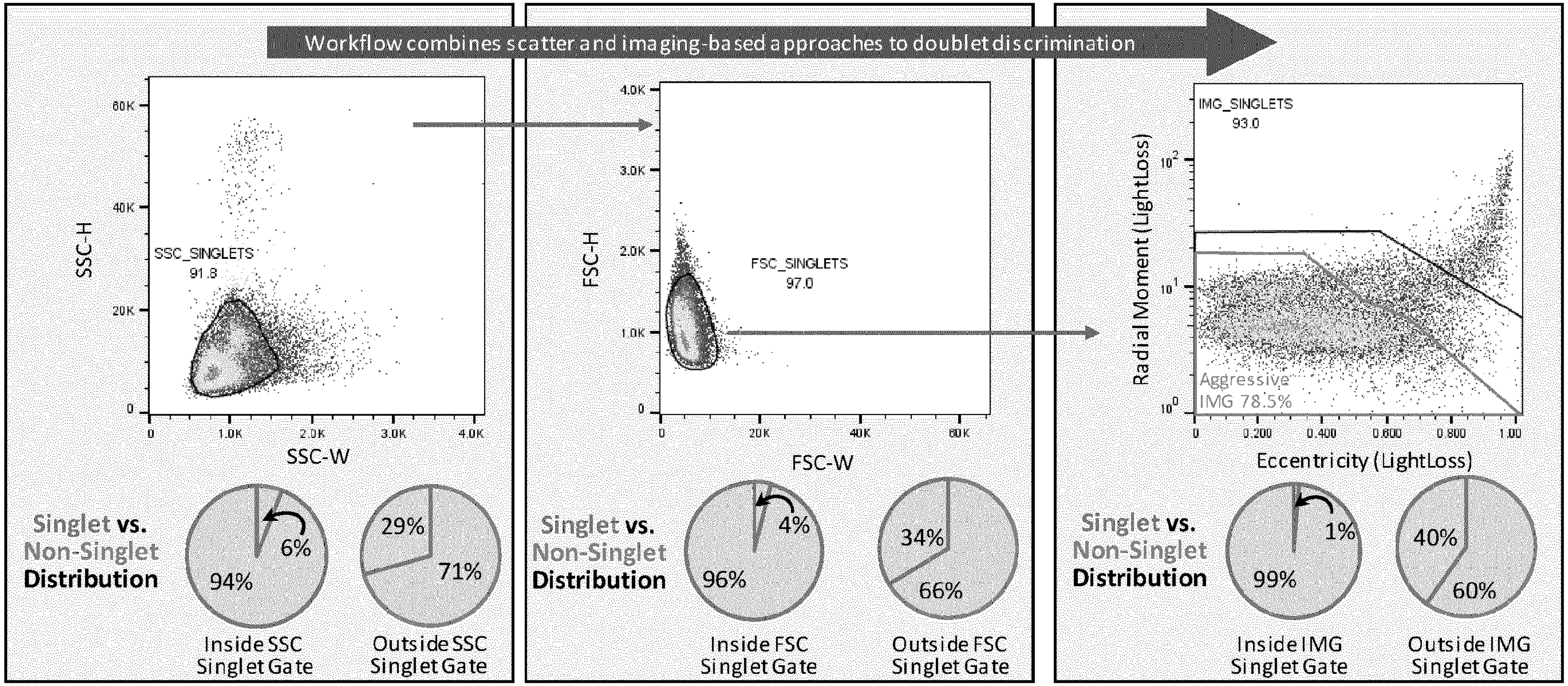
Workflow combines scatter and imaging-based approaches to doublet discrimination
SSC-H
SSC-W
SSC_SINGLETS
91.8
0
20K
40K
60K
1.0K
2.0K
3.0K
4.0K
Singlet vs.
Non-Singlet
Distribution
94%
6%
Inside SSC
Singlet Gate
29%
71%
Outside SSC
Singlet Gate
FSC-H
FSC-W
FSC_SINGLETS
97.0
0
1.0K
2.0K
3.0K
4.0K
20K
40K
60K
Singlet vs.
Non-Singlet
Distribution
96%
4%
Inside FSC
Singlet Gate
34%
66%
Outside FSC
Singlet Gate
Radial Moment (LightLoss)
Eccentricity (LightLoss)
IMG_SINGLETS
93.0
Aggressive
IMG 78.5%
0
0.200
0.400
0.600
0.800
1.00
Singlet vs.
Non-Singlet
Distribution
99%
1%
Inside IMG
Singlet Gate
40%
60%
Outside IMG
Singlet Gate

FIG. 11B

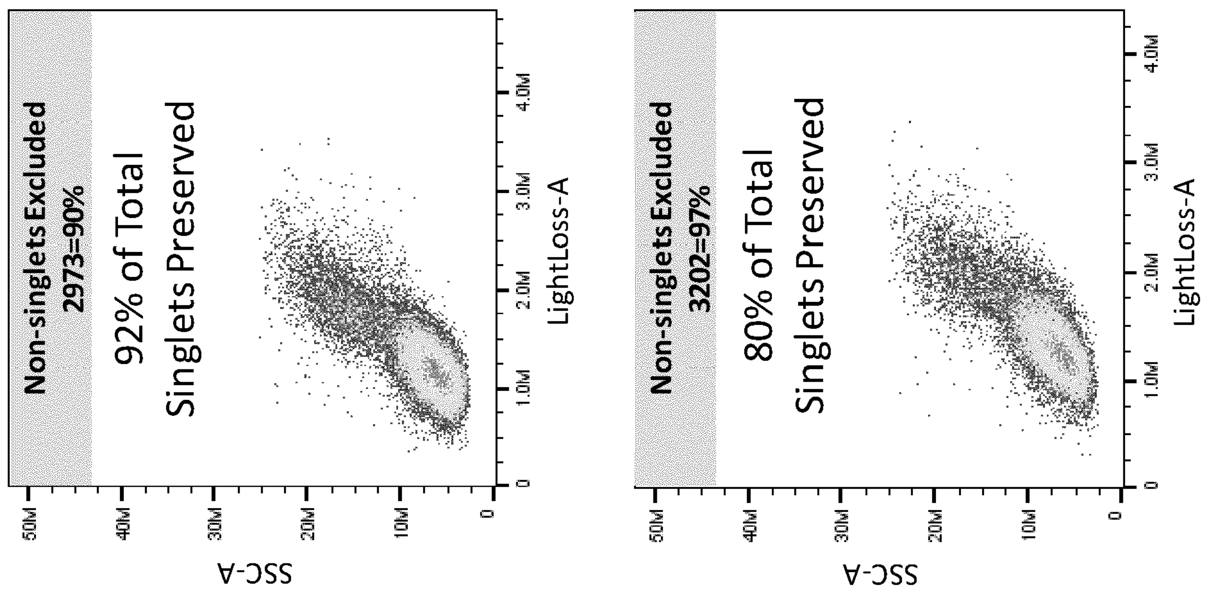
Non-singlets Excluded
2973=90%
92% of Total
Singlets Preserved
SSC-A
LightLoss-A
Non-singlets Excluded
3202=97%
80% of Total
Singlets Preserved
SSC-A
LightLoss-A

FIG. 12A

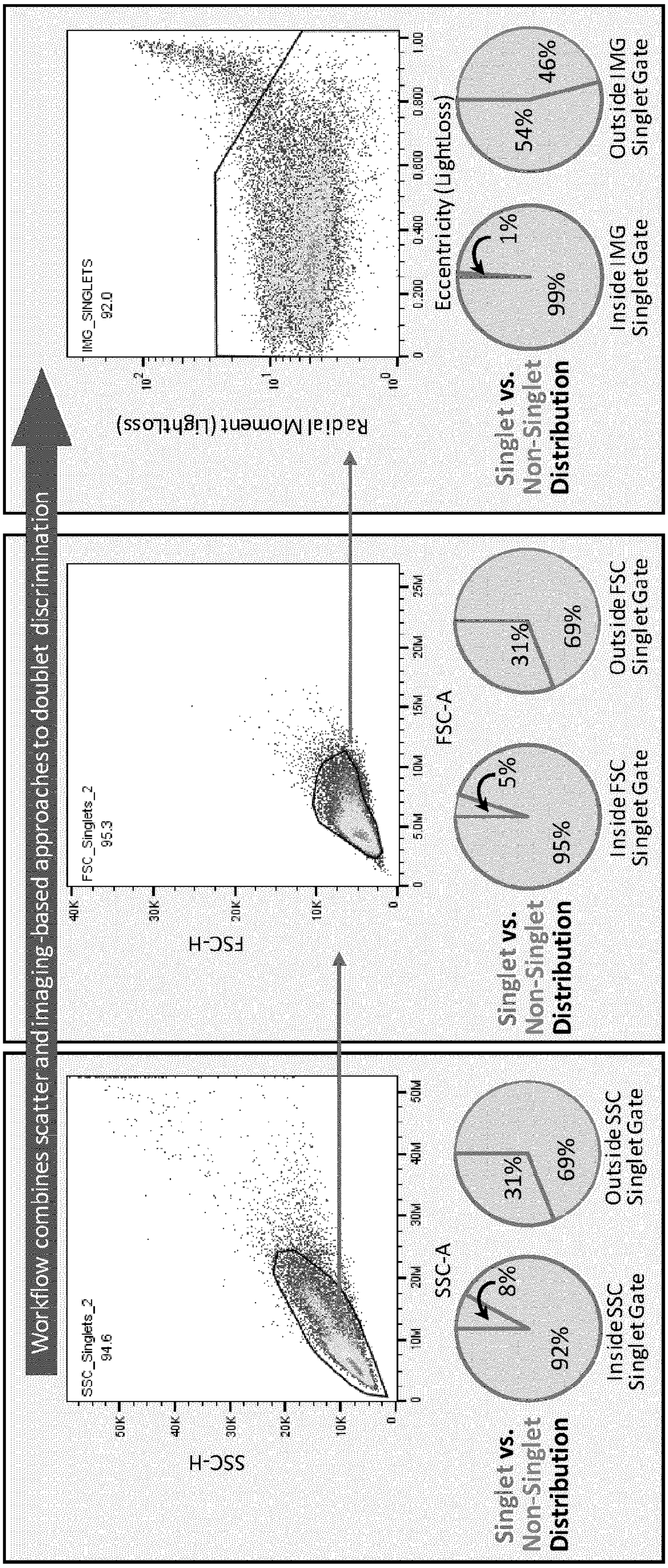
Workflow combines scatter and imaging-based approaches to doublet discrimination
SSC_Singlets_2
94.6
SSC-H
SSC-A
Singlet vs. Non-Singlet Distribution
92%
8%
Inside SSC Singlet Gate
31%
69%
Outside SSC Singlet Gate
FSC_Singlets_2
95.3
FSC-H
FSC-A
Singlet vs. Non-Singlet Distribution
95%
5%
Inside FSC Singlet Gate
31%
69%
Outside FSC Singlet Gate
IMG_SINGLETS
92.0
Radial Moment (LightLoss)
Eccentricity (LightLoss)
Singlet vs. Non-Singlet Distribution
99%
1%
Inside IMG Singlet Gate
54%
46%
Outside IMG Singlet Gate

FIG. 12B

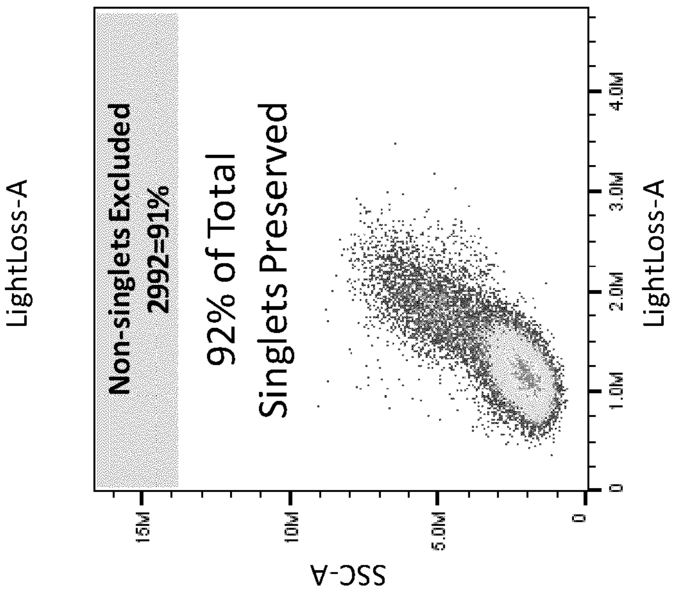
LightLoss-A
Non-singlets Excluded
2992=91%
92% of Total
Singlets Preserved
SSC-A
15M
10M
5.0M
0
1.0M
2.0M
3.0M
4.0M
LightLoss-A

FIG. 13A

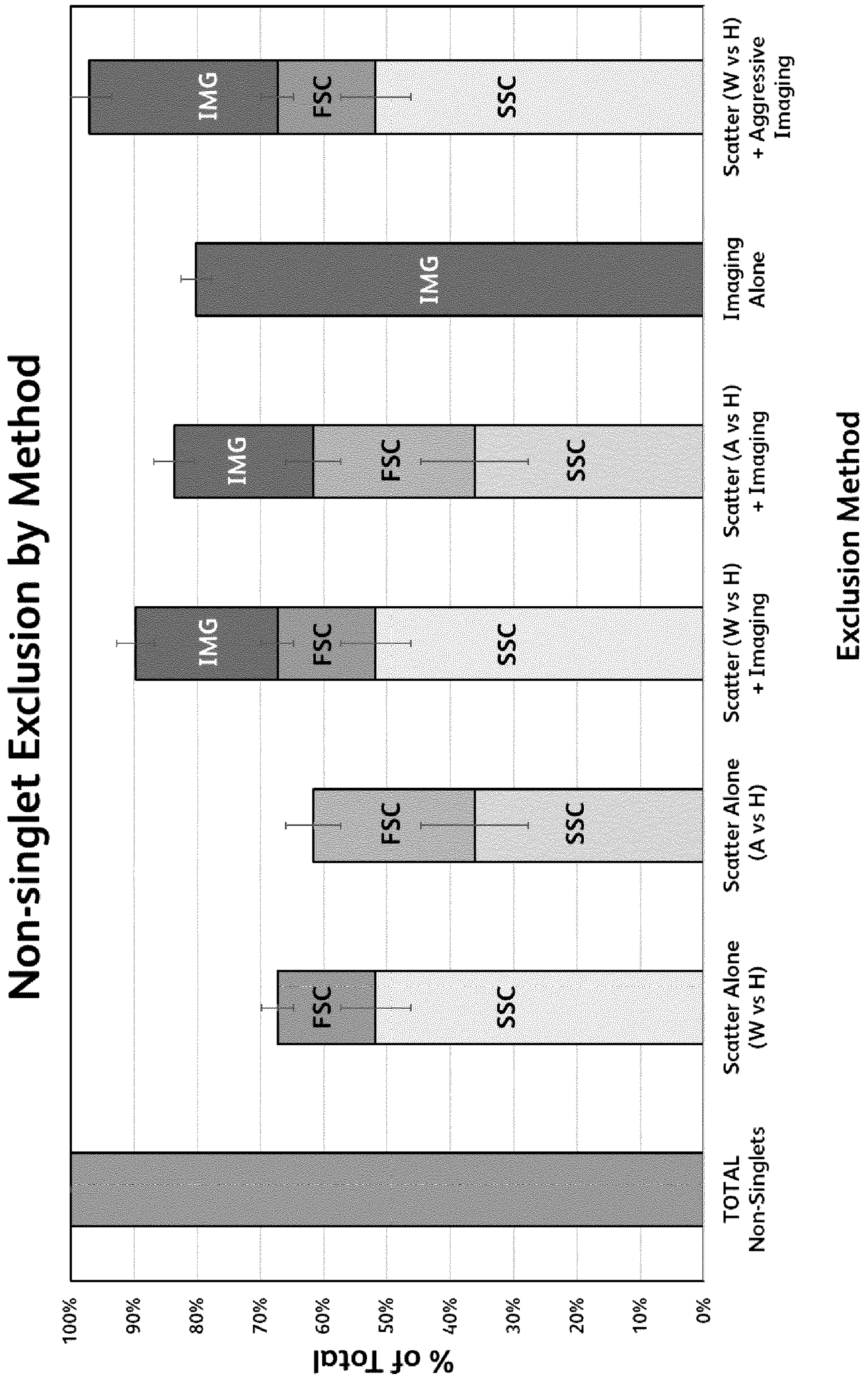
Non-singlet Exclusion by Method
% of Total
100%
90%
80%
70%
60%
50%
40%
30%
20%
10%
0%
TOTAL Non-Singlets
Scatter Alone (W vs H)
Scatter Alone (A vs H)
Scatter (W vs H) + Imaging
Scatter (A vs H) + Imaging
Imaging Alone
Scatter (W vs H) + Aggressive Imaging
IMG
FSC
SSC
Exclusion Method

FIG. 13B

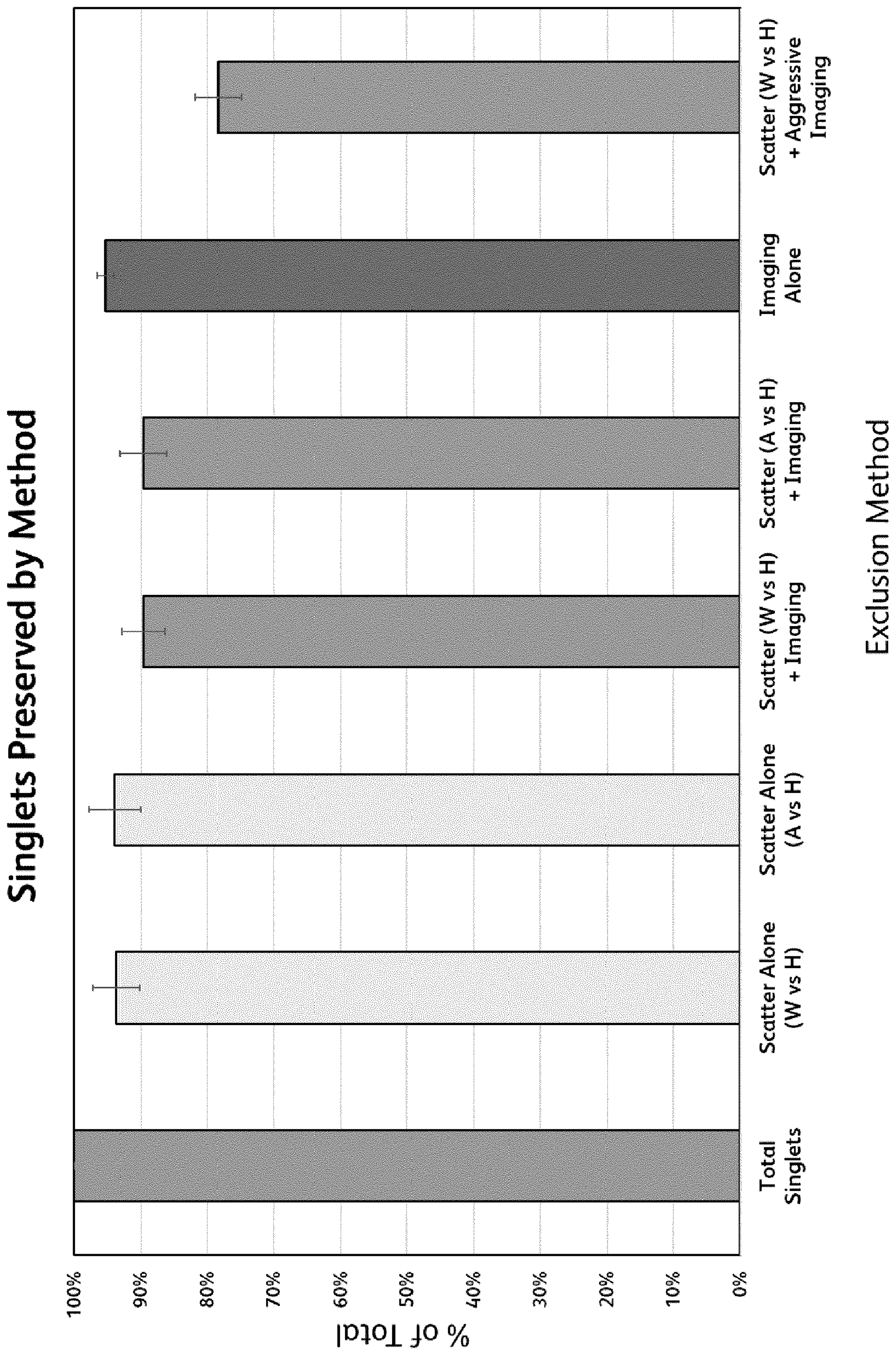
Singlets Preserved by Method
% of Total
100%
90%
80%
70%
60%
50%
40%
30%
20%
10%
0%
Total Singlets
Scatter Alone (W vs H)
Scatter Alone (A vs H)
Scatter (W vs H) + Imaging
Scatter (A vs H) + Imaging
Imaging Alone
Scatter (W vs H) + Aggressive Imaging
Exclusion Method

FIG. 14A

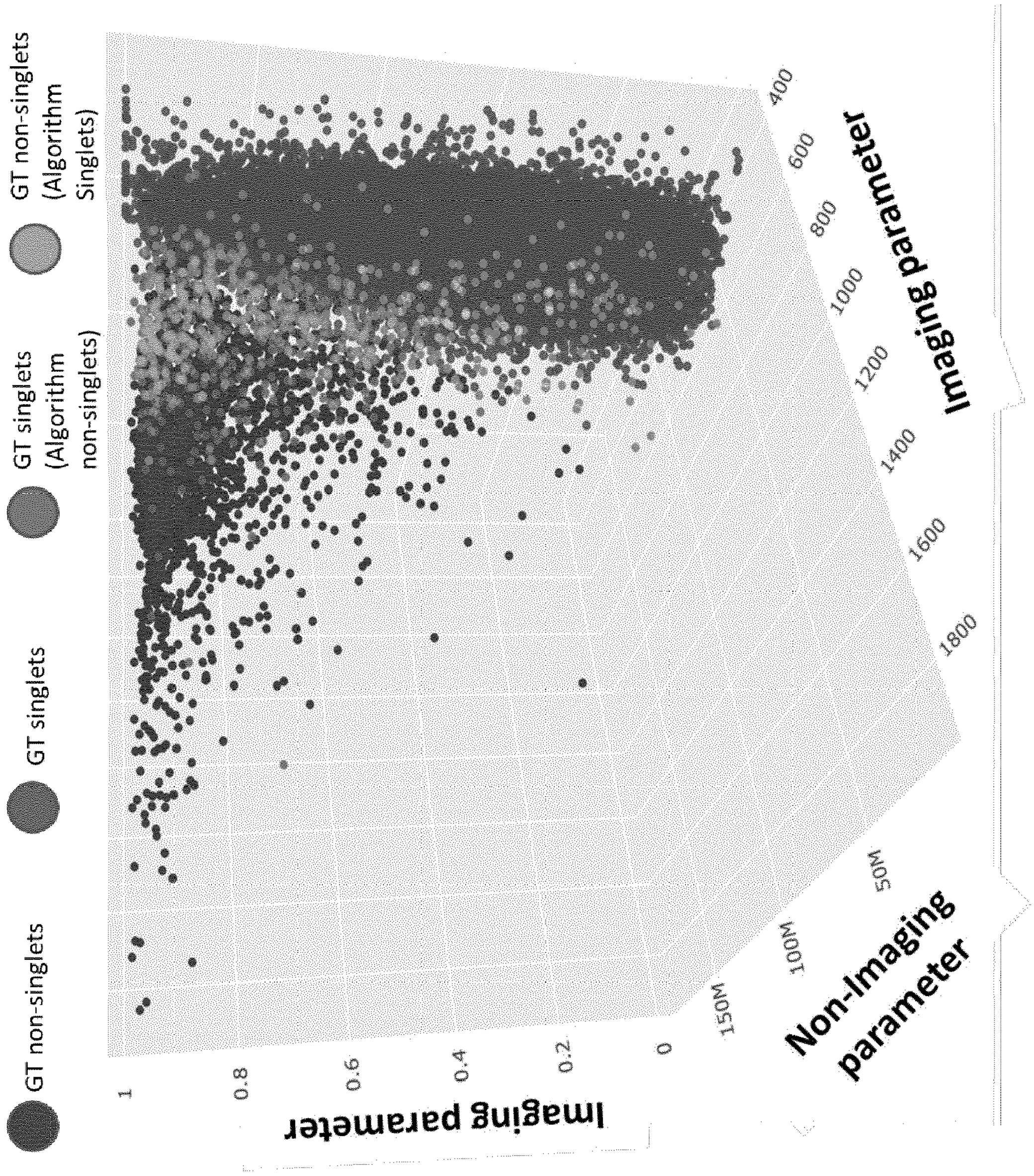
GT non-singlets
GT singlets
GT singlets (Algorithm non-singlets)
GT non-singlets (Algorithm Singlets)
Imaging parameter
1
0.8
0.6
0.4
0.2
0
150M
100M
50M
Non-Imaging parameter
400
600
800
1000
1200
1400
1600
1800
Imaging parameter

FIG. 14B

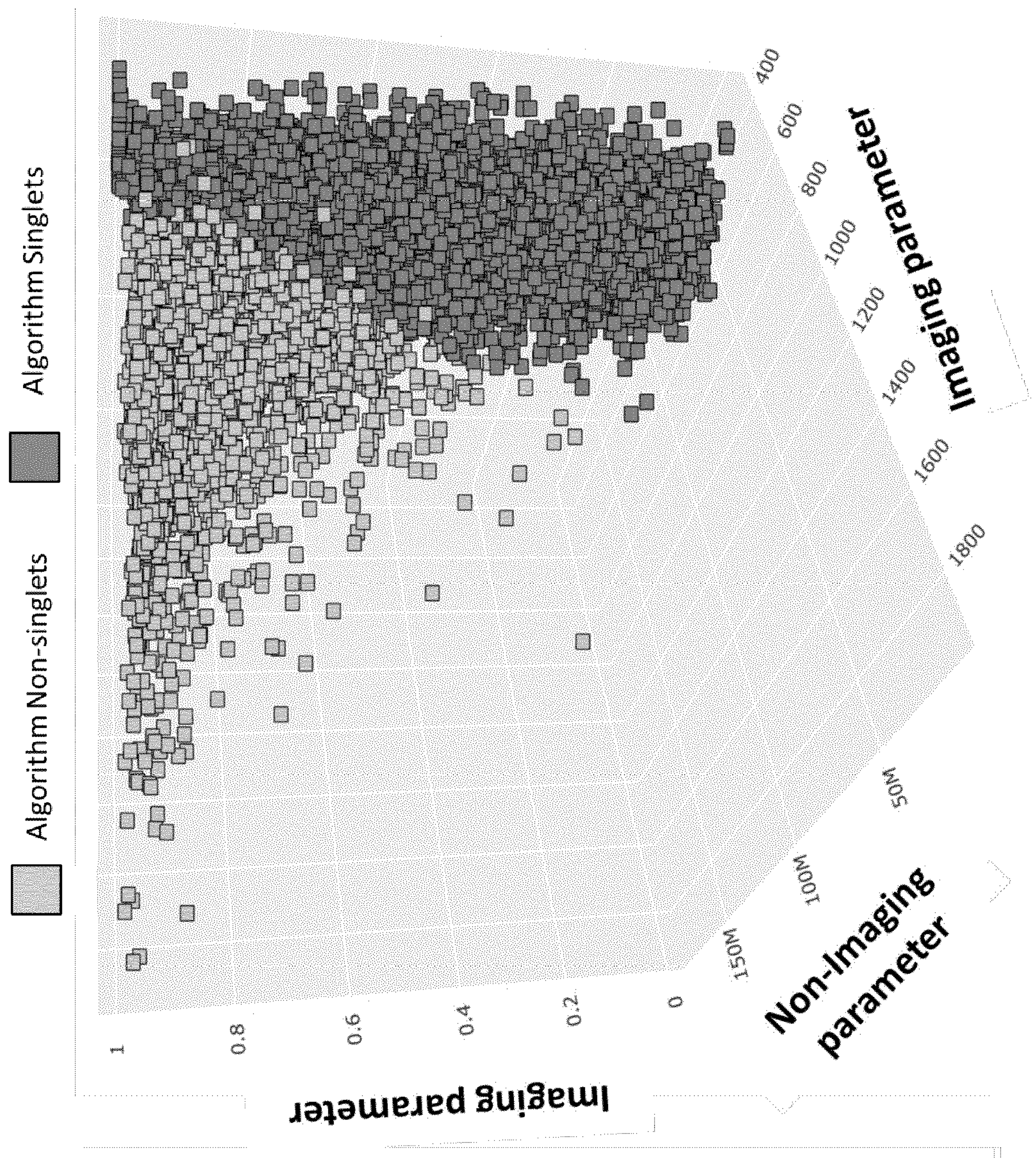
Algorithm Non-singlets
Algorithm Singlets
Imaging parameter
400
600
800
1000
1200
1400
1600
1800
Non-Imaging parameter
50M
100M
150M
1
0.8
0.6
0.4
0.2
0
Imaging parameter

FIG. 15

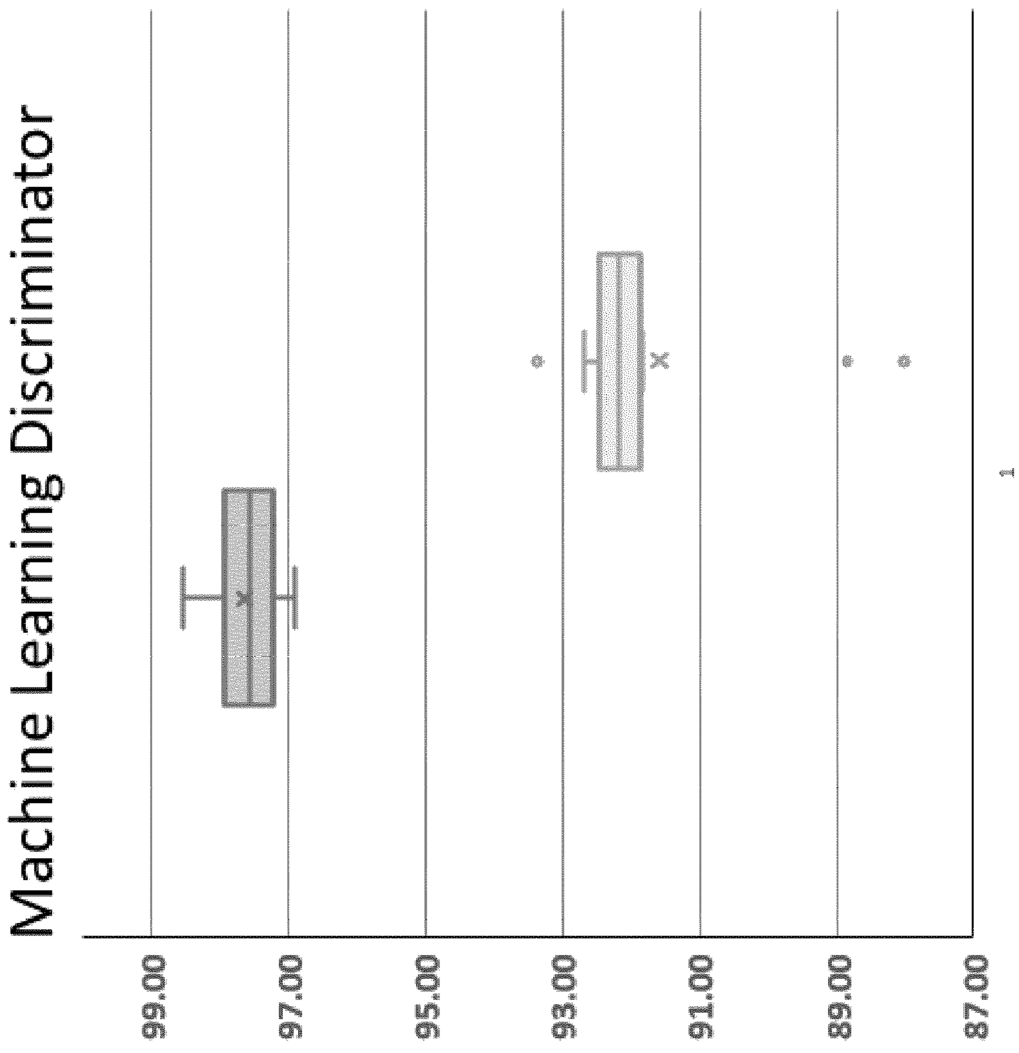
Machine Learning Discriminator
99.00
97.00
95.00
93.00
91.00
89.00
87.00
1

Europäisches Patentamt
European Patent Office
Office européen des brevets

# EUROPEAN SEARCH REPORT

Application Number
EP 24 17 4781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/310053 A1 (SUGIMOTO KEIKI [JP]) 7 October 2021 (2021-10-07) * paragraphs [0003], [0005], [0010], [0070], [0098], [0091], [0099], [0100], [0103], [0115], [0116]; figures 1,4F * | 1-15 | INV. G01N15/1433 G01N15/1429 |
| X | WO 2023/006714 A1 (HOFFMANN LA ROCHE [CH]; HOFFMANN LA ROCHE [US]) 2 February 2023 (2023-02-02) * paragraphs [0001], [0007], [0029], [0052], [0054], [0061], [0063], [0064], [0075], [0077], [0080], [0083]; tables 1,4 * | 1-15 | |
| X | WO 2021/260159 A1 (UNIV LAUSANNE [CH]) 30 December 2021 (2021-12-30) * page 1, first paragraph; paragraph bridging pages 9 and 10; page 10, first complete paragraph; page 81, the paragraph referring to Figure 2; page 82; page 93, section "Data preprocessing", first paragraph; page 94, section "Artificial neural network reconstruction"; page 103, first paragraph; figures 2,3,4b,7d * | 1-15 | TECHNICAL FIELDS SEARCHED (IPC) G01N |
| X | US 2018/032665 A1 (TANG LIN [US] ET AL) 1 February 2018 (2018-02-01) * paragraphs [0007], [0010], [0120], [0154], [0163], [0170], [0197], [0200] - [0202]; figures 4,5A * | 1-15 | |

The present search report has been drawn up for all claims

1

| Place of search | Date of completion of the search | Examiner |
|---|---|---|
| The Hague | 12 September 2024 | Eidmann, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 17 4781

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2024

| Patent document cited in search report | Publication date | Patent family member(s) | Publication date |
|---|---|---|---|
| US 2021310053 A1 | 07-10-2021 | CN 113195718 A | 30-07-2021 |
| | | EP 3867374 A1 | 25-08-2021 |
| | | JP 2022512767 A | 07-02-2022 |
| | | US 2021310053 A1 | 07-10-2021 |
| | | WO 2020081819 A1 | 23-04-2020 |
| WO 2023006714 A1 | 02-02-2023 | EP 4377670 A1 | 05-06-2024 |
| | | WO 2023006714 A1 | 02-02-2023 |
| WO 2021260159 A1 | 30-12-2021 | EP 4172851 A1 | 03-05-2023 |
| | | US 2023401449 A1 | 14-12-2023 |
| | | WO 2021260159 A1 | 30-12-2021 |
| US 2018032665 A1 | 01-02-2018 | US 2014235474 A1 | 21-08-2014 |
| | | US 2018032665 A1 | 01-02-2018 |
| | | US 2021233609 A1 | 29-07-2021 |
| | | WO 2012177792 A2 | 27-12-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63465057
- US 63569559
- US 9423353 B
- US 9784661 B
- US 10006852 B
- US 20170133857 A
- US 20170350803 A
- US 20220341838
- US 10663476 B
- US 10620111 B
- US 10613017 B
- US 10605713 B
- US 10585031 B
- US 10578542 B
- US 10578469 B
- US 10481074 B
- US 10302545 B
- US 10145793 B
- US 10113967 B
- US 9952076 B
- US 9933341 B
- US 9726527 B
- US 9453789 B
- US 9200334 B
- US 9097640 B
- US 9095494 B
- US 9092034 B
- US 8975595 B
- US 8753573 B
- US 8233146 B
- US 8140300 B
- US 7544326 B
- US 7201875 B
- US 7129505 B
- US 6821740 B
- US 6813017 B
- US 6809804 B
- US 6372506 B
- US 5700692 A
- US 5643796 A
- US 5627040 A
- US 5620842 A
- US 5602039 A
- US 4987086 A
- US 4498766 A
- US 63431803
- US 63465057 B
- US 20170299493 A
- US 20200256781 A
- US 20210404943 A
- US 7679039 B


### Non-patent literature cited in the description


- **ESTER et al.** *Proceedings of the Second International Conference on Knowledge Discovery and Data,* 1996, vol. 96 (34), 226-231
- **LLOYD, STUART P.** *IEEE Transactions on Information Theory.,* 1967, vol. 28 (2), 129-137
- **ZHANG et al.** *ACM sigmod record.,* 1996, vol. 25 (2), 103-114
- **VON LUXBURG, U.** *Statistics and computing,* 2007, vol. 17, 395-416
- Flow Cytometry: A Practical Approach. Oxford Univ. Press, 1997
- Flow Cytometry Protocols, Methods in Molecular Biology. Humana Press, 1997
- Practical Flow Cytometry. Wiley-Liss, 1995
- **VIRGO et al.** *Ann Clin Biochem.,* January 2012, vol. 49, 17-28
- **LINDEN.** *Semin Throm Hemost.,* October 2004, vol. 30 (5), 502-11
- **ALISON et al.** *J Pathol,* December 2010, vol. 222 (4), 335-344
- **HERBIG et al.** *Crit Rev Ther Drug Carrier Syst.,* 2007, vol. 24 (3), 203-255
- **DIEBOLD et al.** *Nature Photonics,* 2013, vol. 7 (10), 806-810
- **SCHRAIVOGEL et al.** High-speed fluorescence image-enabled cell sorting. *Science,* 2022, vol. 375 (6578), 315-320